# EUROPEAN PATENT APPLICATION

(11) **EP 4 119 554 A1**
(43) Date of publication of application: **18.01.2023**
(21) Application number: 21768272.3
(22) Date of filing: 11.03.2021
(51) Int. Cl.: C07D 405/12, C07D 405/14, C07D 409/14, H01L 51/00, H01L 51/50

(54) **AMINE COMPOUND HAVING FUSED RING, AND ORGANIC LIGHT-EMITTING DEVICE COMPRISING SAME**

(30) Priority: 12.03.2020 KR 20200030764
(71) Applicant: SFC Co., Ltd., Cheongju-si, Chungcheongbuk-do 28122 (KR)
(72) Inventor: LEE, Chun-young, Cheongju-si Chungcheongbuk-do 28122 (KR); SHIN, Yoo-na, Cheongju-si Chungcheongbuk-do 28122 (KR); KIM, Tae-young, Cheongju-si Chungcheongbuk-do 28122 (KR); KIM, Sung-woo, Cheongju-si Chungcheongbuk-do 28122 (KR); LEE, Ji-won, Cheongju-si Chungcheongbuk-do 28122 (KR)
(74) Representative: Grosse, Felix Christopher
(86) International application number: PCT/KR2021/003023
(87) International publication number: WO 2021/182888

(57) **Abstract**

The present invention relates to an amine compound having a fused ring that can be employed in various organic layers in an organic light-emitting device, and a high-efficiency organic light-emitting device comprising same and having significantly improved luminous efficiency. The amine compound having a fused ring, according to the present invention, makes it possible to implement a high-efficiency organic light-emitting device by being employed as an organic layer in the device, particularly as a hole transport or electron blocking material, and thus can be industrially utilized in a flat panel display device, a flexible display device, a monochromatic or white flat panel lighting device, a monochromatic or white flexible lighting device, a vehicle display device, a virtual or augmented reality display device, and the like.

## Description

### [Technical Field]

The present invention relates to an amine compound having a fused ring, and a highly efficient and long-lasting organic light-emitting device that exhibits remarkably improved luminous efficacy using the same.

### [Background Art]

An organic light-emitting device is a self-luminous device that emits light when energy is released from excitons which are formed by recombination of electrons injected from an electron injection electrode (cathode) and holes injected from a hole injection electrode (anode) in a light-emitting layer. Such an organic light-emitting device attracts a great deal of attention as a next-generation light source due to applicability to full-color flat panel light-emitting displays based on advantages such as low driving voltage, high luminance, wide viewing angle, and rapid response speed thereof.

In order for the organic light-emitting device to exhibit the characteristics, the structure of the organic layer in the organic light-emitting device should be optimized, and the material constituting each organic layer, namely, a hole injection material, a hole transport material, a light-emitting material, an electron transport material, an electron injection material, or an electron blocking material should be based on stable and efficient ingredients. However, there is a continuing need to develop organic layer structures and respective materials thereof for stable and efficient organic light-emitting devices.

As such, there is a continuing need for the development of the structure of an organic light-emitting device capable of improving the luminous characteristics thereof and the development of novel materials supporting the structure.

### [Disclosure]

### [Technical Problem]

Therefore, the present invention has been made in view of the above problems, and it is one object of the present invention to provide an organic light-emitting compound that can be used in an organic layer of a device to realize a highly efficient organic light-emitting device and an organic light-emitting device including the same.

### [Technical Solution]

In accordance with the present invention, the above and other objects can be accomplished by the provision of a compound having a skeletal structure represented by the following [Formula A] or [Formula B], and an amine group introduced therein. 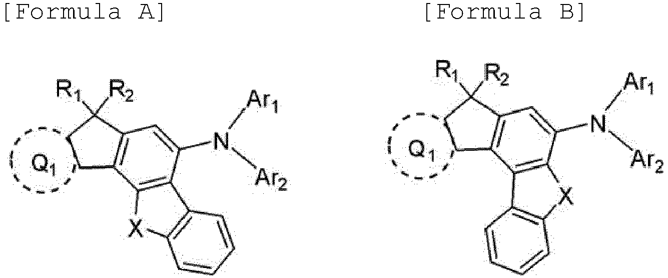 wherein at least one of Ar₁ and Ar₂ is represented by the following [Structural Formula 1]:

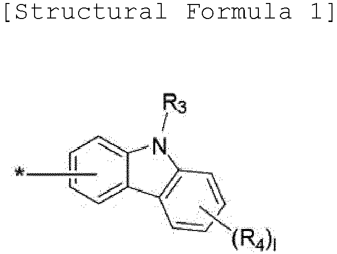

Specific structures of [Formula A] and [Formula B], the compounds obtained thereby, and definitions of X, Q₁, R₁ to R₃ and Ar₁ to Ar₂ will be given later.

In accordance with another aspect of the present invention, provided is an organic light-emitting device including a first electrode, a second electrode facing the first electrode, and an organic layer interposed between the first electrode and the second electrode, wherein the organic layer includes at least one compound represented by [Formula A] or [Formula B].

In an embodiment of the present invention, the organic layer including at least one compound represented by [Formula A] or [Formula B] may be a hole transport layer or an electron blocking layer.

### [Advantageous Effects]

The amine compound having a fused ring according to the present invention can be used as a material for an - organic layer, particularly, as a material for a hole transport or electron blocking layer in the device to realize a highly efficient organic light-emitting device.

### [Best Mode]

Hereinafter, the present invention will be described in detail with reference to the annexed drawings.

The amine compound having a fused ring according to the present invention is represented by the following [Formula A] or [Formula B] and is capable of realizing a highly efficient organic light-emitting device based on an amine group introduced into such a skeleton structure. 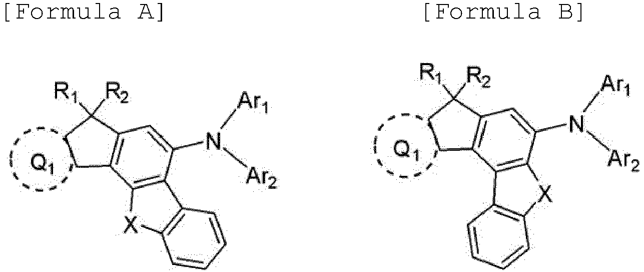 wherein
Q₁ is selected from a substituted or unsubstituted C6-C30 aryl group, and a substituted or unsubstituted C2-C50 heteroaryl group,
X is an oxygen atom (O) or a sulfur atom (S),
R₁ and R₂ are identical to or different from each other, and are each independently selected from hydrogen, deuterium, a substituted or unsubstituted C1-C30 alkyl group, a substituted or unsubstituted C3-C30 cycloalkyl group, a substituted or unsubstituted C6-C50 aryl group, and a substituted or unsubstituted C2-C50 heteroaryl group, with the proviso that R₁ and R₂ are bonded to each other to form an alicyclic or aromatic monocyclic or polycyclic ring,
Ar₁ and Ar₂ are identical to or different from each other, and are each independently a substituted or unsubstituted C6-C50 aryl group, and a substituted or unsubstituted C2-C50 heteroaryl group, with the proviso that at least one of Ar₁ and Ar₂ is represented by the following Structural Formula 1: 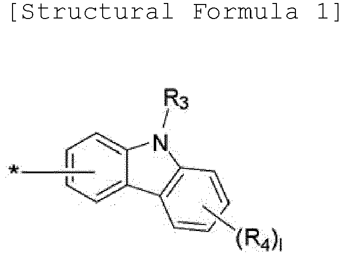 wherein
R₃ is selected from hydrogen, deuterium, a substituted or unsubstituted C1-C30 alkyl group, a substituted or unsubstituted C3-C30 cycloalkyl group, a substituted or unsubstituted C6-C50 aryl group, and a substituted or unsubstituted C2-C50 heteroaryl group,
R₄ is selected from hydrogen, deuterium, a cyano group, a halogen group, a hydroxyl group, a nitro group, a C1-C24 alkyl group, a C1-C24 halogenated alkyl group, a C1-C24 cycloalkyl group, a C1-C24 alkenyl group, a C1-C24 alkynyl group, a C1-C24 heteroalkyl group, a C6-C30 aryl group, a C6-C30 arylalkyl group, a C2-C30 heteroaryl group, a C2-C30 heteroarylalkyl group, a C1-C24 alkoxy group, a C1-C24 alkylamino group, a C6-C30 arylamino group, a C2-C30 heteroarylamino group, a C1-C24 alkylsilyl group, a C6-C30 arylsilyl group, and a C6-C30 aryloxy group,
l is an integer from 0 to 4, provided that when l is 2 or more, R₄' s are identical to or different from each other, and
"-*" means a site bonding to a nitrogen atom at the positions Ar₁ and Ar₂ in [Formula A] or [Formula B].

According to an embodiment of the present invention, R₁ and R₂ are each independently selected from a substituted or unsubstituted C1-C30 alkyl group, a substituted or unsubstituted C3-C30 cycloalkyl group, a substituted or unsubstituted C6-C50 aryl group, and a substituted or unsubstituted C2-C50 heteroaryl group.

In addition, when R₁ and R₂ are bonded to each other to form a ring, a compound represented by the following [Structural Formula 2] may be obtained: 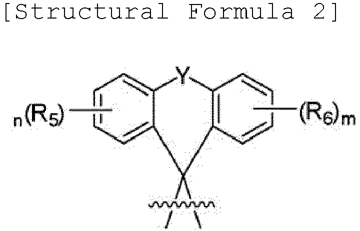 wherein
Y is a single bond, an oxygen atom (O) or a sulfur atom (S),
R₅ and R₆ are selected from hydrogen, deuterium, a cyano group, a halogen group, a hydroxyl group, a nitro group, a C1-C24 alkyl group, a C1-C24 halogenated alkyl group, a C1-C24 cycloalkyl group, a C1-C24 alkenyl group, a C1-C24 alkynyl group, a C1-C24 heteroalkyl group, a C6-C30 aryl group, a C6-C30 arylalkyl group, a C2-30 heteroaryl group, a C2-C30 heteroarylalkyl group, a C1-C24 alkoxy group, a C1-C24 alkylamino group, a C6-C30 arylamino group, a C2-30 heteroarylamino group, a C1-C24 alkylsilyl group, a C6-C30 arylsilyl group and a C6-C30 aryloxy group, n and m are each an integer from 0 to 4, with the proviso that, when n and m are each 2 or more, R₅'s and R₆'s are identical to or different from each other.

According to an embodiment of the present invention, Q₁ may be represented by the following [Structural Formula 3] : 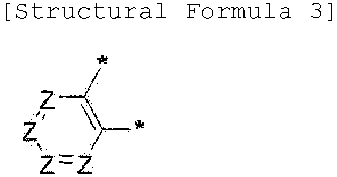 wherein
Z is N or CR, wherein R is selected from hydrogen, deuterium, a substituted or unsubstituted C1-C30 alkyl group, a substituted or unsubstituted C6-C50 aryl group, a substituted or unsubstituted C3-C30 cycloalkyl group, a substituted or unsubstituted C2-C50 heteroaryl group, a substituted or unsubstituted C1-C30 alkoxy group, a substituted or unsubstituted C6-C30 aryloxy group, a substituted or unsubstituted C1-C30 alkylthioxy group, a substituted or unsubstituted C5-C30 arylthioxy group, a substituted or unsubstituted C1-C30 alkylamine group, a substituted or unsubstituted C5-C30 arylamine group, a substituted or unsubstituted C1-C30 alkylsilyl group, a substituted or unsubstituted C5-C30 arylsilyl group, a nitro group, a cyano group, and a halogen group, and
R's are bonded to each other or each thereof is bonded to an adjacent substituent to form at least one alicyclic or aromatic monocyclic or polycyclic ring, and the carbon atom of the formed alicyclic, aromatic monocyclic or polycyclic ring is substituted with at least one heteroatom selected from (N), a sulfur atom (S), and an oxygen atom (O).

Meanwhile, as used herein, the term "substituted" indicates substitution of various substituents defined in each of the formulas with one or more substituents selected from deuterium, a cyano group, a halogen group, a hydroxyl group, a nitro group, an alkyl group, a halogenated alkyl group, a cycloalkyl group, an alkenyl group, an alkynyl group, a heteroalkyl group, an aryl group, an arylalkyl group, an alkylaryl group, a heteroaryl group, a heteroarylalkyl group, an alkoxy group, an amine group, a silyl group, an aryloxy group and a mixed aliphatic-aromatic ring group, or substitution with a substituent including two or more of the substituents linked to each other. The term "unsubstituted" in the same definition indicates having no substituent.

In addition, the range of the number of the carbon atoms of the alkyl group or aryl group in the term "substituted or unsubstituted C1-C30 alkyl group", "substituted or unsubstituted C6-C50 aryl group" or the like refers to the total number of carbon atoms constituting the alkyl or aryl moiety when the corresponding group is not substituted without considering the number of carbon atoms in the substituent(s). For example, a phenyl group substituted at the para position with a butyl group corresponds to an aryl group having 6 carbon atoms substituted with a butyl group having 4 carbon atoms.

In addition, as used herein, the expression "a substituent is bonded to an adjacent substituent to form a ring" means that the corresponding substituent is bonded to the adjacent substituent to form a substituted or unsubstituted alicyclic or aromatic ring, and the term "adjacent substituent" may mean a substituent substituted for an atom which is directly attached to an atom substituted with the corresponding substituent, a substituent sterically disposed at the nearest position to the corresponding substituent, or another substituent substituted for an atom which is substituted with the corresponding substituent. For example, two substituents substituted at the ortho position in a benzene ring and two substituents substituted at the same carbon in the aliphatic ring may be considered "adjacent" to each other.

As used herein, the alkyl group may be a linear or branched alkyl group. Examples of the alkyl group include, but are not limited to, a methyl group, an ethyl group, a propyl group, an n-propyl group, an isopropyl group, a butyl group, an n-butyl group, an isobutyl group, a tert-butyl group, a sec-butyl group, a 1-methylbutyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group, and the like.

As used herein, the alkenyl group may include a linear or branched alkenyl group and may be further substituted with another substituent. Specifically, examples of the alkenyl group include, but are not limited to, a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group, and the like.

As used herein, the alkynyl group may also include a linear or branched alkynyl group, and may be further substituted with another substituent, and examples of the substituent may include, but are not limited to, ethynyl, 2-propynyl, and the like.

As used herein, the aromatic hydrocarbon ring or the aryl group may be monocyclic or polycyclic, examples of the monocyclic aryl group include a phenyl group, a biphenyl group, a terphenyl group, a stilbene group, and the like, and examples of the polycyclic aryl group include, but are not limited to, a naphthyl group, an anthracenyl group, a phenanthrenyl group, a pyrenyl group, a perylenyl group, a tetracenyl group, a chrysenyl group, a fluorenyl group, an acenaphthcenyl group, a triphenylene group, a fluoranthene group, and the like, but the scope of the present invention is not limited thereto.

As used herein, the aromatic heterocyclic or heteroaryl group is an aromatic ring containing at least one heteroatom and examples thereof include, but are not limited to, thiophene, furan, pyrrole, imidazole, triazole, oxazole, oxadiazole, triazole, pyridyl, bipyridyl, pyrimidyl, triazine, triazole, acridyl, pyridazine, pyrazinyl, quinolinyl, quinazoline, quinoxalinyl, phthalazinyl, pyridopyrimidinyl, pyridopyrazinyl, pyrazinopyrazinyl, isoquinoline, indole, carbazole, benzoxazole, benzimidazole, benzothiazole, benzocarbazole, benzothiophene, dibenzothiophene, benzofuranyl, dibenzofuranyl, phenanthroline, thiazolyl, isoxazolyl, oxadiazolyl, thiadiazolyl, benzothiazolyl, and phenothiazinyl groups and the like.

As used herein, the aliphatic hydrocarbon ring refers to a non-aromatic ring that contains only carbon and hydrogen atoms, for example, includes a monocyclic or polycyclic ring, and may be further substituted with another substituent. The term "polycyclic" means that the polycyclic group may be directly attached to or fused with at least one other cyclic group, the other cyclic group may be an aliphatic hydrocarbon ring, or a different type of ring group, for example, an aliphatic heterocyclic group, an aryl group, a heteroaryl group, and the like. Specifically, examples thereof include, but are not limited to, cycloalkyls such as a cyclopropyl group, a cyclobutyl group, a cyclopentyl group, an adamantyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, and a cyclooctyl group, cycloalkanes such as cyclohexane and cyclopentane, and cycloalkenes such as cyclohexene and cyclobutene.

As used herein, the aliphatic heterocyclic ring refers to an aliphatic ring that contains at least one of heteroatoms such as O, S, Se, N and Si, also includes a monocyclic or polycyclic ring, and may be further substituted with another substituent. The term "polycyclic" means that the polycyclic group may be directly attached to or fused with at least one other cyclic group, and the other cyclic group may be an aliphatic hydrocarbon ring, or a different type of ring group, for example, an aliphatic heterocyclic group, an aryl group, a heteroaryl group, or the like.

As used herein, the mixed aliphatic-aromatic ring group refers to a ring in which two or more rings are attached to and fused with each other, and aliphatic and aromatic rings are fused together to be overall non-aromatic, and a polycyclic mixed aliphatic-aromatic ring may contain a heteroatom selected from N, O, P and S, in addition to C.

As used herein, specifically, the alkoxy group may be methoxy, ethoxy, propoxy, isobutyloxy, sec-butyloxy, pentyloxy, iso-amyloxy, hexyloxy, or the like, but is not limited thereto.

As used herein, the silyl group is represented by - SiH₃, and may be an alkylsilyl group, an arylsilyl group, an alkylarylsilyl group, an arylheteroarylsilyl group, or the like, and specific examples of the silyl group include trimethylsilyl, triethylsilyl, triphenylsilyl, trimethoxysilyl, dimethoxyphenylsilyl, diphenylmethylsilyl, diphenylvinylsilyl, methylcyclobutylsilyl, dimethylfurylsilyl, and the like.

As used herein, the amine group is represented by -NH₂, or may be an alkylamine group, an arylamine group, an arylheteroarylamine group, or the like. The arylamine group refers to amine substituted with aryl, the alkylamine group refers to amine substituted with alkyl, and the arylheteroarylamine group refers to an amine substituted with aryl and heteroaryl. For example, the arylamine group includes a substituted or unsubstituted monoarylamine group, a substituted or unsubstituted diarylamine group, or a substituted or unsubstituted triarylamine group. The aryl group and the heteroaryl group in the arylamine group and the arylheteroarylamine group may be a monocyclic aryl group or a monocyclic heteroaryl group, or a polycyclic aryl group or a polycyclic heteroaryl group. The arylamine group and the arylheteroarylamine group that contain two or more aryl groups and two or more heteroaryl groups, respectively, include a monocyclic aryl group (heteroaryl group), a polycyclic aryl group (heteroaryl group), or both of the monocyclic aryl group (heteroaryl group) and the polycyclic aryl group (heteroaryl group). In addition, the aryl group and the heteroaryl group in the arylamine group and the arylheteroarylamine group may be selected from examples of aryl groups and heteroaryl groups described above.

As used herein, examples of the aryl group in the aryloxy group and the arylthioxy group are the same as examples of the aryl group described above and specifically, examples of the aryloxy group include a phenoxy group, a p-tolyloxy group, an m-tolyloxy group, a 3,5-dimethylphenoxy group, a 2,4,6-trimethylphenoxy group, a p-tert-butylphenoxy group, a 3-biphenyloxy group, a 4-biphenyloxy group, a 1-naphthyloxy group, a 2-naphthyloxy group, a 4-methyl-1-naphthyloxy group, a 5-methyl-2-naphthyloxy group, a 1-anthryloxy group, a 2-anthryloxy group, a 9-anthryloxy group, a 1-phenanthryloxy group, a 3-phenanthryloxy group, a 9-phenanthryloxy group, and the like, and examples of the arylthioxy group include, but are not limited to, a phenylthioxy group, a 2-methylphenylthioxy group, a 4-tert-butylphenylthioxy group, and the like.

In the present invention, examples of the halogen group include fluorine, chlorine, bromine, and iodine.

More specifically, the compound represented by [Formula A] or [Formula B] according to the present invention is selected from compounds represented by the following formulas, which clearly show specific substituents, but these compounds should not be construed as limiting the scope of [Formula A] or [Formula B] according to the present invention.

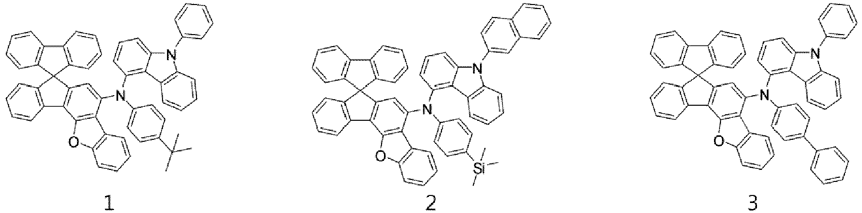

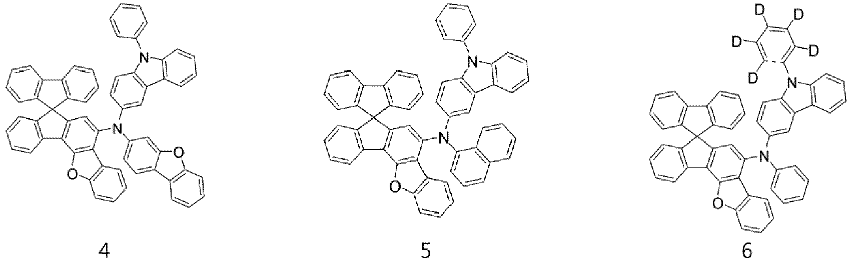

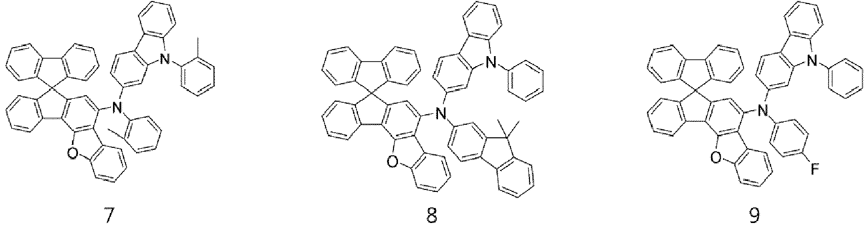

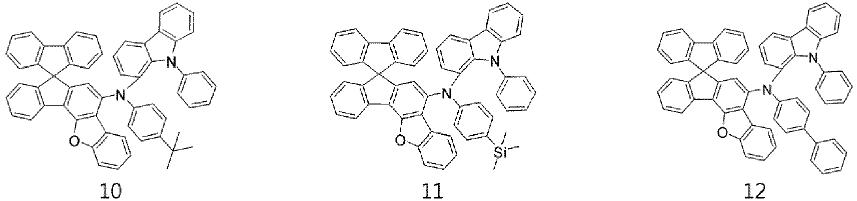

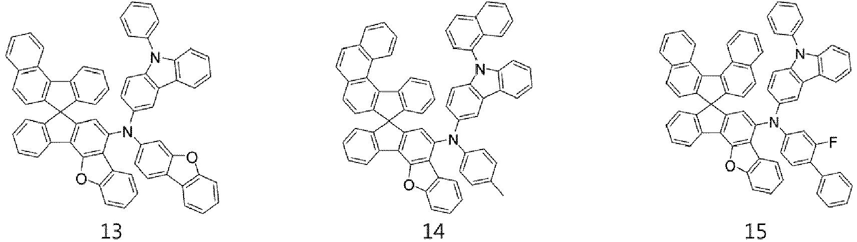

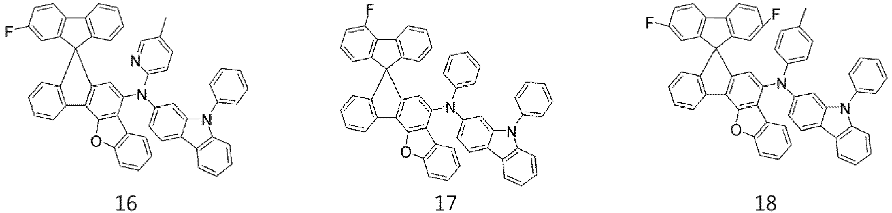

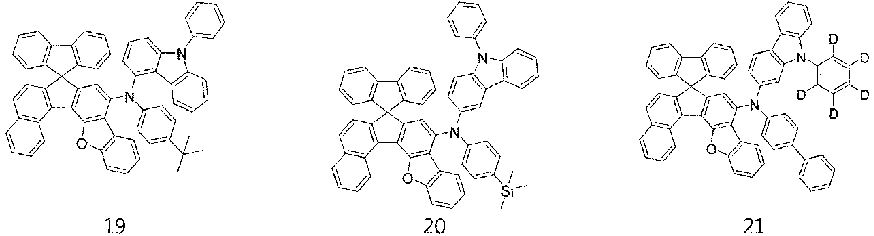

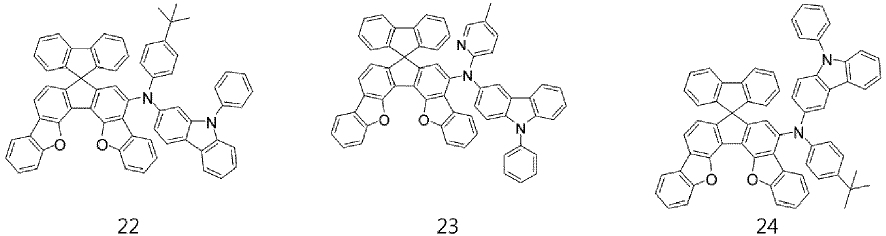

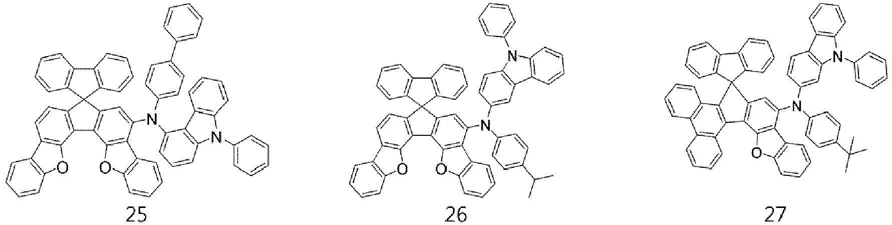

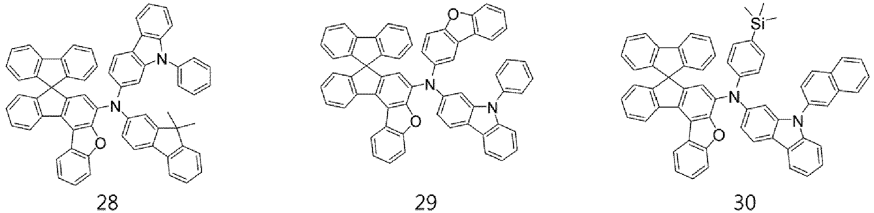

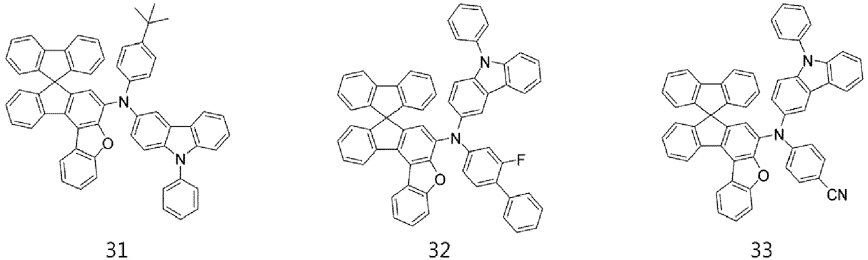

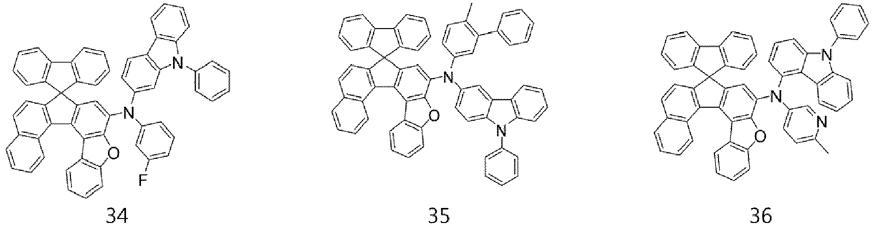

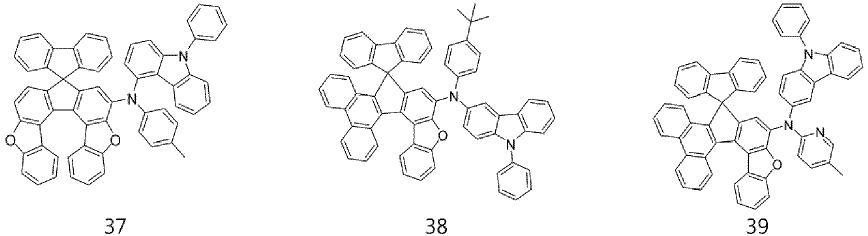

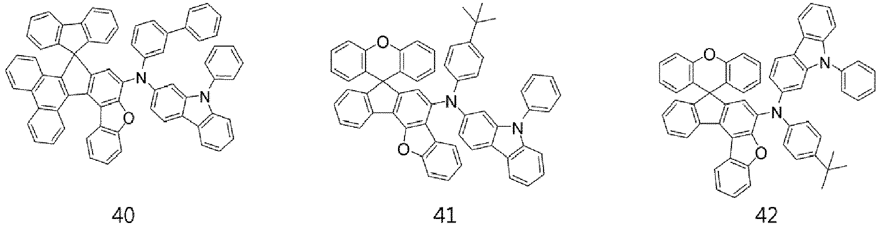

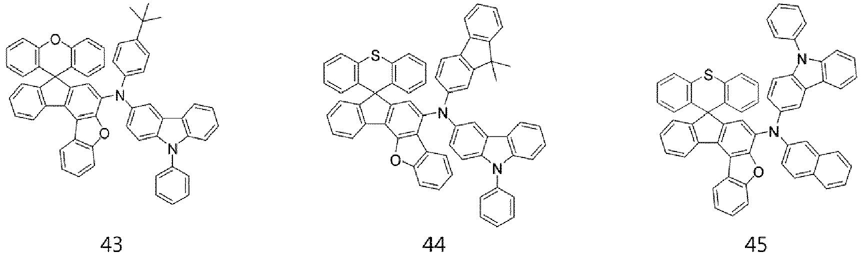

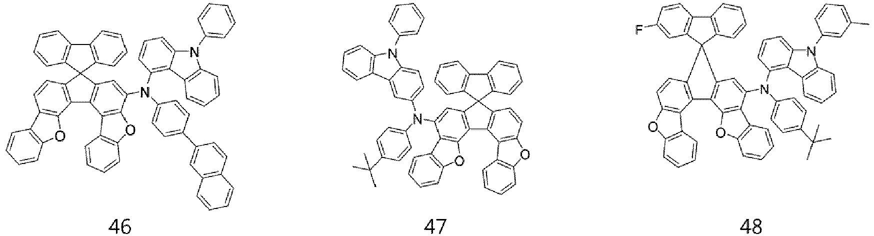

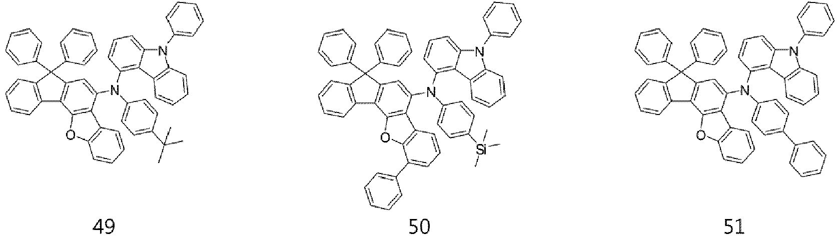

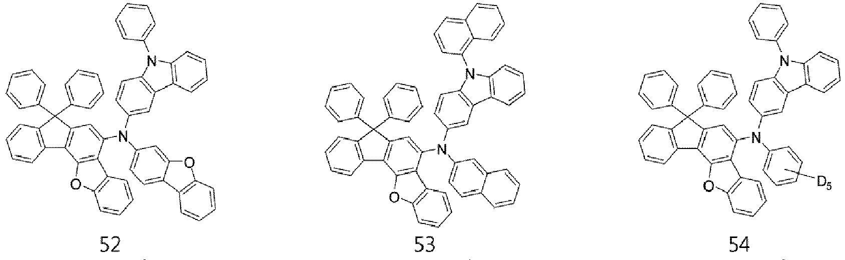

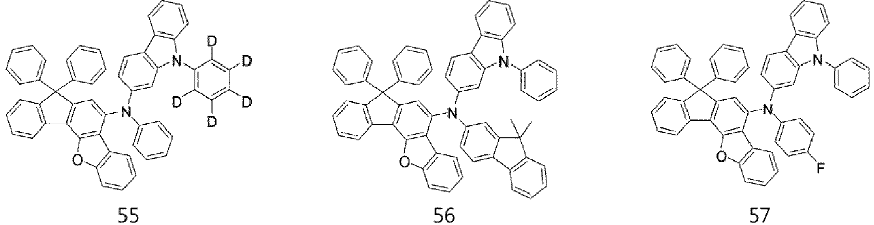

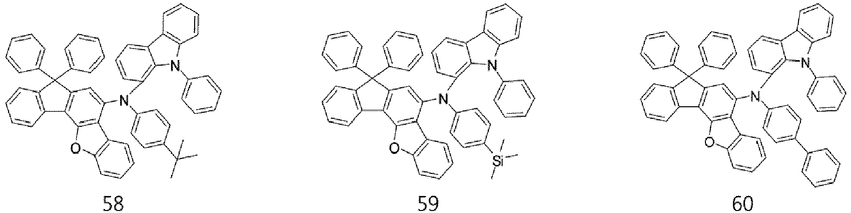

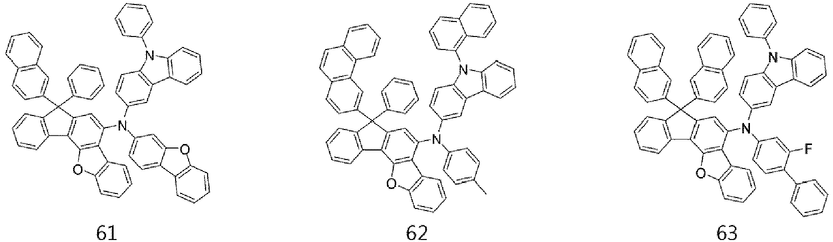

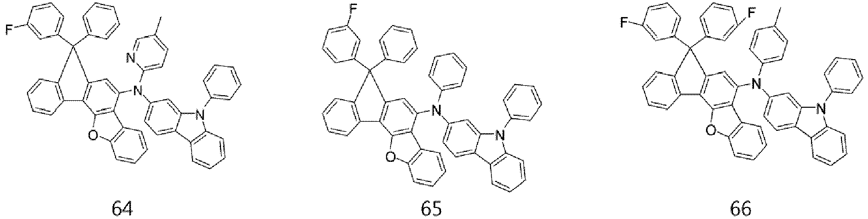

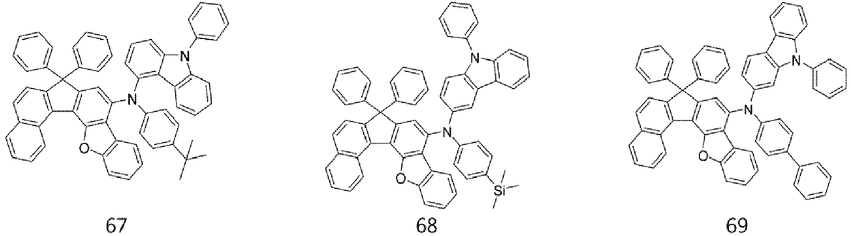

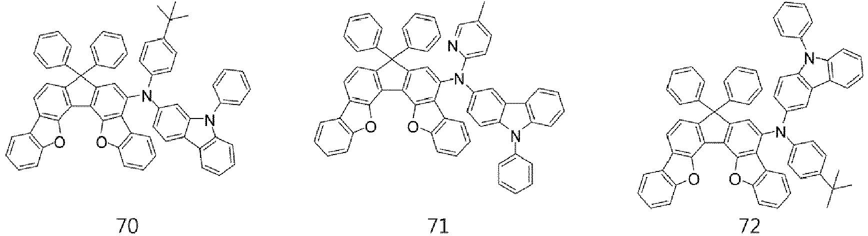

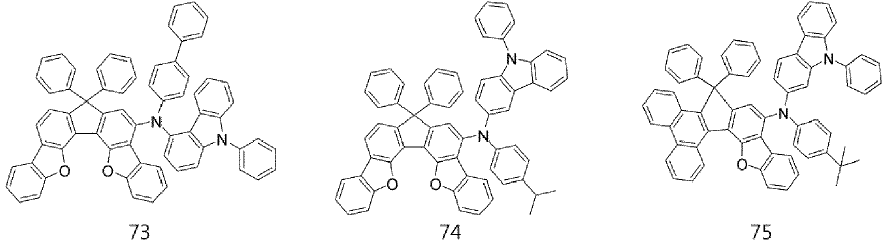

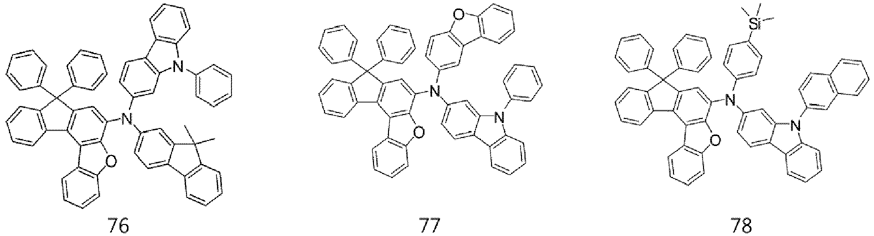

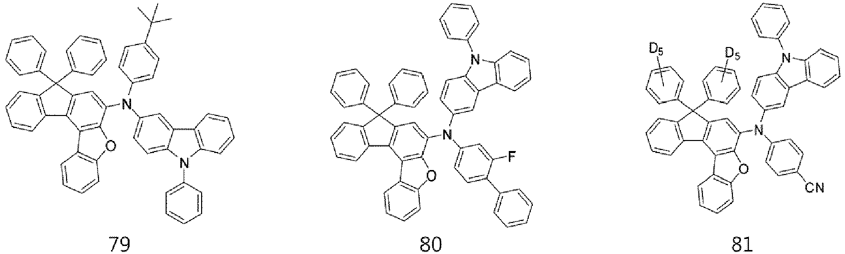

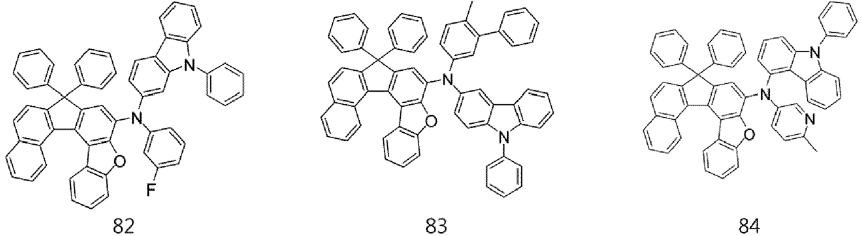

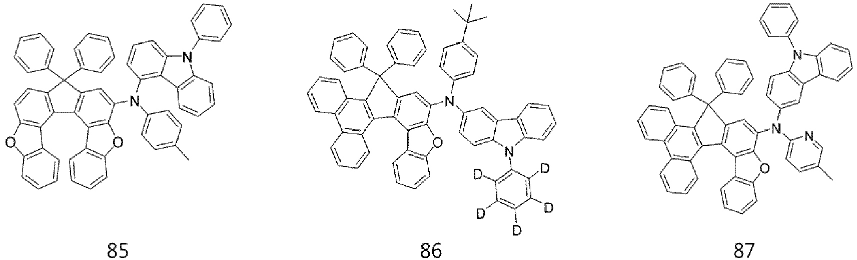

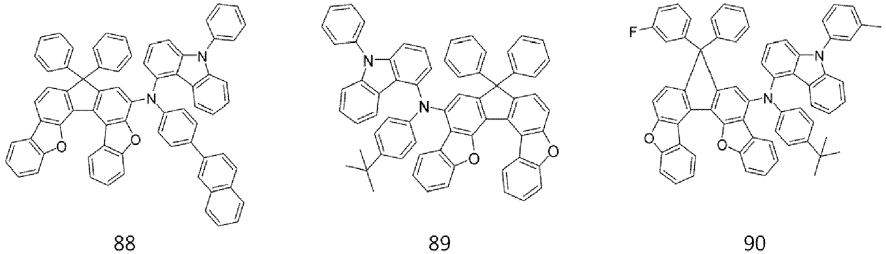

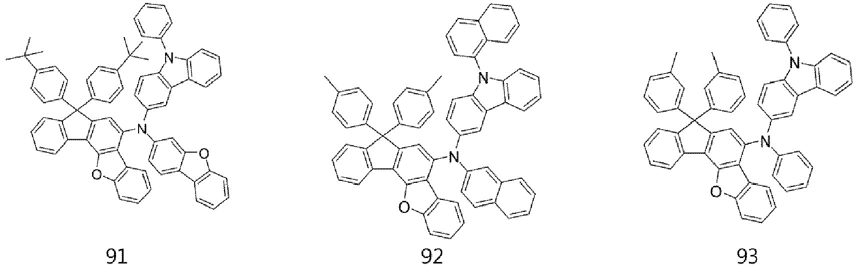

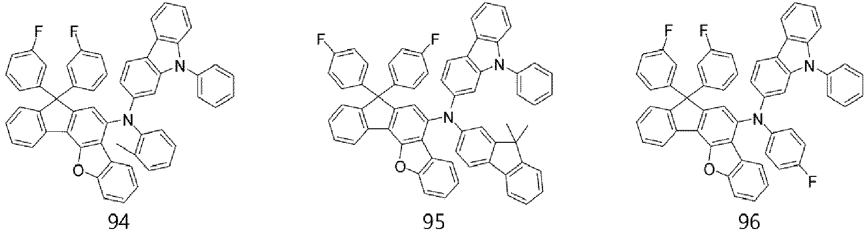

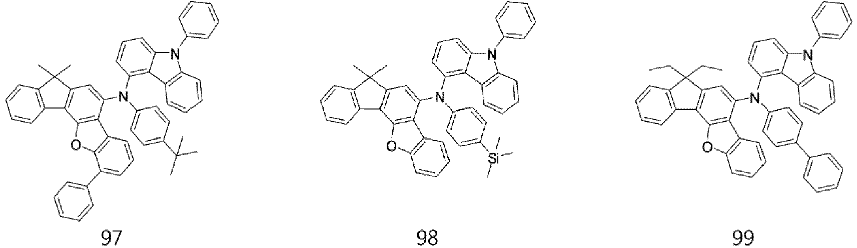

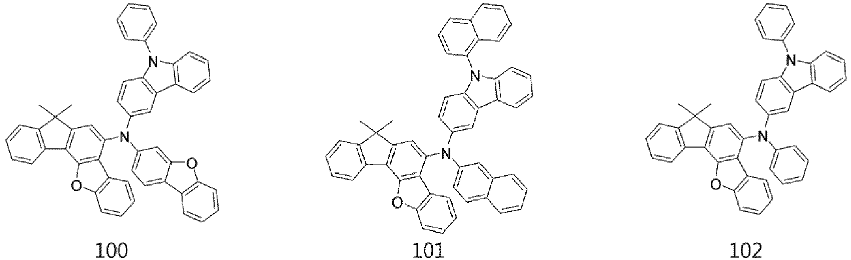

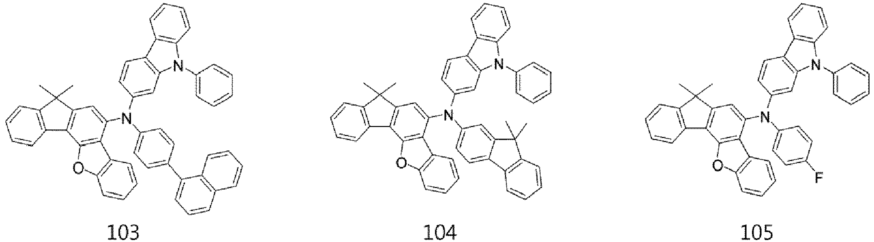

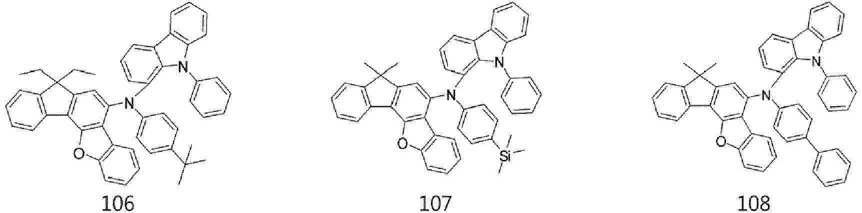

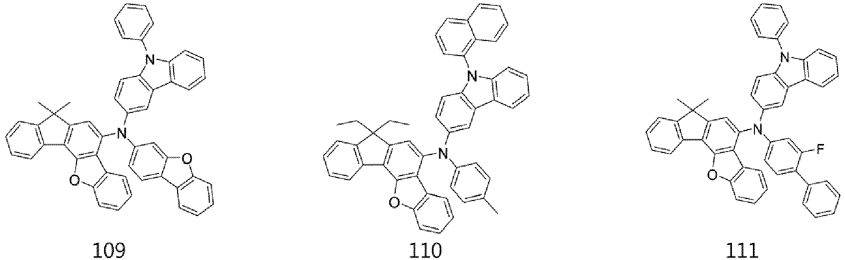

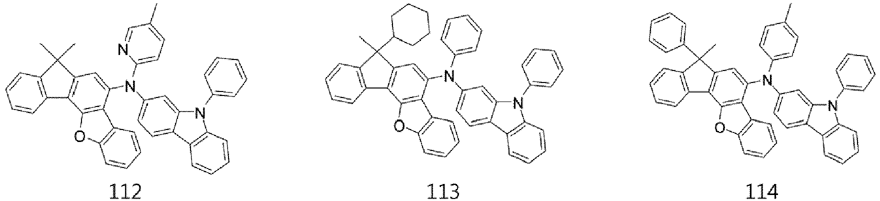

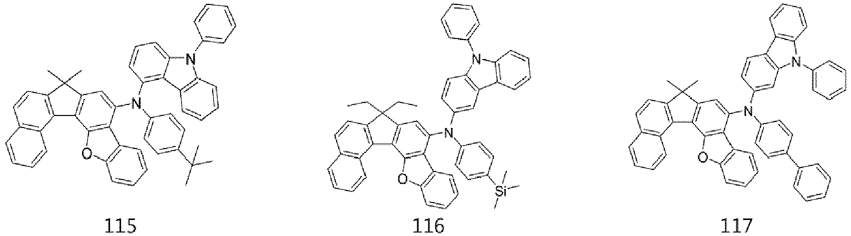

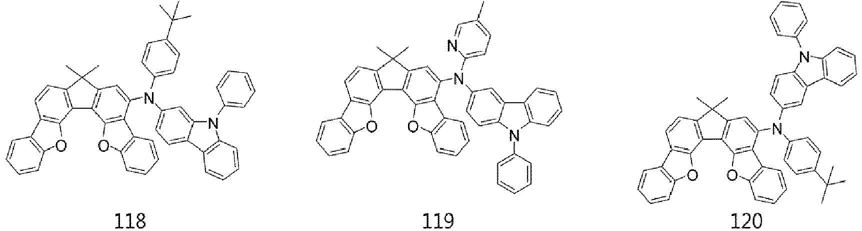

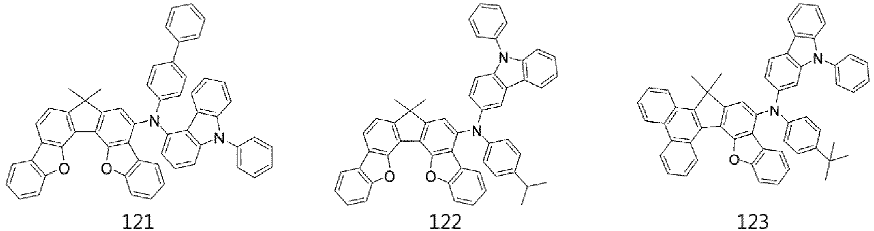

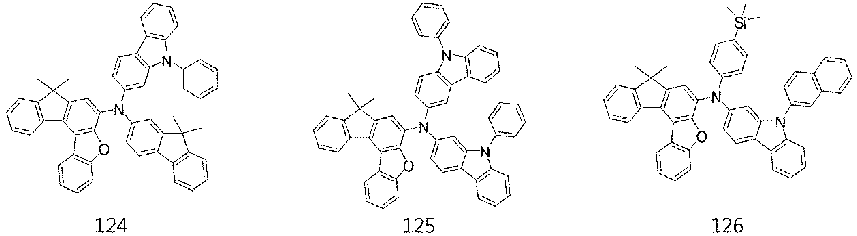

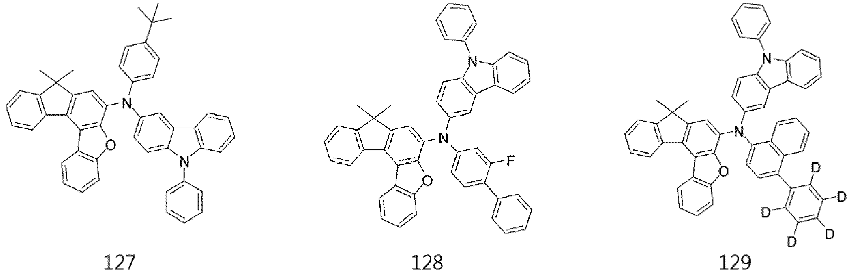

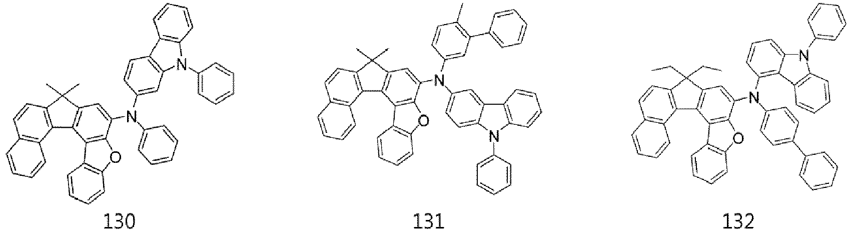

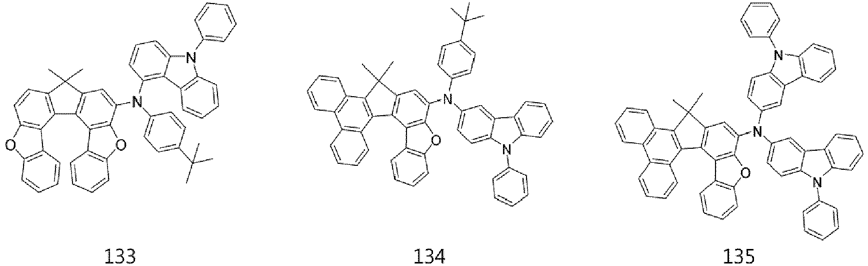

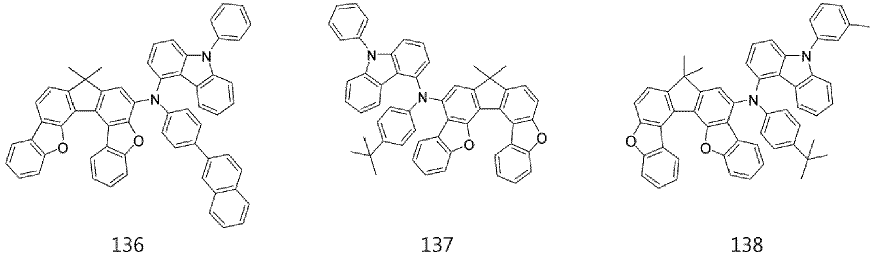

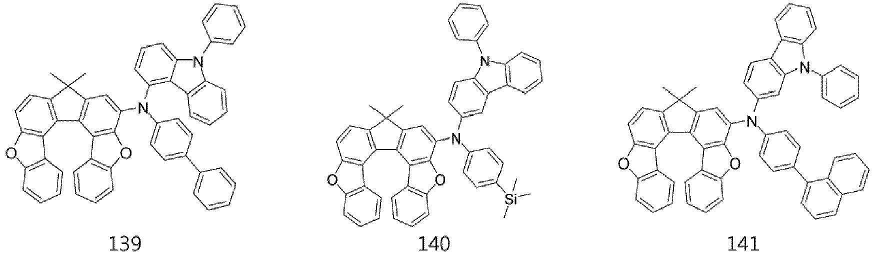

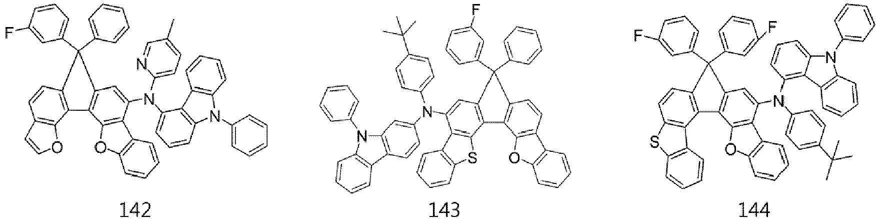

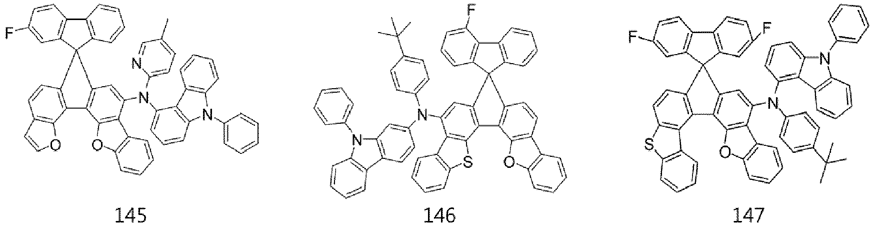

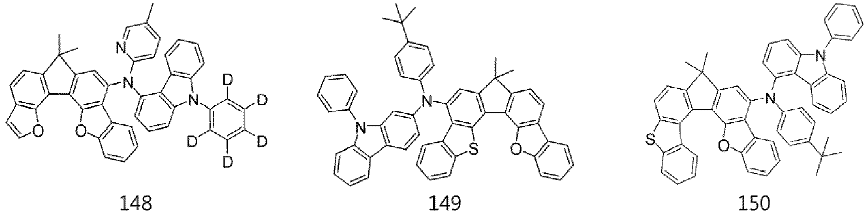

As can be seen from the specific compounds, the compound represented by [Formula A] or [Formula B] according to the present invention has a structure in which various fused ring types of skeletons are formed and an amine substituent having a characteristic structure is introduced into the skeletons. As such, it is possible to synthesize an organic light-emitting material having intrinsic characteristics of the skeleton and substituent and realize a highly efficient organic light-emitting device based thereon.

In addition, in another aspect, the present invention is directed to an organic light-emitting device including a first electrode, a second electrode, and at least one organic layer interposed between the first electrode and the second electrode, wherein the organic layer includes at least one organic light-emitting compound represented by [Formula A] or [Formula B] according to the present invention.

The organic layer includes at least one of an electron injection layer, an electron transport layer, a hole injection layer, a hole transport layer, an electron blocking layer, a hole blocking layer, and a light-emitting layer, wherein at least one of these layers includes the organic light-emitting compound represented by [Formula A] or [Formula B].

According to an embodiment of the present invention, the hole transport layer or the electron blocking layer includes the organic light-emitting compound represented by [Formula A] or [Formula B].

That is, the organic light-emitting device according to an embodiment of the present invention may have a structure including a first electrode, a second electrode and at least one organic layer disposed therebetween, and the organic light-emitting device may be fabricated using a conventional method and materials for manufacturing devices.

The organic light-emitting device according to the present invention may have a structure including a first electrode, a second electrode and an organic layer disposed therebetween, and the organic layer of the organic light-emitting device according to the present invention may have a single layer structure or a multilayer structure in which two or more organic layers are stacked. For example, the organic layer may have a structure including a hole injection layer, a hole transport layer, a hole blocking layer, a light-emitting layer, an electron blocking layer, an electron transport layer, an electron injection layer, and the like. However, the structure of the organic layer is not limited thereto and may include a smaller or larger number of organic layers, and the preferred organic material layer structure of the organic light-emitting device according to the present invention will be described in more detail in Example which will be described later.

Hereinafter, an embodiment of the organic light-emitting device according to the present invention will be described in more detail.

The organic light-emitting device according to the present invention includes an anode, a hole transport layer, a light-emitting layer, an electron transport layer and a cathode, and if necessary, may further include a hole injection layer between the anode and the hole transport layer, may further include an electron injection layer between the electron transport layer and the cathode, may further include one or two intermediate layers, and may further include a hole blocking layer or an electron blocking layer. As described above, the organic light-emitting device may further include an organic layer, such as a capping layer, having various functions depending on characteristics thereof.

The organic light-emitting device according to the present invention may include an anthracene derivative represented by the following [Formula C] as a host compound in the light-emitting layer. 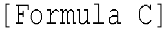 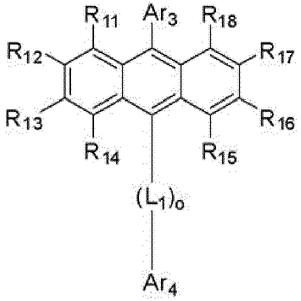 wherein
R₁₁ to R₁₈ are identical to or different from each other, and are each independently hydrogen, deuterium, a substituted or unsubstituted C1-C30 alkyl group, a substituted or unsubstituted C6-C50 aryl group, a substituted or unsubstituted C2-C50 heteroaryl group, a substituted or unsubstituted C1-C30 alkylsilyl group, a substituted or unsubstituted C5-C30 arylsilyl group, a cyano group, and a halogen group,
Ar₃ and Ar₄ are identical to or different from each other, and are each independently selected from a substituted or unsubstituted C6-C50 aryl group and a substituted or unsubstituted C2-C50 heteroaryl group,
L₁ is a single bond, or is selected from a substituted or unsubstituted C6-C20 arylene group and a substituted or unsubstituted C2-C20 heteroarylene group, and o is an integer from 1 to 3, provided that, when o is 2 or more, L₁'s are identical to or different from each other.

In addition, in an embodiment of the present invention, the compound of [Formula C] may be represented by the following [Formula C-1]: 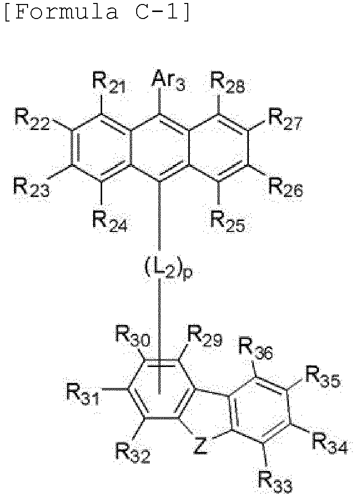 wherein
R₂₁ to R₃₆ are identical to or different from each other, and are each independently hydrogen, deuterium, a substituted or unsubstituted C1-C30 alkyl group, a substituted or unsubstituted C3-C30 cycloalkyl group, a substituted or unsubstituted C6-C50 aryl group, a substituted or unsubstituted C2-C50 heteroaryl group, a substituted or unsubstituted C1-C30 alkylsilyl group, a substituted or unsubstituted C5-C30 arylsilyl group, a cyano group, and a halogen group,
Ar₃ is a substituted or unsubstituted C6-C50 aryl group or a substituted or unsubstituted C2-C50 heteroaryl group,
Z is an oxygen atom (O) or a sulfur atom (S),
L₂ is a single bond or is a substituted or unsubstituted C6-C20 arylene group or a substituted or unsubstituted C2-C20 heteroarylene group, and p is an integer from 1 to 3, provided that, when p is 2 or more, L₂'s are identical to or different from each other.

In addition, more specifically, the host compound for the light-emitting layer represented by [Formula C] according to the present invention is selected from compounds represented by the following formulas, which clearly show specific substituents, but these compounds should not be construed as limiting the scope of [Formula C] according to the present invention.

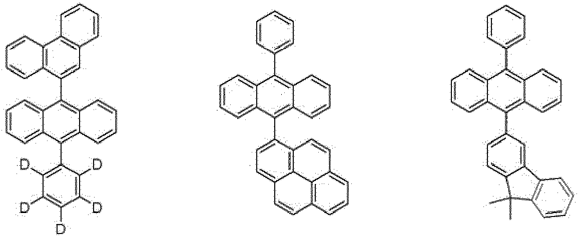

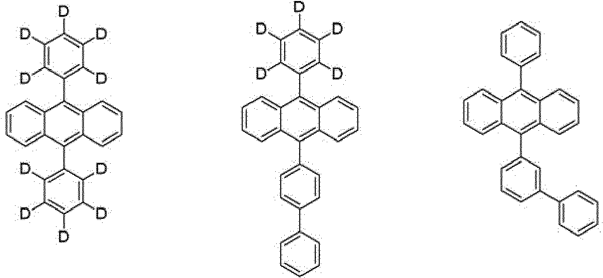

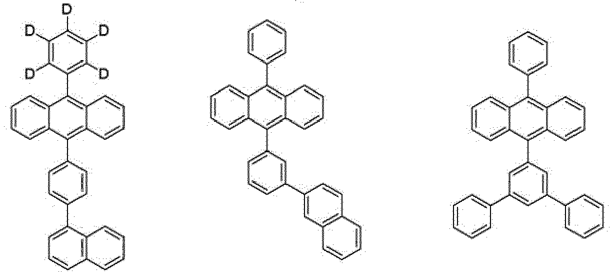

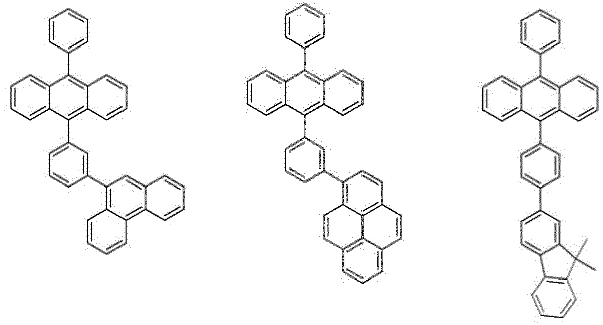

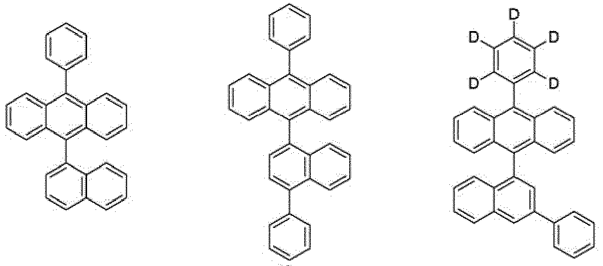

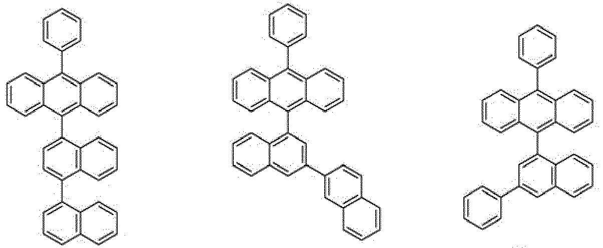

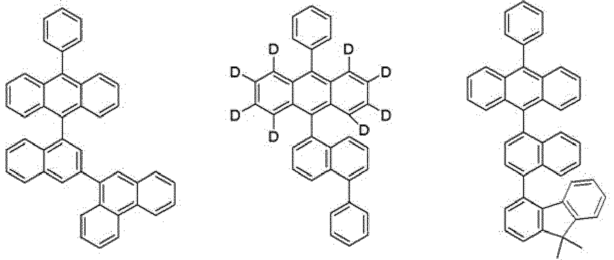

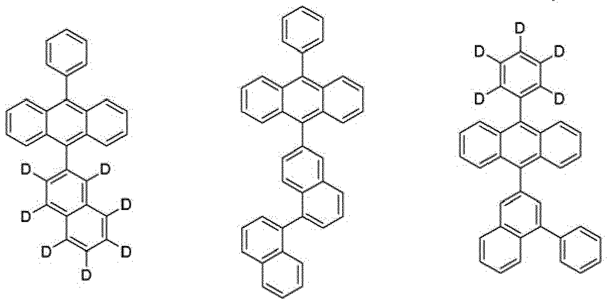

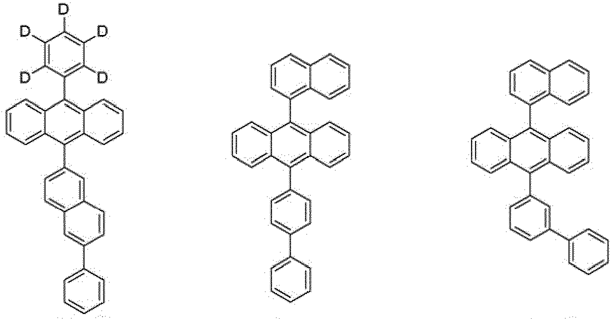

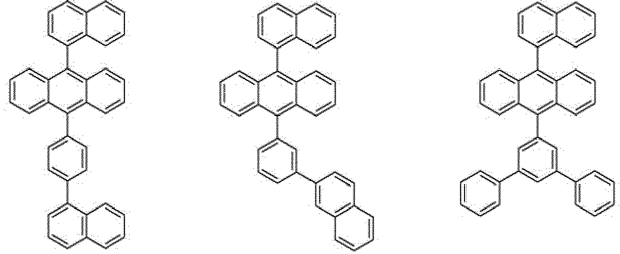

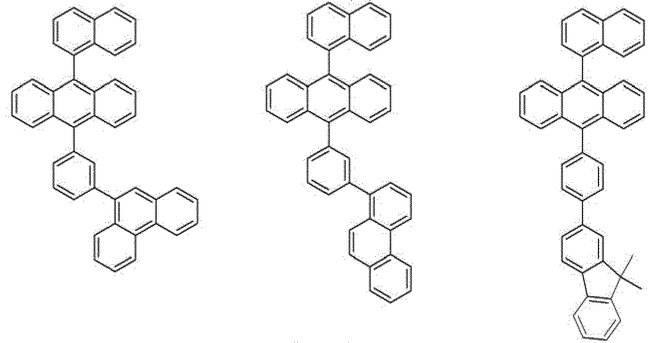

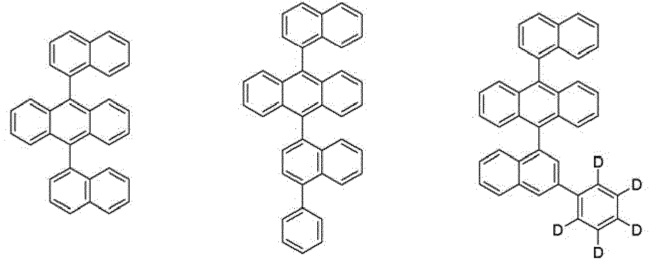

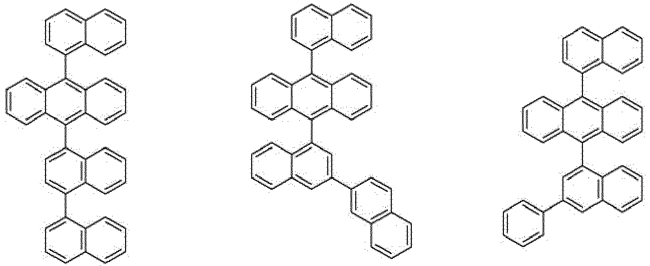

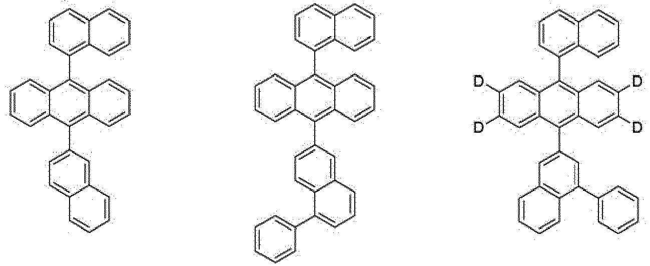

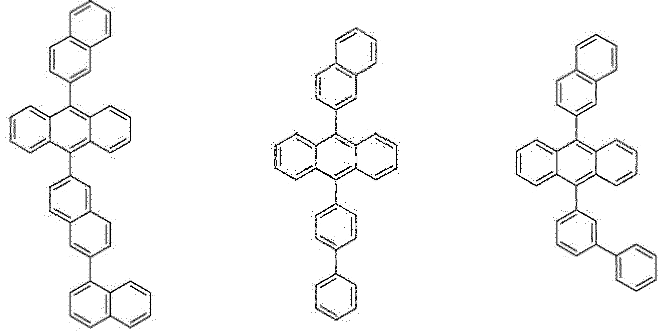

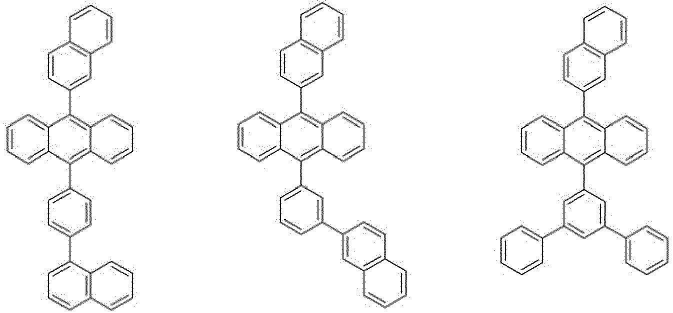

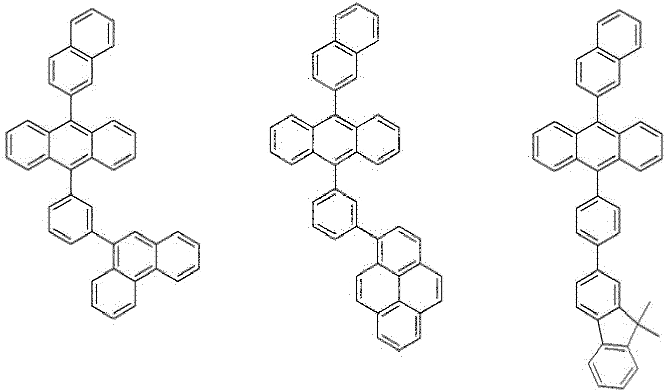

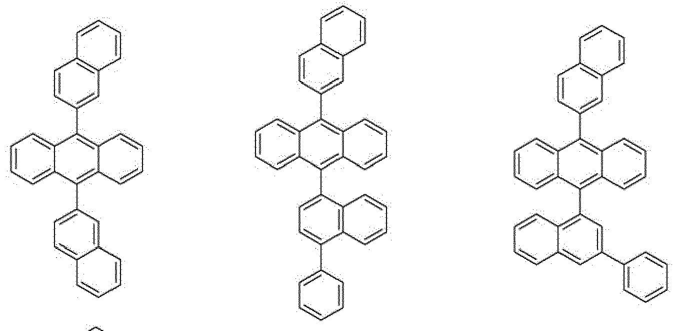

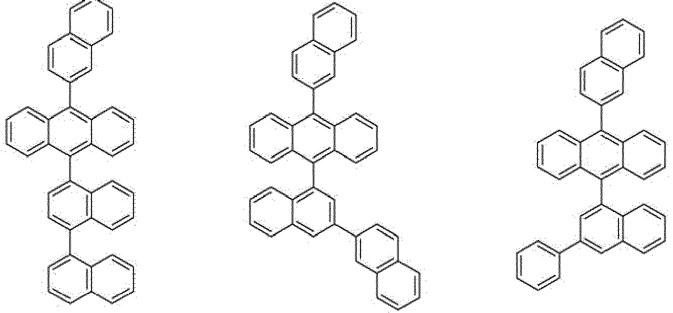

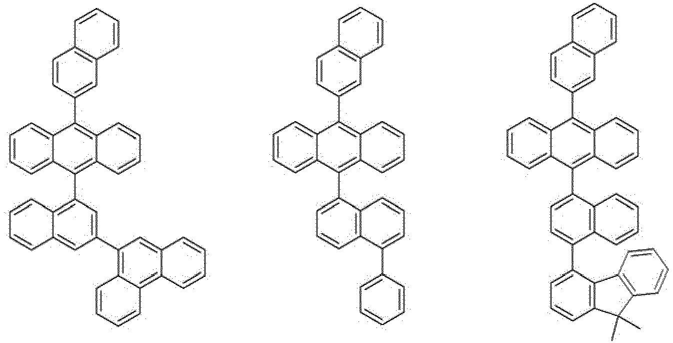

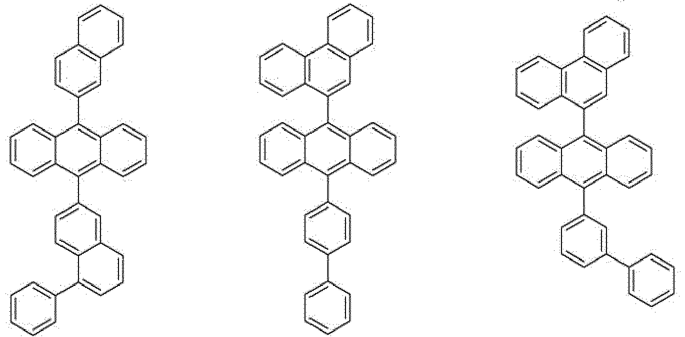

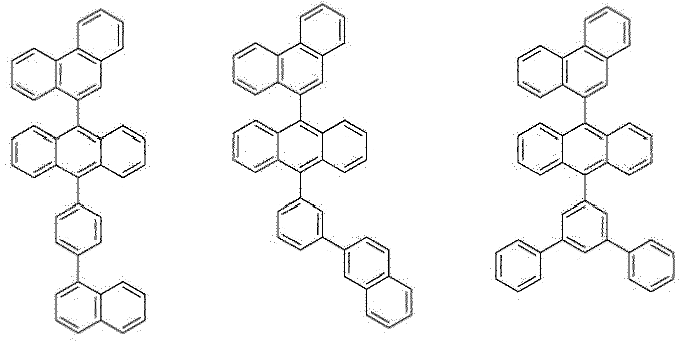

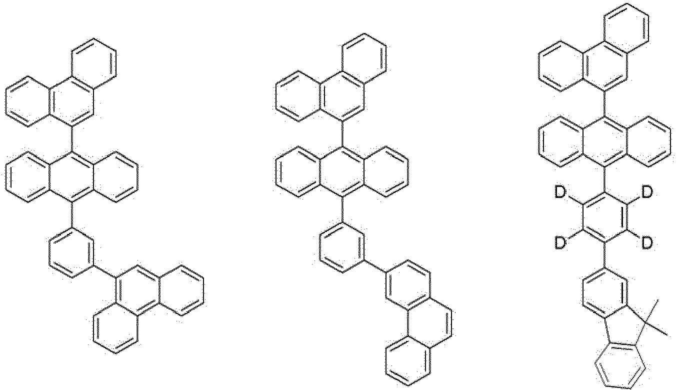

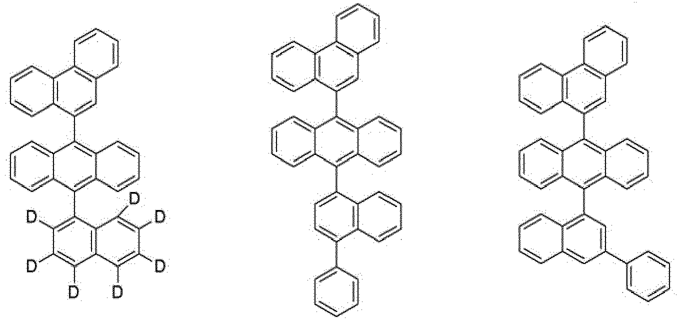

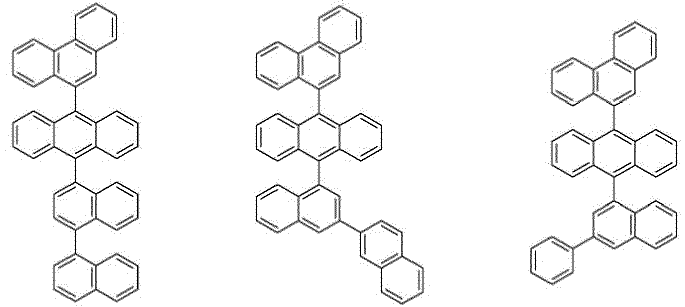

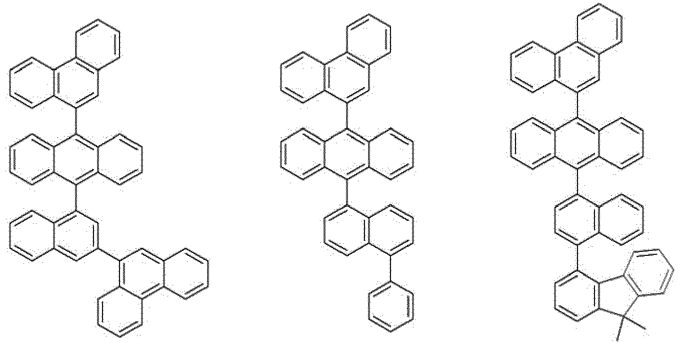

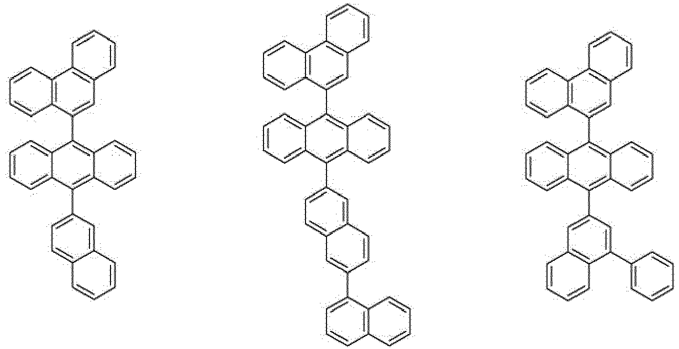

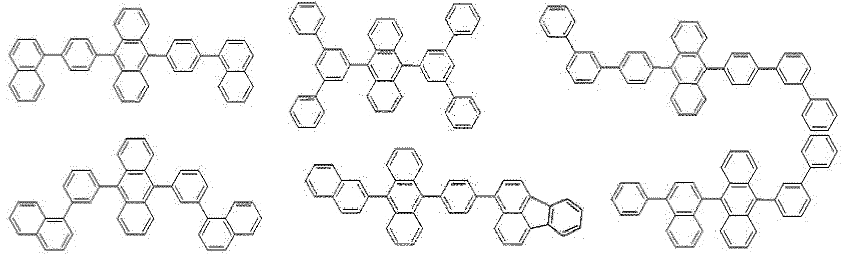

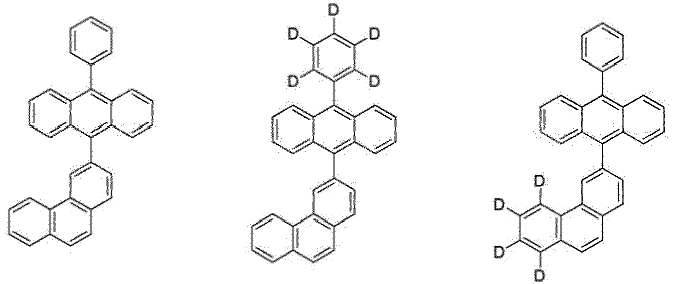

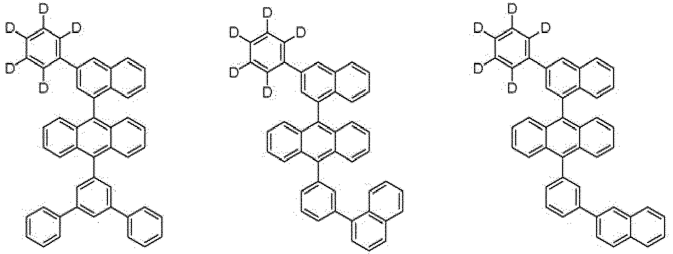

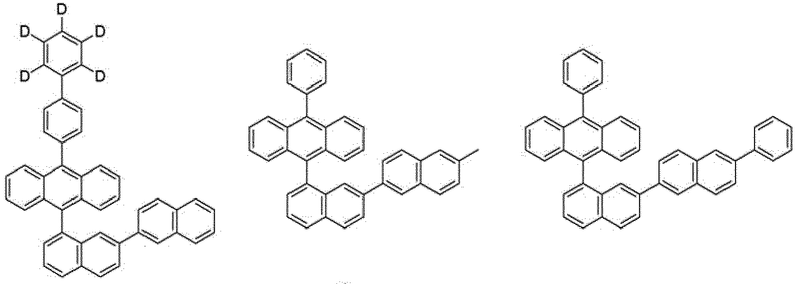

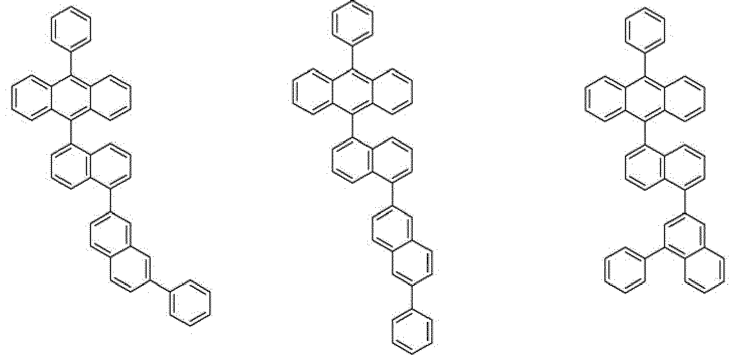

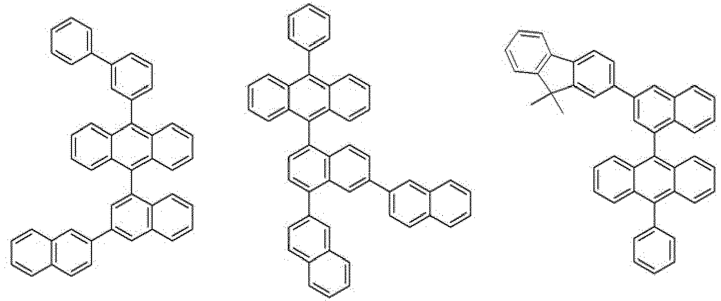

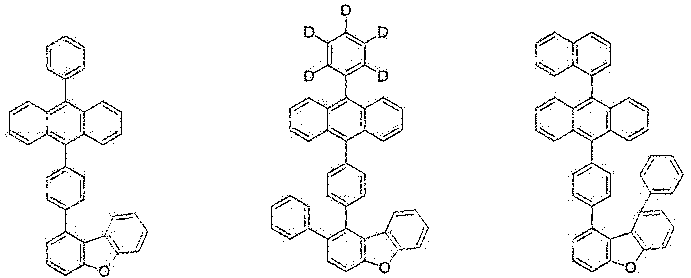

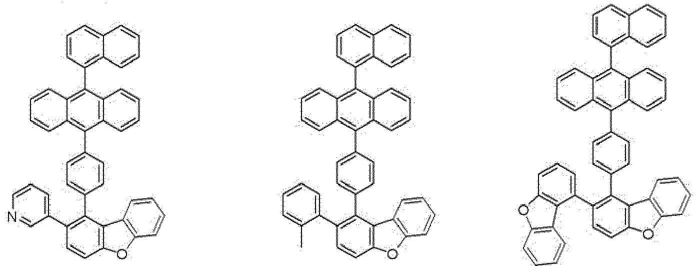

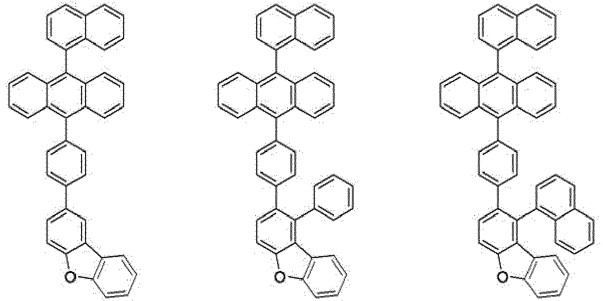

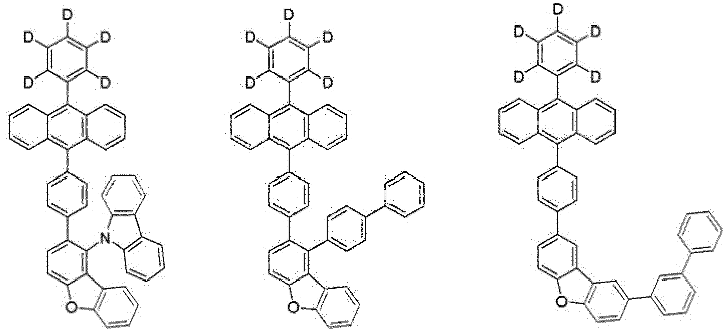

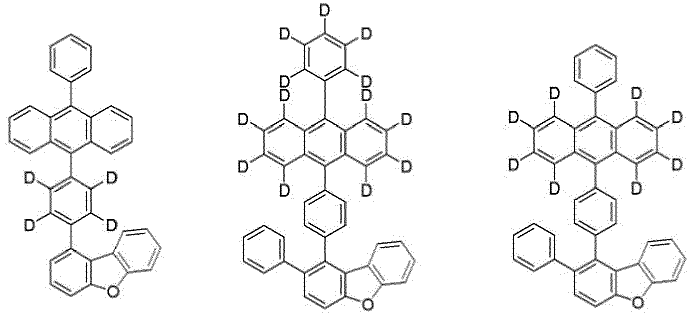

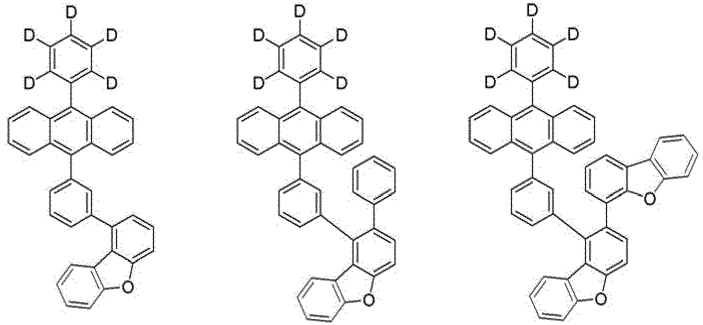

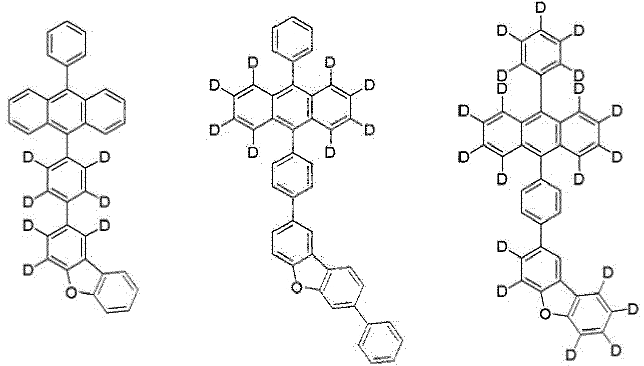

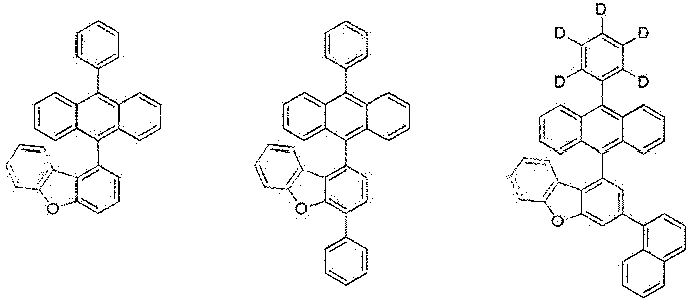

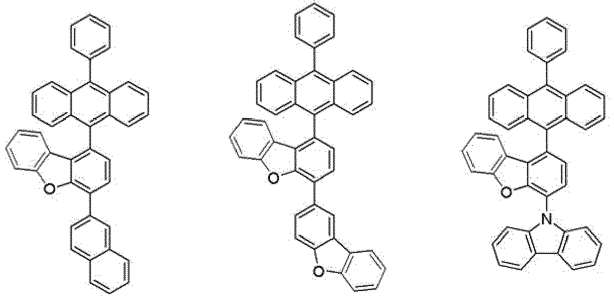

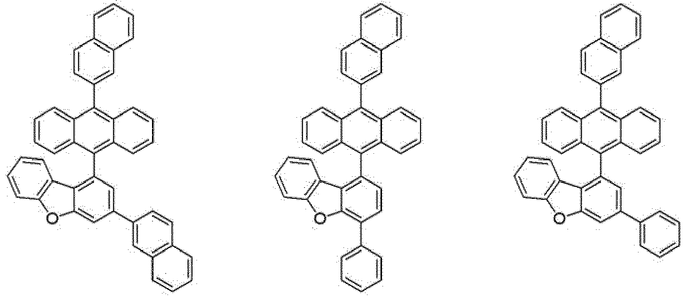

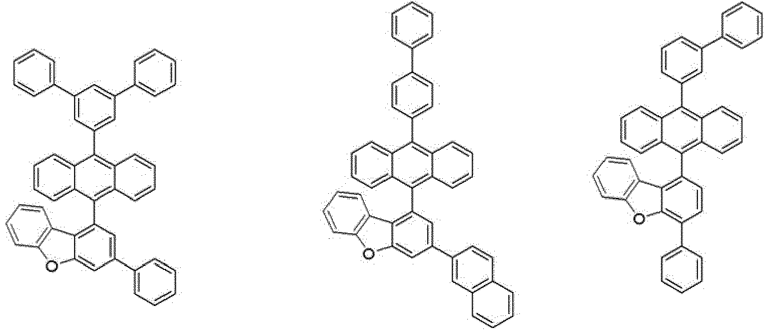

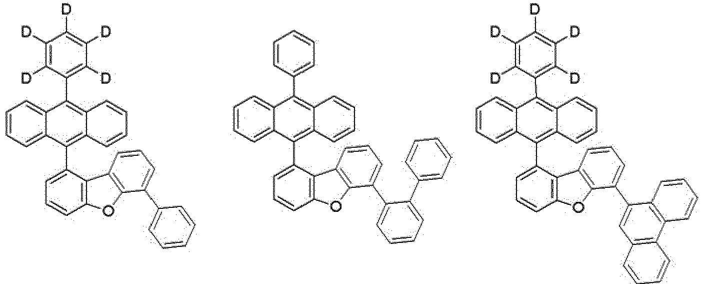

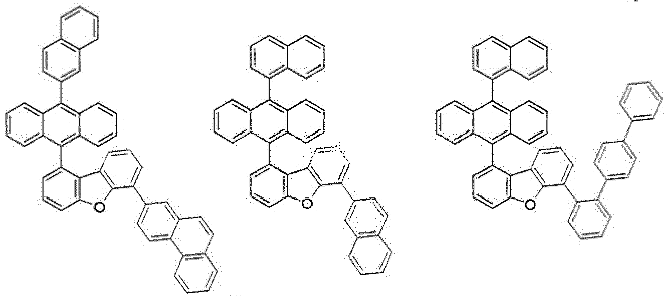

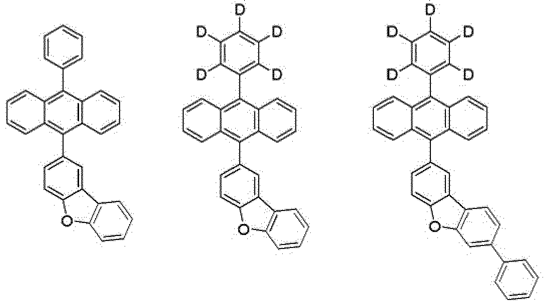

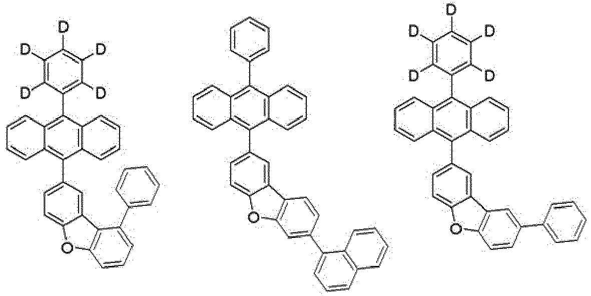

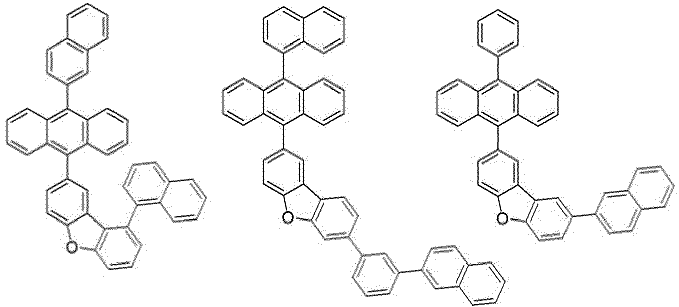

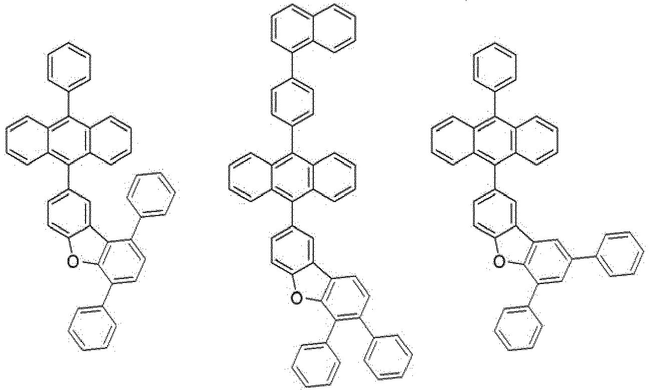

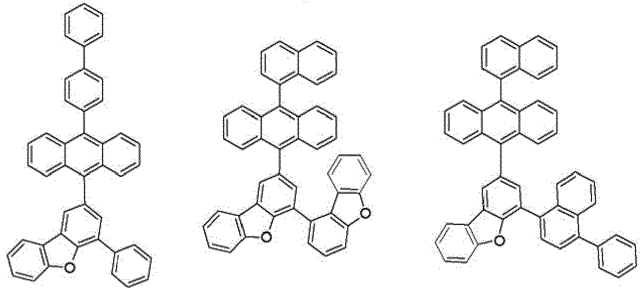

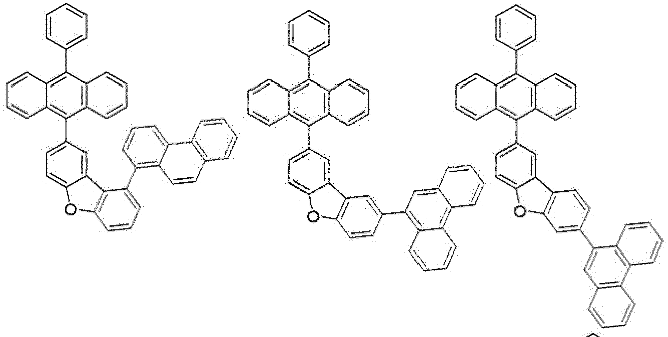

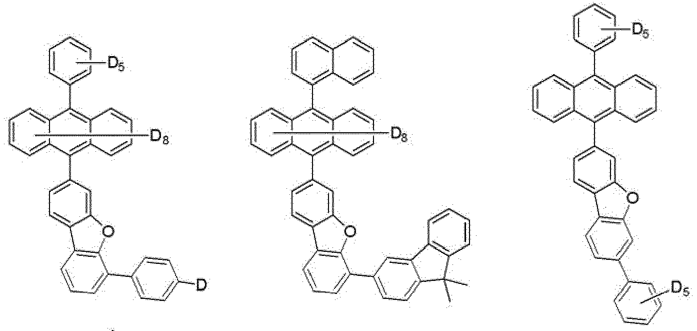

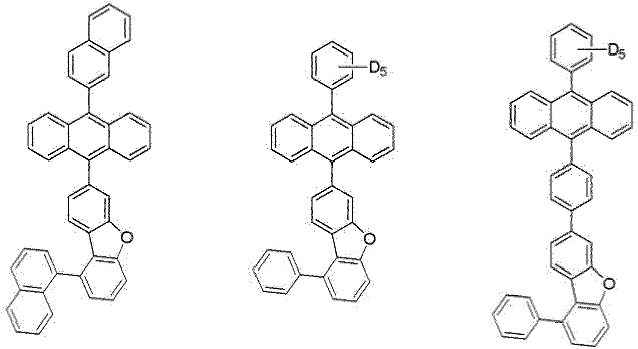

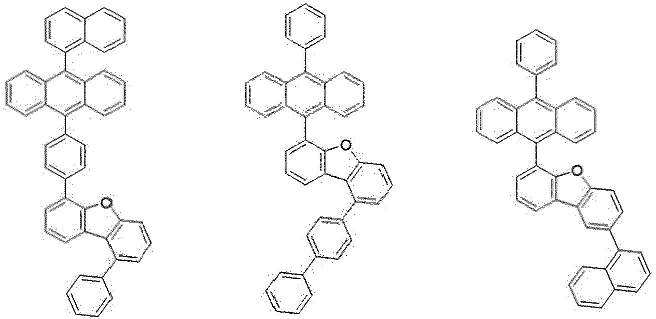

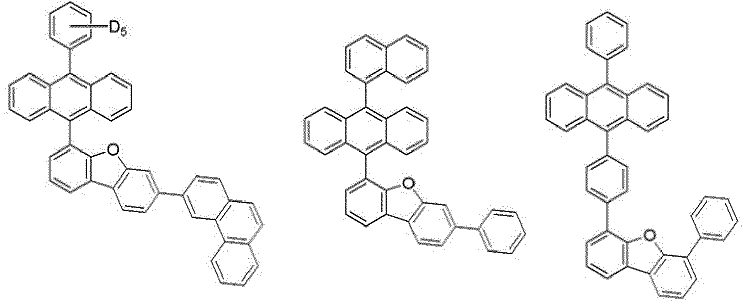

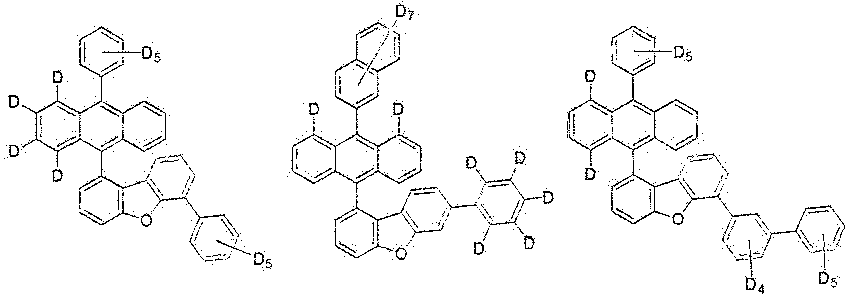

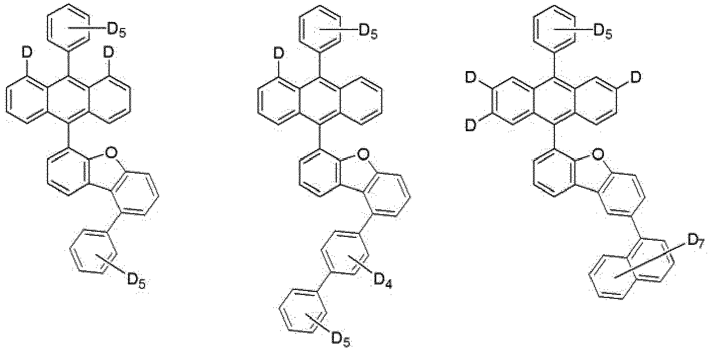

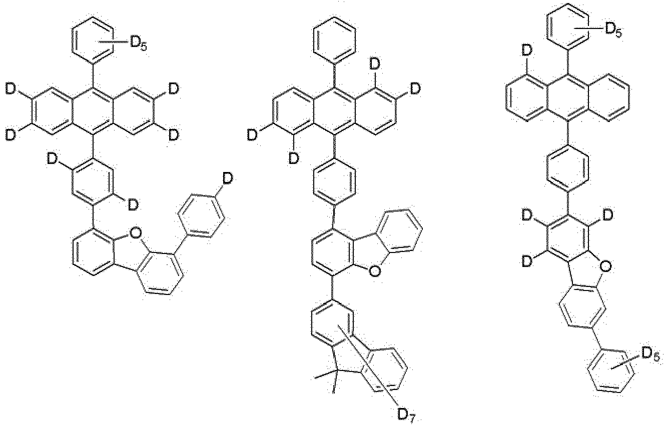

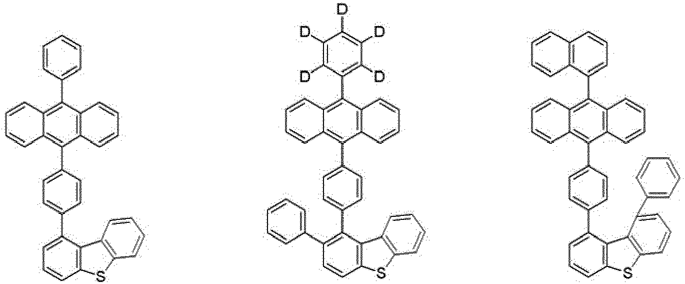

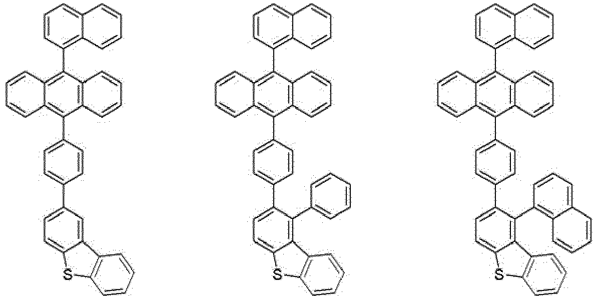

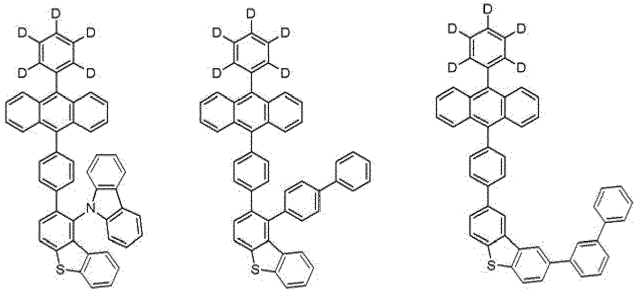

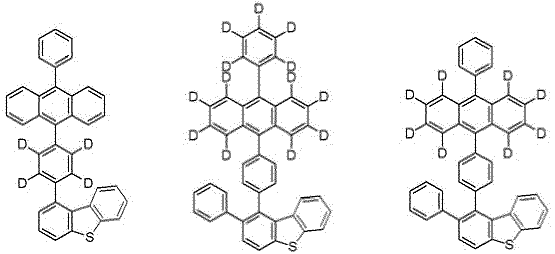

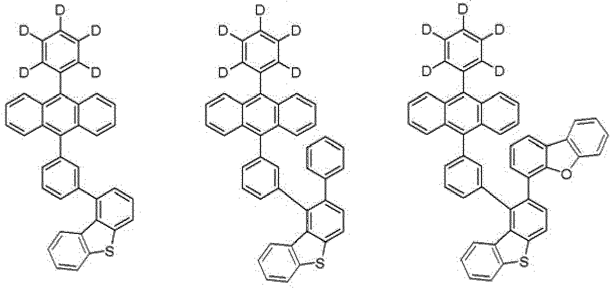

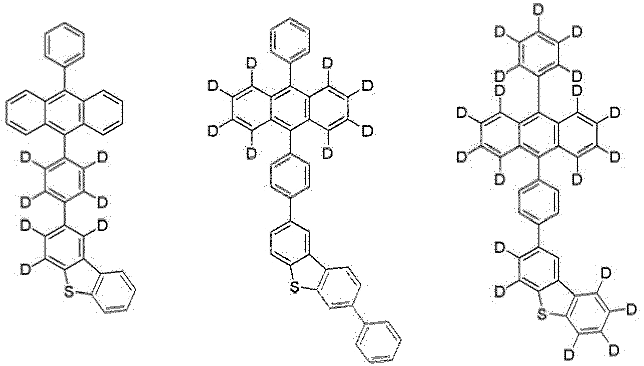

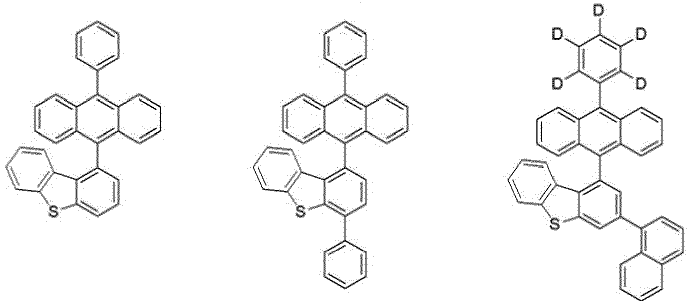

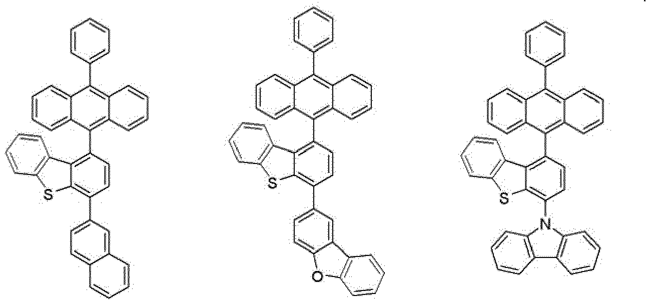

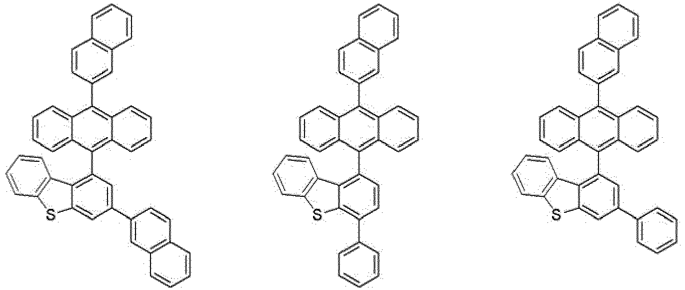

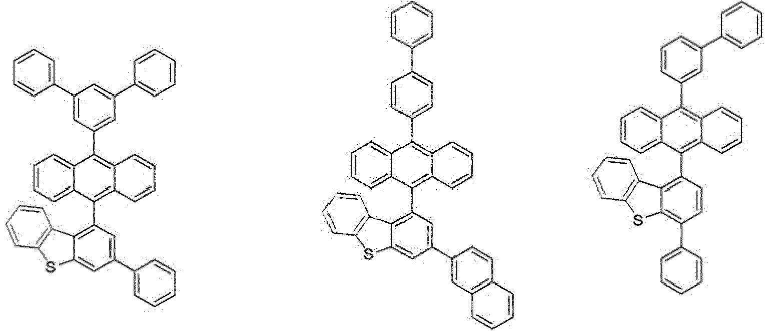

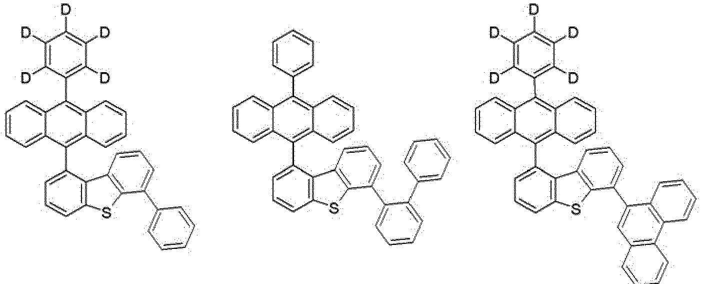

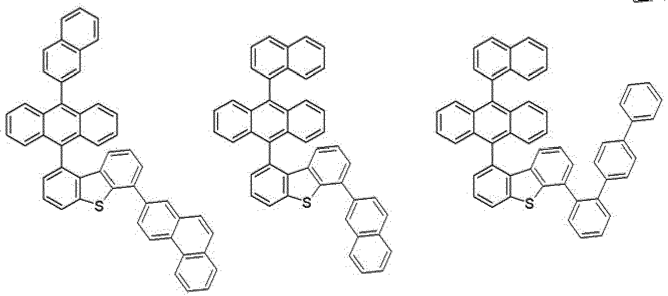

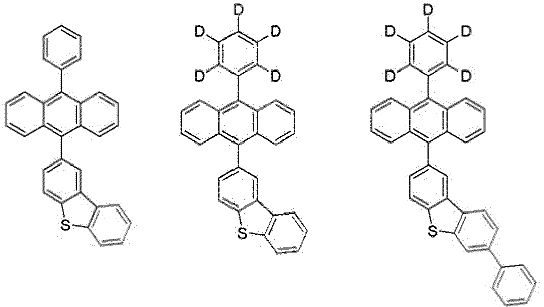

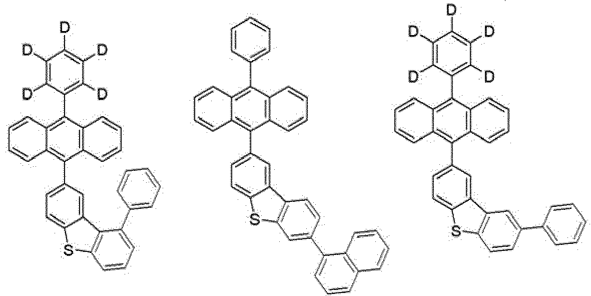

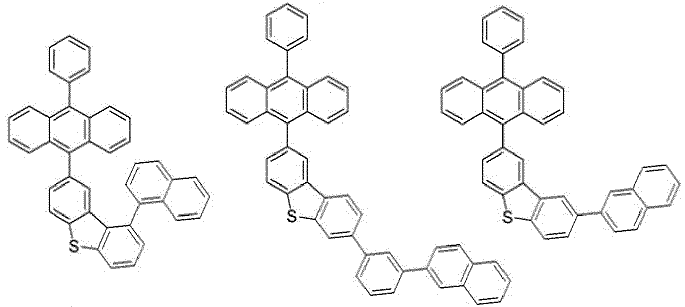

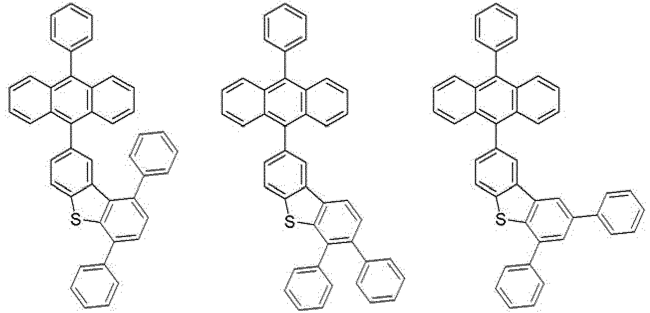

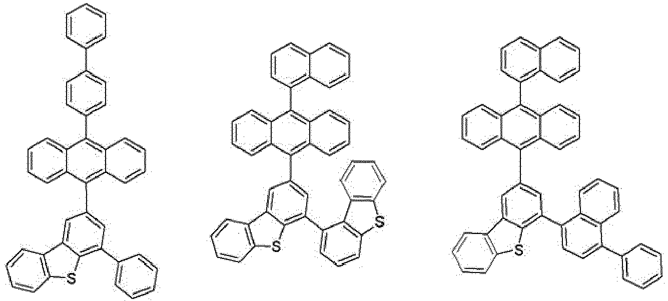

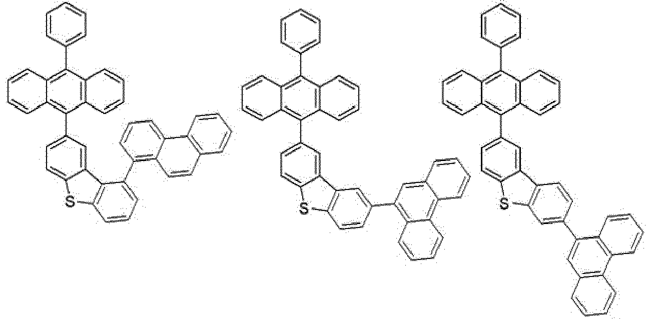

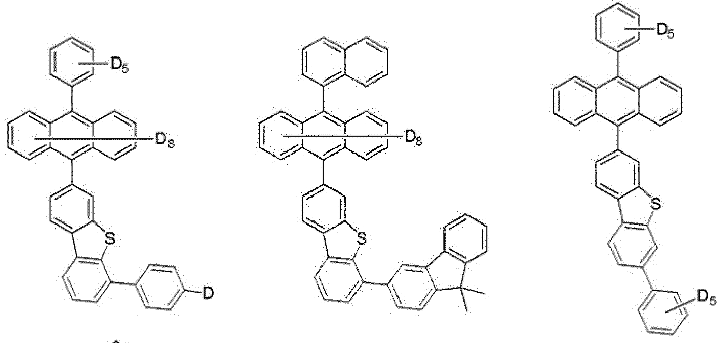

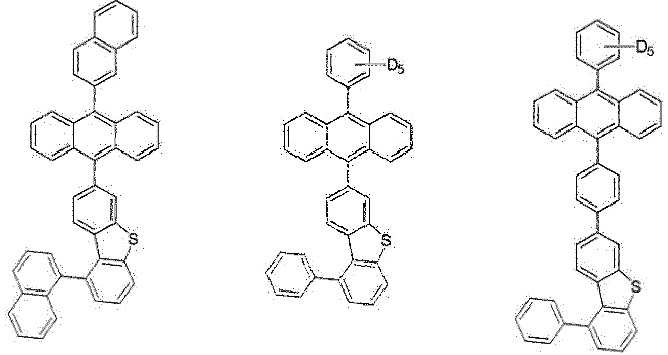

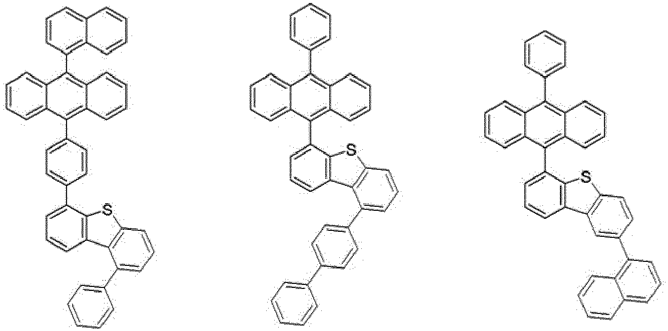

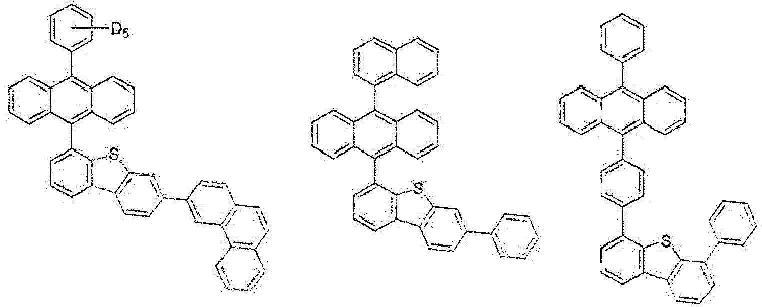

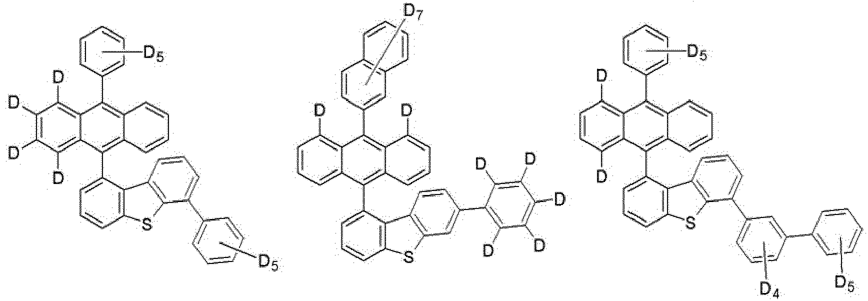

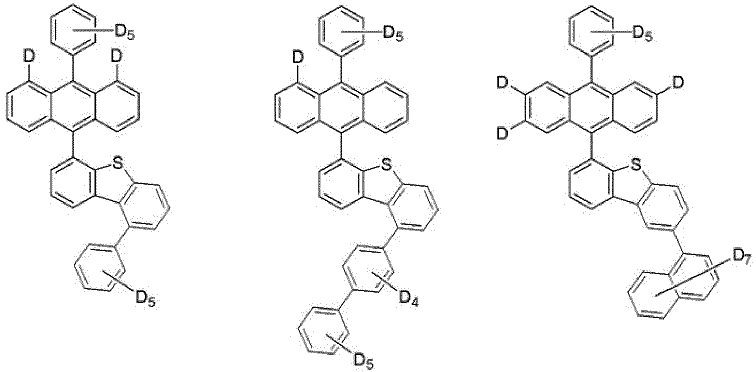

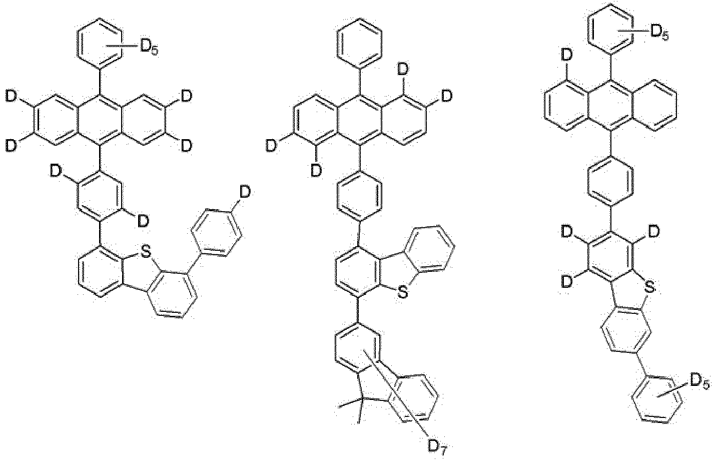

Also, in addition to the host, the light-emitting layer may further include various host materials and various dopant materials.

Meanwhile, the specific structure of the organic light-emitting device according to an embodiment of the present invention, the method of manufacturing the same, and respective organic layer materials, excluding the hole transport layer and the electron blocking layer to which the compound according to the present invention is applied, will be described as follows.

First, a substrate is coated with a material for an anode to form the anode. The substrate used herein is a substrate generally used for organic light-emitting devices and is preferably an organic substrate or a transparent plastic substrate that has excellent transparency, surface evenness, handleability and waterproofness. In addition, a material for the anode is indium tin oxide (ITO), indium zinc oxide (IZO), tin oxide (SnO₂), zinc oxide (ZnO), or the like, which is transparent and has excellent conductivity.

A hole injection layer is formed on the anode by vacuum thermal evaporation or spin coating using a material for the hole injection layer, and then a hole transport layer is formed on the hole injection layer by vacuum thermal evaporation or spin coating using a material for the hole transport layer.

The material for the hole injection layer may be used without particular limitation as long as it is commonly used in the art and specific examples thereof include 2-TNATA [4,4',4"-tris(2-naphthylphenyl-phenylamino)-triphenylamine], NPD [N,N'-di(1-naphthyl)-N,N'-diphenylbenzidine)], TPD [N,N'-diphenyl-N,N'-bis(3-methylphenyl)-1,1'-biphenyl-4,4'-diamine], DNTPD [N,N'-diphenyl-N,N'-bis-[4-(phenyl-m-tolyl-amino)-phenyl]-biphenyl-4,4'-diamine], and the like.

In addition, the material for the hole transport layer is also used without particular limitation as long as it is commonly used in the art and is, for example, N,N'-bis(3-methylphenyl)-N,N'-diphenyl-[1,1-biphenyl]-4,4'-diamine (TPD) or N,N'-di(naphthalen-1-yl)-N,N'-diphenylbenzidine (a-NPD).

Subsequently, a hole auxiliary layer and a light-emitting layer are sequentially stacked on the hole transport layer, and a hole blocking layer is selectively deposited on the light-emitting layer by vacuum deposition or spin coating to form a thin film. Because the lifetime and efficiency of the device are reduced when holes are introduced into the cathode through the organic light-emitting layer, the hole blocking layer is formed using a material having a very low HOMO (highest occupied molecular orbital) level so as to prevent this problem. The hole blocking material used herein is not particularly limited and is typically BAlq, BCP or TPBI that has an electron transport ability and has an ionization potential higher than that of a light-emitting compound.

The material used for the hole blocking layer may be BAlq, BCP, Bphen, TPBI, NTAZ, BeBq₂, OXD-7, Liq, or the like, but is not limited thereto.

An electron transport layer is deposited on the hole blocking layer through vacuum deposition or spin coating and a metal for forming a cathode is formed on the electron injection layer through vacuum thermal evaporation to form a cathode. As a result, an organic light-emitting device according to an embodiment is completed.

Here, the metal for forming the cathode may be lithium (Li), magnesium (Mg), aluminum (Al), aluminum-lithium (Al-Li), calcium (Ca), magnesium-indium (Mg-In), magnesium-silver (Mg-Ag) or the like. A transmissive cathode using ITO or IZO may be used in order to obtain a top-emission type light-emitting device.

The material for the electron transport layer functions to stably transport electrons injected from the cathode and may be a well-known electron transport material. Examples of the well-known electron transport material include quinoline derivatives, especially, tris(8-quinolinolate)aluminum (Alq3), TAZ, BAlq, beryllium bis(benzoquinolin-10-olate: Bebq2) and oxadiazole derivatives (PBD, BMD, BND, etc.).

In addition, each of the organic layers may be formed by a monomolecular deposition or solution process. The deposition is a method of forming a thin film by evaporating a material for forming each layer through heating in the presence of a vacuum or low pressure and the solution process is a method of forming a thin film by mixing a material for forming each layer with a solvent and forming the thin film from the mixture through a method such as inkjet printing, roll-to-roll coating, screen printing, spray coating, dip coating, or spin coating.

In addition, the organic light-emitting device according to the present invention may further include a light-emitting layer of a blue light-emitting material, a green light-emitting material, or a red light-emitting material that emits light in a wavelength range of 380 nm to 800 nm. That is, the light-emitting layer of the present invention includes a plurality of light-emitting layers, and a blue light-emitting material, a green light-emitting material, or a red light-emitting material in the additionally formed light-emitting layer may be a fluorescent material or a phosphorescent material.

In addition, the organic light-emitting device is used for a display or lighting system selected from flat panel displays, flexible displays, monochromatic or white flat panel lighting systems, monochromatic or white flexible lighting systems, vehicle displays, and displays for virtual or augmented reality.

### [Mode for Invention]

Hereinafter, the present invention will be described in more detail with reference to preferred examples. However, it will be obvious to those skilled in the art that these examples are merely provided for illustration of the present invention, and should not be construed as limiting the scope of the present invention.

### Synthesis Example 1. Synthesis of Formula 5

### Synthesis Example 1-(1): Synthesis of Intermediate 1-a

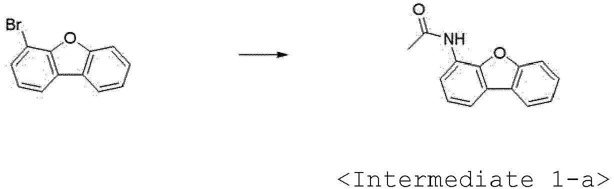

4-bromodibenzofuran (100 g, 0.405 mol), (1R,2R)-cyclohexane-1,2-diamine (46.2 g, 0.404 mol), acetamide (71.7 g, 1.21 mol), Copper (I) iodide (77.1 g, 0404 mol), potassium carbonate (200 g, 0.809 mol) and 1,000 ml of toluene were added to a round bottom flask and then stirred under reflux overnight. After the reaction was completed, the reaction product was filtered through a celite pad and washed with ethyl acetate. The filtrate was extracted with water and ethyl acetate, and the organic layer was separated. The organic layer was dehydrated with magnesium sulfate, filtered and concentrated under reduced pressure. The result was recrystallized in dichloromethane and petroleum ether to obtain 50 g of <Intermediate 1-a>. (yield 32%)

### Synthesis Example 1-(2): Synthesis of Intermediate 1-b

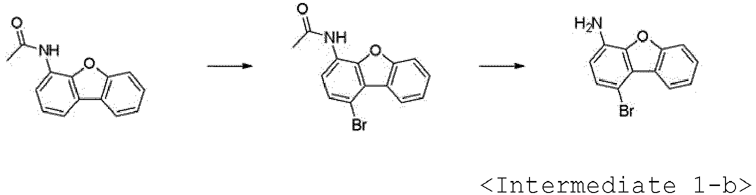

<Intermediate 1-a> (50 g, 0.222 mol) was dissolved in 600 mL of acetic acid in a round-bottom flask and stirred at room temperature. A dilution of bromine (11.37 mL, 0.222 mol) in 200 mL of acetic acid was added dropwise to the reaction solution, followed by stirring for about 4 hours. After completion of the reaction, the resulting solid was filtered and washed with water. The solid was dissolved in 1,000 mL of a tetrahydrofuran/water/ethanol (1:1:1) solution, potassium hydroxide (250 g, 1.11 mol) was added thereto, and the mixture was stirred under reflux overnight. After completion of the reaction, the solvent was concentrated under reduced pressure and extracted with ethyl acetate and water. The organic layer was separated, dehydrated with magnesium sulfate, filtered, and concentrated under reduced pressure. The result was recrystallized with ethyl acetate and heptane to obtain 40 g of <Intermediate 1-b>. (yield 68%)

### Synthesis Example 1-(3): Synthesis of Intermediate 1-c

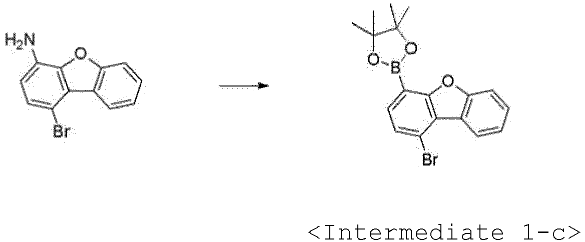

<Intermediate 1-b> (40 g, 0.153 mol), bis(pinacolato)diboron (51.7 g, 0.183 mol), and 400 ml of acetonitrile were added to a round-bottom flask and stirred at room temperature. Tert-butylnitrite (26.2 g, 0229 mol) was added portionwise to the reaction solution, followed by stirring at 80°C for 2 hours. After completion of the reaction, the reaction product was cooled to room temperature. The reaction solution was concentrated under reduced pressure and separated by column chromatography to obtain 20 g of <Intermediate 1-c>. (yield 35%)

### Synthesis Example 1-(4): Synthesis of Intermediate 1-d

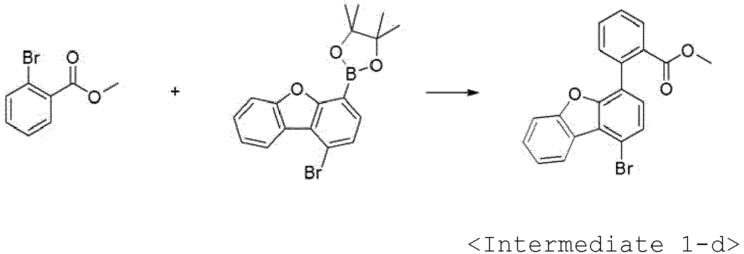

Methyl 2-bromo-benzoate (15.7 g, 73 mmol), <Intermediate 1-c> (32.8 g, 88 mmol), tetrakis(triphenylphosphine)palladium (1.7 g, 0.15 mmol) and potassium carbonate (20.2 g, 146.7 mmol) were added to a round-bottom flask, and 125 mL of toluene, 125 mL of tetrahydrofuran, and 50 mL of water were further added thereto. The temperature of the reactor was raised to 80°C and stirring was performed for 10 hours. When the reaction was completed, the temperature of the reactor was lowered to room temperature, the reaction product was extracted with ethyl acetate, and the organic layer was separated. The organic layer was concentrated under reduced pressure and separated by column chromatography to obtain 18.6 g of <Intermediate 1-d>. (yield 67%)

### Synthesis Example 1-(5): Synthesis of Intermediate 1-e

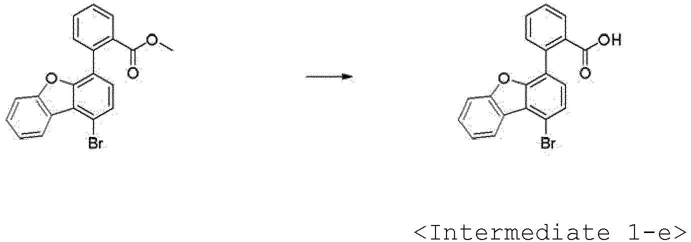

<Intermediate 1-d> (17.1 g, 45 mmol), sodium hydroxide (2.14 g, 54 mmol), and 170 mL of ethanol were added to a round-bottom flask, followed by stirring under reflux for 48 hours. Completion of the reaction was identified by thin layer chromatography and then the reaction product was cooled to room temperature. The cooled solution was acidified by dropwise addition of 2-normal hydrochloric acid thereto and the resulting solid was stirred for 30 minutes and then filtered. The resulting product was recrystallized with dichloromethane and n-hexane to obtain 14.2 g of <Intermediate 1-e>. (yield 86%)

### Synthesis Example 1-(6): Synthesis of Intermediate 1-f

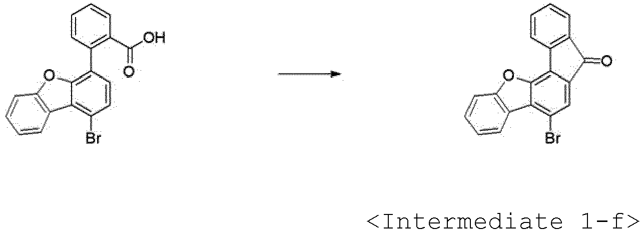

<Intermediate 1-e> (14.3 g, 39 mmol) and 145 mL of methanesulfonic acid were added to a round-bottom flask, the temperature was raised to 80°C, and the mixture was stirred for 3 hours. Completion of the reaction was identified by thin film chromatography and then the reaction product was cooled to room temperature. The reaction solution was slowly added dropwise to 150 mL of ice water, followed by stirring for 30 minutes. The resulting solid was filtered and washed with water and methanol to obtain 12.0 g of <Intermediate 1-f>. (yield 88%)

### Synthesis Example 1-(7): Synthesis of Intermediate 1-g

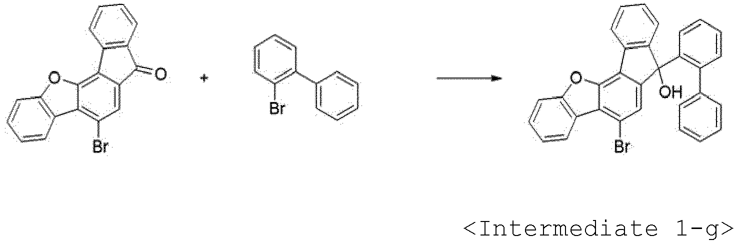

2-bromobiphenyl (8.4 g, 0.036 mol) and 110 mL of tetrahydrofuran were added to a round-bottom flask, and cooled to -78°C in a nitrogen atmosphere. Normal butyllithium (19.3 mL, 0.031 mol) was added dropwise to the cooled reaction solution at the same temperature. <Intermediate 1-f> (9.1 g, 0.026 mol) was added portionwise to the reaction solution, followed by stirring at room temperature. When the color of the reaction solution changed, completion of the reaction was identified by TLC. The reaction was terminated by addition of 50 mL of H₂O and the reaction product was extracted with ethyl acetate and water. The organic layer was separated, concentrated under reduced pressure and recrystallized with acetonitrile to obtain 10.2 g of <Intermediate 1-g>. (yield 78%)

### Synthesis Example 1-(8): Synthesis of Intermediate 1-h

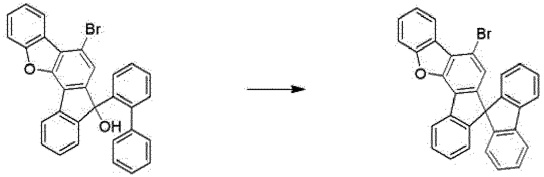

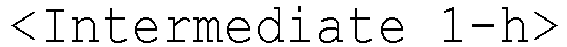

<Intermediate 1-g> (10.6 g, 0.021 mol), 120 mL of acetic acid, and 2 mL of sulfuric acid were added to a round-bottom flask, followed by stirring under reflux for 5 hours. When a solid was formed, completion of the reaction was identified by thin film chromatography and the reaction product was then cooled to room temperature. The resulting solid was filtered, washed with H₂O and methanol, dissolved in monochlorobenzene, filtered through silica gel, concentrated and cooled to room temperature to obtain 8.6 g of <Intermediate 1-h>. (yield 84%)

### Synthesis Example 1-(9): Synthesis of Intermediate 1-i

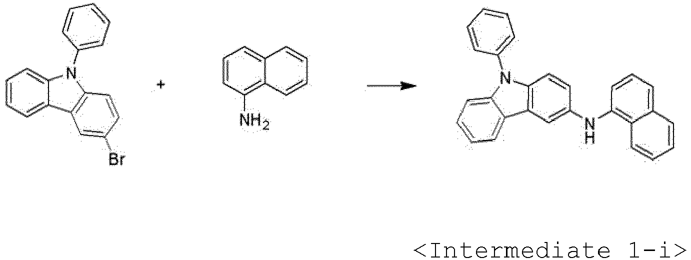

3-bromo-9-phenyl-9H-carbazole (11.3 g, 0.035 mol), 1-naphthylamine (5.6 g, 0.039 mol), tris(dibenzylideneacetone)dipalladium (0) (0.65 g, 0.0007 mol), sodium tert-butoxide (6.79 g, 0.0706 mol), 2,2'-bis(diphenylphosphino)-1,1'-binaphthalene (0.44 g, 0.0007 mol) and 100 mL of toluene were added to a round-bottom flask, followed by stirring under reflux for 3 hours. After completion of the reaction, the mixture was cooled to room temperature and extracted with ethyl acetate and water. The organic layer was separated, dehydrated with magnesium sulfate, and then concentrated under reduced pressure. The result was separated by column chromatography to obtain 11.3 g of <Intermediate 1-i>. (yield 84%)

### Synthesis Example 1-(10): Synthesis of Formula 5

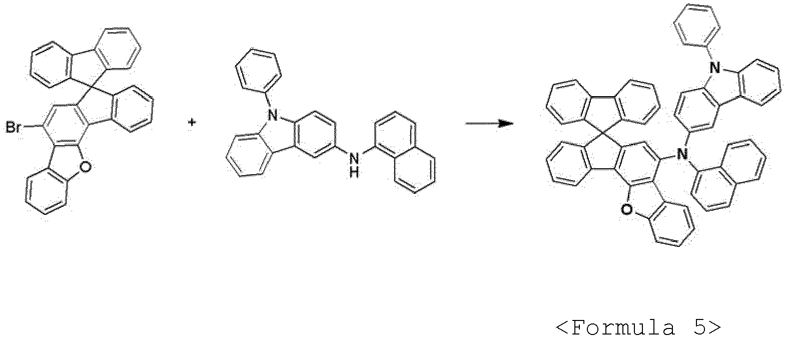

<Intermediate 1-h> (4.4 g, 0.009 mol), <Intermediate 1-i> (5.0 g, 0.013 mol), palladium (II) acetate (0.08 g, 0.4 mmol), sodium tert-butoxide (3.4 g, 0.035 mol), tri-tert-butylphosphine (0.07 g, 0.4 mmol), and toluene (60 mL) were added to a round-bottom flask, followed by stirring under reflux for 2 hours. After completion of the reaction, the reaction product was cooled to room temperature. The reaction solution was extracted with dichloromethane and water. The organic layer was separated, dehydrated with magnesium sulfate, and then concentrated under reduced pressure. The product was separated, purified by column chromatography and recrystallized with dichloromethane and acetone to obtain 3.3 g of <Formula 5> (yield 46%).

MS (MALDI-TOF): m/z 788.28[M⁺]

### Synthesis Example 2: Synthesis of Formula 8

### Synthesis Example 2-(1): Synthesis of Formula 8

<Formula 8> (yield 44%) was synthesized in the same manner as in Synthesis Example 1-(9) and Synthesis Example 1-(10) except that 2-bromo-9-phenyl-9H-carbazole was used instead of 3-bromo-9-phenyl-9H-carbazole used in Synthesis Example 1-(9), and 2-amino-9,9-dimethylfluorene was used instead of 1-naphthylamine.

MS (MALDI-TOF) : m/z 854.33[M⁺]

### Synthesis Example 3: Synthesis of Formula 47

### Synthesis Example 3-(1): Synthesis of Intermediate 3-a

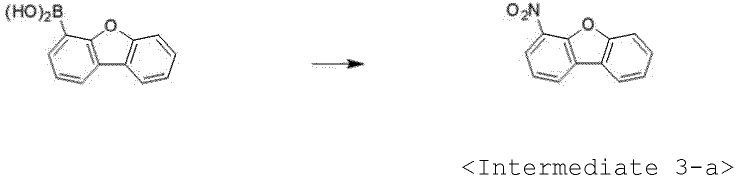

4-dibenzofuranboronic acid (85.0 g, 401 mmol), bismuth (III) nitrate pentahydrate (99.2 g, 200 mmol), and 400 mL of toluene were added to a round-bottom flask, followed by stirring at 70°C in a nitrogen atmosphere for 3 hours. After completion of the reaction, the reaction product was cooled to room temperature and the resulting solid was filtered. The filtrate was washed with toluene to obtain 61.5 g of <Intermediate 3-a>. (yield 72%)

### Synthesis Example 3-(2): Synthesis of Intermediate 3-b

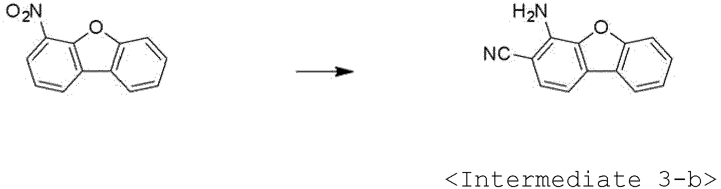

Ethylcyanoacetate (202.9 g, 1.794 mol) and 500 ml of dimethylformamide were added to a round-bottom flask. Potassium hydroxide (67.10 g, 1.196 mol) and potassium cyanide (38.95 g, 0.598 mol) were further added thereto, and 200 mL of dimethylformamide was further added thereto, followed by stirring at room temperature. <Intermediate 3-a> (127. g, 0.737 mol) was added portionwise to the reaction solution, followed by stirring at 50°C for 72 hours. After completion of the reaction, 200 mL of a sodium hydroxide aqueous solution (25%) was added to the reaction product, followed by stirring under reflux for 3 hours. The reaction product was cooled to room temperature and extracted with ethyl acetate and water. The organic layer was separated, concentrated under reduced pressure, and then purified by column chromatography to obtain 20.0 g of <Intermediate 3-b>. (yield 16%)

### Synthesis Example 3-(3): Synthesis of Intermediate 3-c

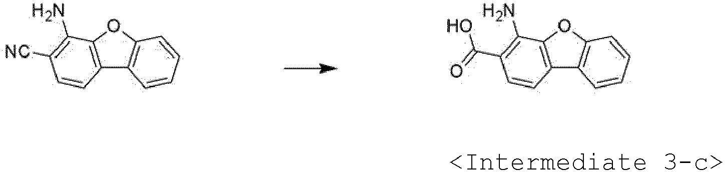

<Intermediate 3-b> (20.0 g, 96 mmol), 600 mL of ethanol, and 170 mL of an aqueous potassium hydroxide solution (142.26 g, 2.53 mol) were added to a round-bottom flask, followed by stirring under reflux for 12 hours. When the reaction was completed, the reaction product was cooled to room temperature. 400 mL of 6N hydrochloric acid was added to the reaction solution to acidify the reaction product, and the resulting solid was stirred for 20 minutes and then filtered. The solid was washed with ethanol to obtain 17.0 g of <Intermediate 3-c>. (yield 88%)

### Synthesis Example 3-(4): Synthesis of Intermediate 3-d

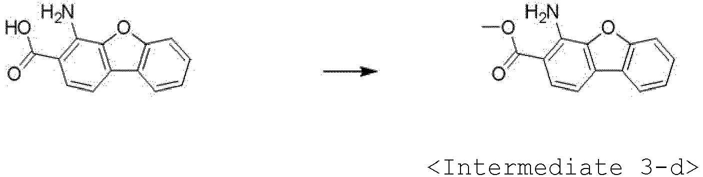

<Intermediate 3-c> (17.0 g, 75 mmol) and 15 mL of sulfuric acid were added to a round-bottom flask, followed by stirring under reflux for 72 hours. After completion of the reaction, the mixture was cooled to room temperature and extracted with ethyl acetate and water. The organic layer was separated and washed with an aqueous sodium hydrogen carbonate solution. An excess of methanol was added to the organic layer during concentration under reduced pressure and the resulting solid was filtered to obtain 14.0 g of <Intermediate 3-d>. (yield 78%)

### Synthesis Example 3-(5): Synthesis of Intermediate 3-e

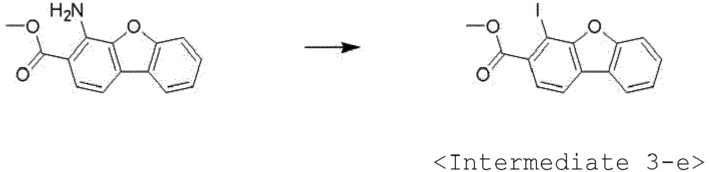

<Intermediate 3-d> (12 g, 50 mmol), 15 mL of hydrochloric acid, and 75 mL of water were added to a round-bottom flask, cooled to 0°C and stirred for 1 hour. 38 mL (5.6 g, 81 mmol) of an aqueous sodium nitrite solution was added dropwise at the same temperature to the reaction solution, followed by stirring for 1 hour. 38 mL of an aqueous potassium iodide solution (22.4 g, 135 mmol) was added dropwise while the temperature of the reaction solution was kept within 5°C or less. The resulting product was stirred at room temperature for 5 hours. After completion of the reaction, the mixture was washed with an aqueous sodium thiosulfate solution and extracted with ethyl acetate and water. The organic layer was separated, concentrated under reduced pressure, and then separated by column chromatography to obtain 11 g of <Intermediate 3-e>. (yield 91%)

### Synthesis Example 3-(6): Synthesis of Intermediate 3-f

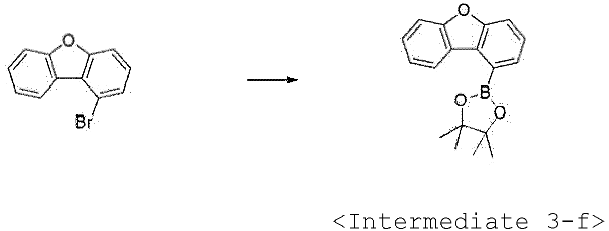

1-bromodibenzofuran (20.0 g, 81 mmol) , bis(pinacolato)diboron (26.7 g, 105 mmol), [1,1'-bis(diphenylphosphino)ferrocene]dichloro palladium (1.3 g, 0.002 mol), potassium acetate (19.9 g, 202 mmol), and 200 mL of 1,4-dioxane were added to a round-bottom flask, followed by stirring under reflux for 10 hours. The reaction product was concentrated under reduced pressure and then was separated by column chromatography. The result was recrystallized with dichloromethane and heptane to obtain 17.0 g of <Intermediate 3-f>. (yield 70%)

### Synthesis Example 3-(7): Synthesis of Intermediate 3-g

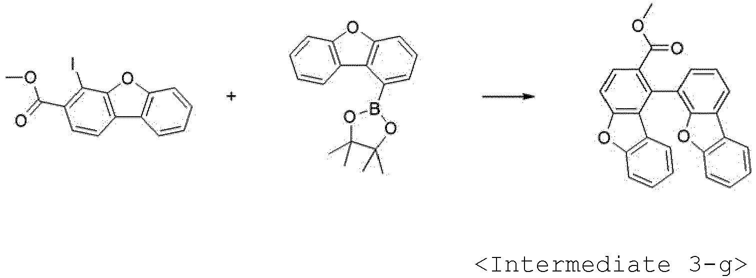

<Intermediate 3-g> (yield 75%) was obtained in the same synthesis manner as in Synthesis Example 1-(4), except that <Intermediate 3-e> was used instead of methyl 2-bromo-benzoate and <Intermediate 3-f> was used instead of <Intermediate 1-c>.

### Synthesis Example 3-(8): Synthesis of Intermediate 3-h

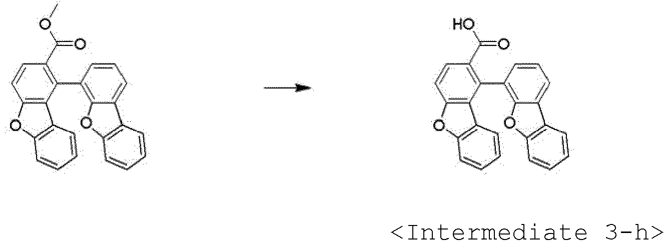

<Intermediate 3-h> (yield 77%) was obtained in the same synthesis manner as in Synthesis Example 1-(5), except that <Intermediate 3-g> was used instead of <Intermediate 1-d>.

### Synthesis Example 3-(9): Synthesis of Intermediate 3-i

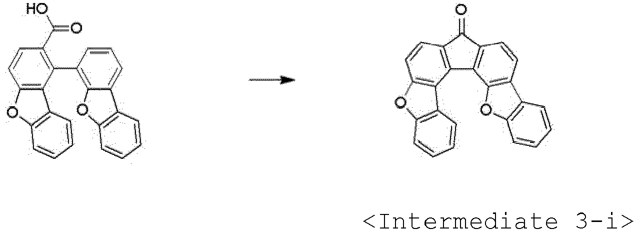

<Intermediate 3-i> (yield 94%) was obtained in the same synthesis manner as in Synthesis Example 1-(6), except that <Intermediate 3-h> was used instead of <Intermediate 1-e>.

### Synthesis Example 3-(10): Synthesis of Intermediate 3-j

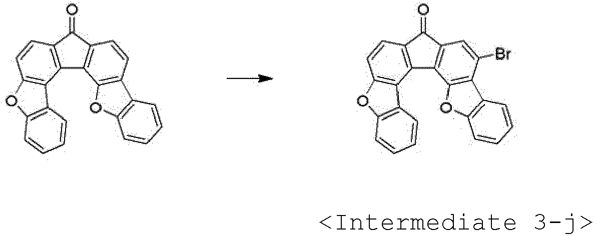

<Intermediate 3-i> (44 g, 122 mmol>, 600 mL of dichloromethane was added to a round-bottom flask, followed by stirring at room temperature. A dilution of bromine (13.7 mL, 85 mmol) in 50 mL of dichloromethane was added dropwise, followed by stirring for about 3 hours. The reaction product was recrystallized with methanol to obtain 40.7 g of <Intermediate 3-j>. (yield 76%)

### Synthesis Example 3-(11): Synthesis of Intermediate 3-k

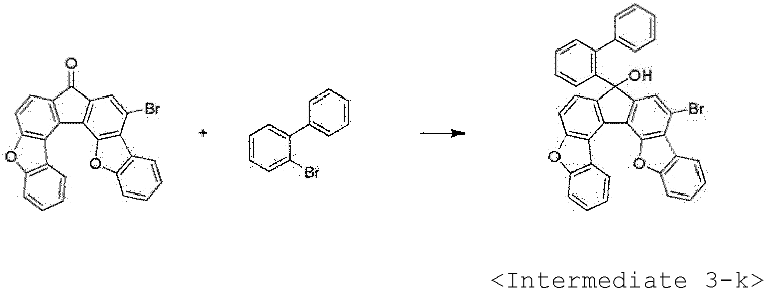

<Intermediate 3-k> (yield 74%) was obtained in the same synthesis manner as in Synthesis Example 1-(7), except that <Intermediate 3-j> was used instead of <Intermediate 1-f>.

### Synthesis Example 3-(12): Synthesis of Intermediate 3-l

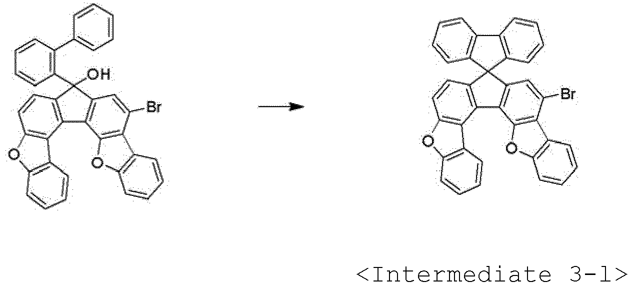

<Intermediate 3-l> (yield 86%) was obtained in the same synthesis manner as in Synthesis Example 1-(8), except that <Intermediate 3-k> was used instead of <Intermediate 1-g>.

### Synthesis Example 3-(13): Synthesis of Formula 47

<Formula 47> (yield 45%) was obtained in the same synthesis manner as in Synthesis Example 1-(9) and Synthesis Example 1-(10) except that 4-tert-butylaniline was used instead of 1-naphthylamine in Synthesis Example 1-(9) and <Intermediate 3-l> was used instead of <Intermediate 1-h> in Synthesis Example 1-(10).

MS (MALDI-TOF) : m/z 884.34[M⁺]

### Synthesis Example 4: Synthesis of Formula 54

### Synthesis Example 4-(1): Synthesis of Intermediate 4-a

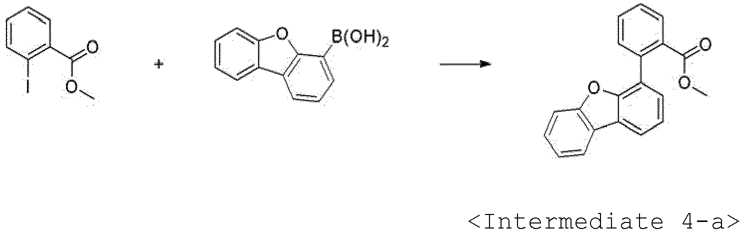

Methyl 2-iodobenzoate (19.1 g, 73 mmol), 4-dibenzofuran boronic acid (18.7 g, 88 mmol), tetrakis (triphenylphosphine)palladium (1.7 g, 0.15 mmol), and potassium carbonate (20.2 g, 146.7 mmol) were added to a round-bottom flask, and 125 mL of toluene, 125 mL of tetrahydrofuran, and 50 mL of water were further added thereto. The temperature of the reactor was raised to 80°C, followed by stirring for 10 hours. When the reaction was completed, the temperature of the reactor was lowered to room temperature, the reaction product was extracted with ethyl acetate, and the organic layer was separated. The organic layer was concentrated under reduced pressure and separated by column chromatography to obtain 9.5 g of <Intermediate 4-a>. (yield 43%)

### Synthesis Example 4-(2): Synthesis of Intermediate 4-b

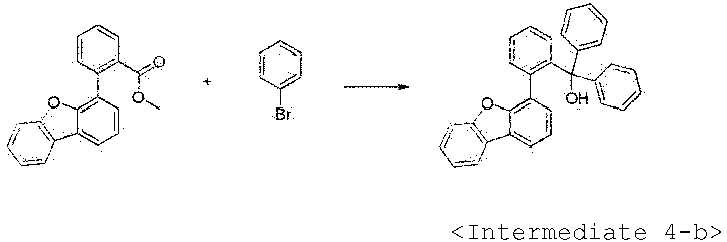

Bromobenzene (13.2 g, 83.97 mmol) and 250 mL of tetrahydrofuran were added to a round-bottom flask, followed by stirring in a nitrogen atmosphere at low temperature. About 58 mL of n-butyllithium was slowly added dropwise at -78°C for 2 hours and then <Intermediate 4-a> (9.4 g, 31.1 mmol) was added thereto. After completion of the reaction, 100 mL of water was added, followed by stirring for 30 minutes and extraction to obtain 3.2 g of <Intermediate 4-b>. (yield 24%)

### Synthesis Example 4-(3): Synthesis of Intermediate 4-c

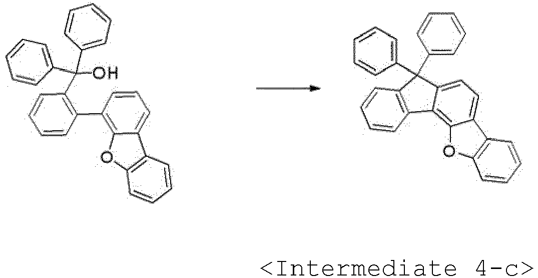

<Intermediate 4-b> (55.0 g, 129 mmol), 500 mL of acetic acid, and 10 mL of sulfuric acid were added to a round-bottom flask, followed by stirring under reflux for 5 hours. After completion of the reaction, the reaction product was cooled to room temperature and the resulting solid was filtered. The result was washed with methanol to obtain 50 g of <Intermediate 4-c>. (yield 95%)

### Synthesis Example 4-(4): Synthesis of Intermediate 4-d

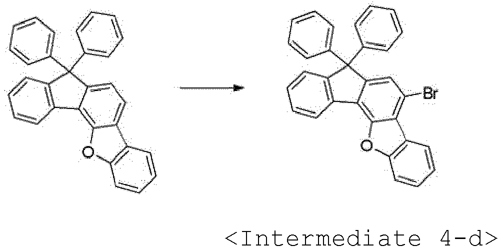

<Intermediate 4-c> (50 g, 122 mmol>, 600 mL of dichloromethane was added to a round-bottom flask, followed by stirring at room temperature. A dilution of bromine (13.7 mL, 85 mmol) in 50 mL of dichloromethane was added dropwise, followed by stirring for about 3 hours. The reaction product was recrystallized with methanol to obtain 45 g of <Intermediate 4-d>. (yield 76%)

### Synthesis Example 4-(5): Synthesis of Formula 54

<Formula 54> (yield 44%) was obtained in the same synthesis manner as in Synthesis Example 1-(9) and Synthesis Example 1-(10) except that aniline-2,3,4,5,6-d5 was used instead of 1-naphthylamine in Synthesis Example 1-(9) and <Intermediate 4-d> was used instead of <Intermediate 1-h> in Synthesis Example 1-(10).

MS (MALDI-TOF) : m/z 745.31[M⁺]

### Synthesis Example 5: Synthesis of Formula 52

### Synthesis Example 5-(1): Synthesis of Formula 52

<Formula 52> (yield 45%) was obtained in the same synthesis manner as in Synthesis Example 1-(9) and Synthesis Example 1-(10) except that 3-aminodibenzofuran was used instead of 1-naphthylamine in Synthesis Example 1-(9) and <Intermediate 4-d> was used instead of <Intermediate 1-h> in Synthesis Example 1-(10).

MS (MALDI-TOF) : m/z 830.29[M⁺]

### Synthesis Example 6: Synthesis of Formula 41

### Synthesis Example 6-(1): Synthesis of Intermediate 6-a

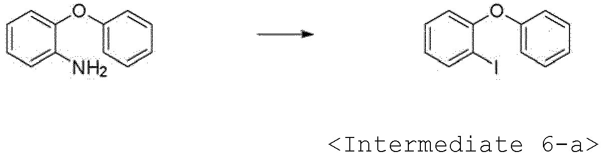

2-phenoxyaniline (25.0 g, 0.135 mol), 30 mL of hydrochloric acid, and 150 mL of water were added to a round-bottom flask, cooled to 0°C and stirred for 1 hour. 75 mL (11.2 g, 0.162 mmol) of an aqueous sodium nitrite solution was added dropwise at the same temperature to the reaction solution, followed by stirring for 1 hour. 75 mL of an aqueous potassium iodide solution (44.8 g, 0.270 mol) was added dropwise while the temperature of the reaction solution was kept within 5°C or less. The resulting product was stirred at room temperature for 5 hours. After completion of the reaction, the mixture was washed with an aqueous sodium thiosulfate solution and extracted with ethyl acetate and water. The organic layer was separated, concentrated under reduced pressure, and then separated by column chromatography to obtain 22.6 g of <Intermediate 6-a>. (yield 56%)

### Synthesis Example 6-(2): Synthesis of Intermediate 6-b

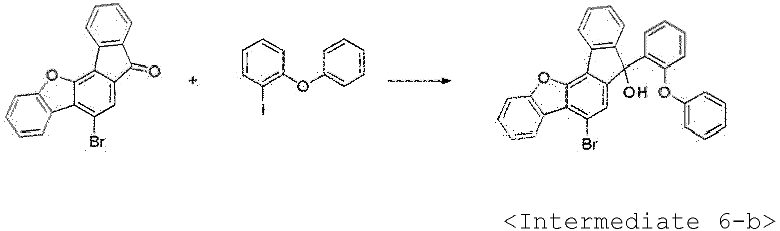

<Intermediate 6-b> (yield 70%) was obtained in the same synthesis manner as in in Synthesis Example 1-(7), except that <Intermediate 6-a> was used instead of 2-bromobiphenyl used in Synthesis Example 1-(7).

### Synthesis Example 6-(3): Synthesis of Intermediate 6-c

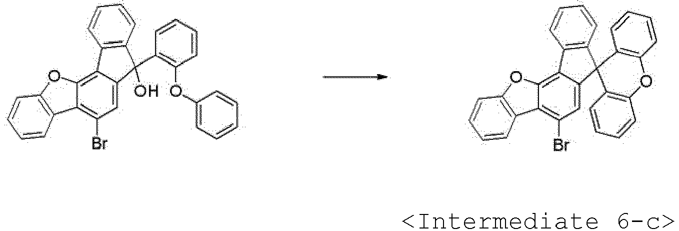

<Intermediate 6-c> (yield 75%) was obtained in the same synthesis manner as in Synthesis Example 1-(8), except that <Intermediate 6-b> was used instead of <Intermediate 1-g> used in Synthesis Example 1-(8).

### Synthesis Example 6-(4): Synthesis of Formula 41

<Formula 41> (yield 44%) was obtained in the same synthesis manner as in Synthesis Example 1-(9) and Synthesis Example 1-(10), except that 2-bromo-9-phenyl-9H-carbazole was used instead of 3-bromo-9-phenyl-9H-carbazole used in Synthesis Example 1-(9), 4-tert-butylaniline was used instead of 1-naphthylamine and <Intermediate 6-c> was used instead of <Intermediate 1-h> used in Synthesis Example 1-(10).

MS (MALDI-TOF) : m/z 810.32 [M⁺]

### Synthesis Example 7: Synthesis of Formula 88

### Synthesis Example 7-(1): Synthesis of Intermediate 7-a

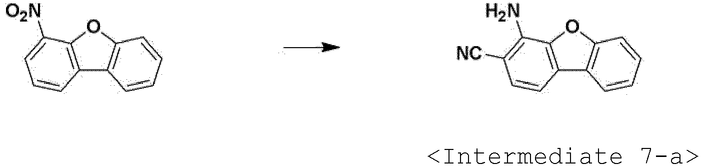

Ethyl cyanoacetate (202.9 g, 1.794 mol) and 500 ml of dimethylformamide were added to a round-bottom flask. Potassium hydroxide (67.1 g, 1.196 mol) and potassium cyanide (38.95 g, 0.598 mol) were further added thereto, and 200 mL of dimethylformamide was further added thereto, followed by stirring at room temperature. 4-nitrobenzofuran (127.5 g, 0.737 mol) was added portionwise to the reaction solution, followed by stirring at 50°C for 72 hours. After completion of the reaction, 200 mL of an aqueous sodium hydroxide solution (25%) was added to the reaction product, followed by stirring under reflux for 3 hours. The reaction product was cooled to room temperature and extracted with ethyl acetate and water. The organic layer was separated, concentrated under reduced pressure, and then purified by column chromatography to obtain 20.0 g of <Intermediate 7-a>. (yield 17%)

### Synthesis Example 7-(2): Synthesis of Intermediate 7-b

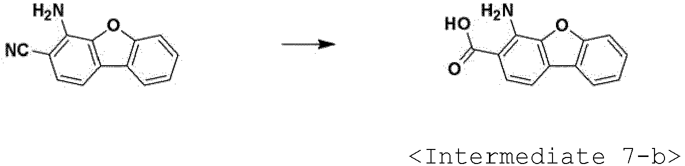

<Intermediate 7-a> (20.0 g, 96 mmol), 600 mL of ethanol, and 170 mL (142.26 g, 2.53 mol) of an aqueous potassium hydroxide solution were added to a round-bottom flask, followed by stirring under reflux for 12 hours. When the reaction was completed, the reaction product was cooled to room temperature. 400 mL of 6N hydrochloric acid was added to the reaction solution to acidify the reaction product, and the resulting solid was stirred for 20 minutes and then filtered. The solid was washed with ethanol to obtain 17.0 g of <Intermediate 7-b>. (yield 88%)

### Synthesis Example 7-(3): Synthesis of Intermediate 7-c

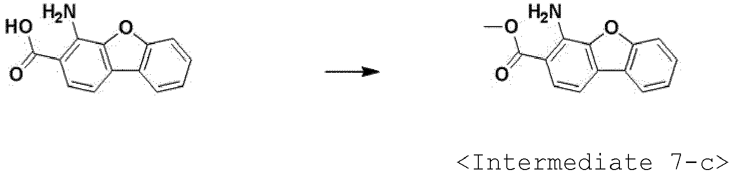

<Intermediate 7-b> (17.0 g, 75 mmol) and 15 mL of sulfuric acid were added to a round-bottom flask, followed by stirring under reflux for 72 hours. After completion of the reaction, the mixture was cooled to room temperature and extracted with ethyl acetate and water. The organic layer was separated and washed with an aqueous sodium hydrogen carbonate solution. An excess of methanol was added to the organic layer during concentration under reduced pressure and the resulting solid was filtered to obtain 14.0 g of <Intermediate 7-c>. (yield 78%)

### Synthesis Example 7-(4): Synthesis of Intermediate 7-d

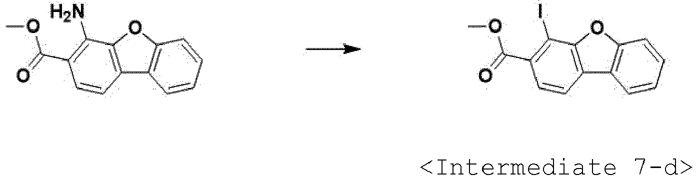

<Intermediate 7-c> (14.0 g, 0.058 mmol), 20 mL of hydrochloric acid, and 100 mL of water were added to a round-bottom flask, cooled to 0°C and stirred for 1 hour. 50 mL (7.4 g, 0.116 mol) of an aqueous sodium nitrite solution was added dropwise at the same temperature to the reaction solution, followed by stirring for 1 hour. 100 mL of an aqueous potassium iodide solution (30.0 g, 0.180 mol) was added dropwise while the temperature of the reaction solution was kept within 5°C or less. The resulting product was stirred at room temperature for 5 hours. After completion of the reaction, the mixture was washed with an aqueous sodium thiosulfate solution and extracted with ethyl acetate and water. The organic layer was separated, concentrated under reduced pressure, and then separated by column chromatography to obtain 9.1 g of <Intermediate 7-d>. (yield 48%)

### Synthesis Example 7-(5): Synthesis of Intermediate 7-e

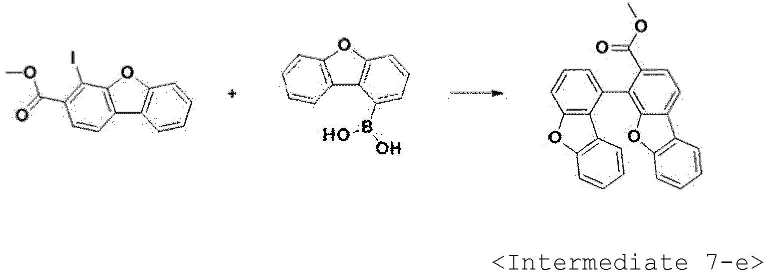

<Intermediate 7-d> (9.3 g, 25 mmol), 1-dibenzofuran boronic acid (8.3 g, 28 mmol), tetrakis(triphenylphosphine)palladium (0.6 g, 0.05 mmol) and potassium carbonate (6.7 g, 50 mmol) were added to a round-bottom flask, and 50 mL of toluene, 50 mL of tetrahydrofuran, and 20 mL of water were further added thereto. The temperature of the reactor was raised to 80°C and stirring was performed for 10 hours. When the reaction was completed, the temperature of the reactor was lowered to room temperature, the reaction product was extracted with ethyl acetate, and the organic layer was separated. The organic layer was concentrated under reduced pressure and separated by column chromatography to obtain 5.3 g of <Intermediate 7-e>. (yield 52%)

### Synthesis Example 7-(6): Synthesis of Intermediate 7-f

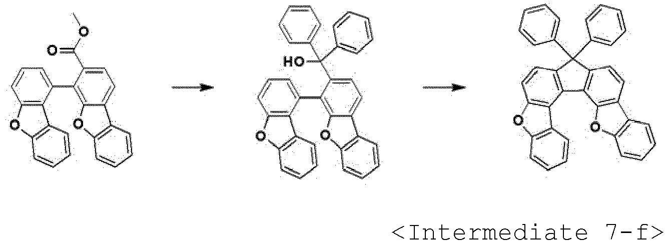

Bromobenzene (25.5 g, 0.163 mol) and 170 mL of tetrahydrofuran were added to a round-bottom flask and the resulting product was cooled to -78°C under a nitrogen atmosphere. Butyllithium (1.6 M) (95.6 mL, 0.153 mol) was slowly added dropwise to the cooled reaction solution. The resulting product was stirred at the same temperature for 1 hour and <Intermediate 7-e> (20.0 g, 0.051 mol) was added thereto, followed by stirring at room temperature for 3 hours. After completion of the reaction, 50 mL of water was added, followed by stirring for 30 minutes. The result was extracted with ethyl acetate and water and the organic layer was separated and concentrated under reduced pressure. 200 mL of acetic acid and 1 mL of hydrochloric acid were added to the concentrate, followed by stirring at an elevated temperature of 80°C. After completion of the reaction, the mixture was filtered at room temperature and washed with methanol to obtain 20.0 g of <Intermediate 7-f>. (yield 78%)

### Synthesis Example 7-(7): Synthesis of Intermediate 7-g

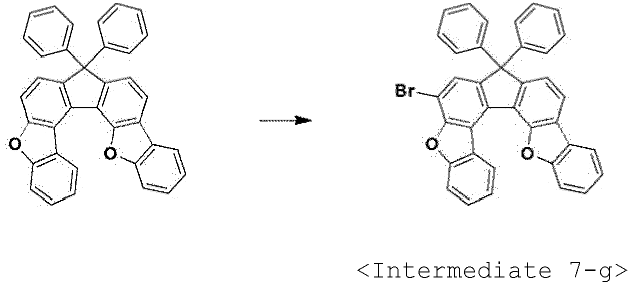

<Intermediate 7-g> (yield 55%) was obtained in the same synthesis manner as in in Synthesis Example 3-(10), except that <Intermediate 7-f> was used instead of <Intermediate 3-i> used in Synthesis Example 3-(10).

### Synthesis Example 7-(8): Synthesis of Formula 88

<Formula 88> (yield 46%) was obtained in the same synthesis manner as in Synthesis Example 1-(9) and Synthesis Example 1-(10), except that 4-bromo-9-phenyl-9H-carbazole was used instead of 3-bromo-9-phenyl-9H-carbazole used in Synthesis Example 1-(9), and <Intermediate 7-g> was used instead of <Intermediate 1-h> used in Synthesis Example 1-(10).

MS (MALDI-TOF): m/z 956.34 [M⁺]

### Synthesis Example 8: Synthesis of Formula 101

### Synthesis Example 8-(1): Synthesis of Intermediate 8-a

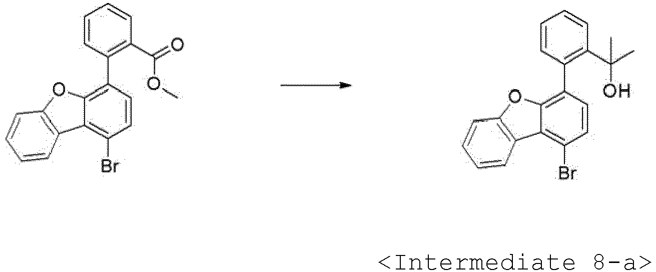

<Intermediate 1-d> (30.5 g, 80 mmol) was added to a round-bottom flask containing 250 mL of tetrahydrofuran, and then the temperature was lowered to -78°C under nitrogen. 30 minutes later, 1.0 M methyl magnesium bromide (210 mL, 240 mmol) was slowly added dropwise. 1 hour later, 1.0 M methyl magnesium bromide (210 mL, 240 mmol) was slowly added dropwise and then warmed to room temperature. The reaction product was stirred at room temperature for about 2 hours, and an aqueous ammonium chloride solution was added dropwise thereto. The result was extracted, distilled under reduced pressure, and recrystallized with hexane to obtain 24.4 g of <Intermediate 8-a> (yield 80%).

### Synthesis Example 8-(2): Synthesis of Intermediate 8-b

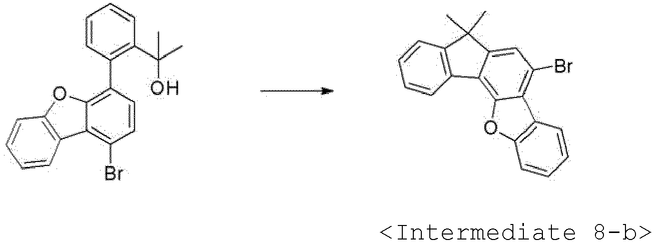

<Intermediate 8-a> (25.2 g, 66 mmol) was added to a round-bottom flask containing 300 mL of acetic acid, followed by stirring at -0°C for 10 minutes. 350 mL of phosphoric acid was added thereto, followed by stirring at room temperature for about 1 hour. The product was neutralized with an aqueous sodium hydroxide solution, extracted, and then concentrated under reduced pressure. The result was separated by column chromatography to obtain 17.5 g of <Intermediate 8-b>. (yield 73%)

### Synthesis Example 8-(3): Synthesis of Formula 101

<Formula 101> (yield 45%) was obtained in the same synthesis manner as in Synthesis Example 1-(9) and Synthesis Example 1-(10), except that 3-bromo-9-(1-naphtyl)-9H-carbazole was used instead of 3-bromo-9-phenyl-9H-carbazole used in Synthesis Example 1-(9), 2-naphthylamine was used instead of 1-naphthylamine, and <Intermediate 8-b> was used instead of <Intermediate 1-h> used in Synthesis Example 1-(10).

MS (MALDI-TOF): m/z 716.28 [M⁺]

### Synthesis Example 9: Synthesis of Formula 102

### Synthesis Example 9-(1): Synthesis of Formula 102

<Formula 102> (yield 48%) was obtained in the same synthesis manner as in Synthesis Example 1-(9) and Synthesis Example 1-(10), except that aniline was used instead of 1-naphthylamine used in Synthesis Example 1-(9), and <Intermediate 8-b> was used instead of <Intermediate 1-h> used in Synthesis Example 1-(10).

MS (MALDI-TOF): m/z 616.25 [M⁺]

### Synthesis Example 10: Synthesis of Formula 103

### Synthesis Example 10- (1): Synthesis of Formula 103

<Formula 103> (yield 43%) was obtained in the same synthesis manner as in Synthesis Example 1-(9) and Synthesis Example 1-(10), except that 2-bromo-9-phenyl-9H-carbazole was used instead of 3-bromo-9-phenyl-9H-carbazole used in Synthesis Example 1-(9), 4-(1-naphthyl)aniline was used instead of 1-naphthylamine, and <Intermediate 8-b> was used instead of <Intermediate 1-h> used in Synthesis Example 1-(10).

MS (MALDI-TOF) : m/z 742.30 [M⁺]

### Synthesis Example 11: Synthesis of Formula 104

### Synthesis Example 11- (1): Synthesis of Formula 104

<Formula 104> (yield 44%) was obtained in the same synthesis manner as in in Synthesis Example 1- (9) and Synthesis Example 1-(10), except that 2-bromo-9-phenyl-9H-carbazole was used instead of 3-bromo-9-phenyl-9H-carbazole used in Synthesis Example 1-(9), 2-amino-9,9-dimethylfluorene was used instead of 1-naphthylamine, and <Intermediate 8-b> was used instead of <Intermediate 1-h> used in Synthesis Example 1-(10).

MS (MALDI-TOF): m/z 732.31 [M⁺]

### Synthesis Example 12: Synthesis of Formula 91

### Synthesis Example 12-(1): Synthesis of Intermediate 12-a

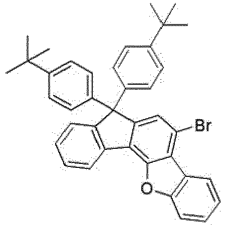

### <Intermediate 12-a>

<Intermediate 12-a> (yield 70%) was obtained in the same synthesis manner as in Synthesis Example 4-(2) to Synthesis Example 4-(4), except that 1-bromo-4-tert-butylbenzene was used instead of bromobenzene used in Synthesis Example 4-(2).

### Synthesis Example 12-(2): Synthesis of Formula 91

<Formula 91> (yield 45%) was obtained in the same synthesis manner as in Synthesis Example 1-(9) and Synthesis Example 1-(10), except that 3-aminodibenzofuran was used instead of 1-naphthylamine and <Intermediate 12-a> was used instead of <Intermediate 1-h> used in Synthesis Example 1-(10).

MS (MALDI-TOF): m/z 942.42 [M⁺]

### Examples 1 to 12: Fabrication of organic light-emitting devices

ITO glass was patterned such that a light-emitting area of the ITO glass was adjusted to 2 mm × 2 mm and was then washed. The ITO glass was mounted in a vacuum chamber, a base pressure was set to 1 × 10⁻⁷ torr, and 2-TNATA (400 Å) and the compound shown in [Table 1] (200 Å) were sequentially deposited on ITO. Then, a mixture of [BH] as a host and [BD] as a dopant (3 wt%) was deposited to a thickness of 250Å to form a light-emitting layer. Then, [Formula E-1] was deposited thereon to a thickness of 300Å to form an electron transport layer, Liq was deposited thereon to a thickness of 10Å to form an electron injection layer, and Al was deposited thereon to a thickness of 1000Å to form an anode. As a result, an organic light-emitting device was fabricated. The luminous efficacy of the organic light-emitting device was measured at 10 mA/cm².

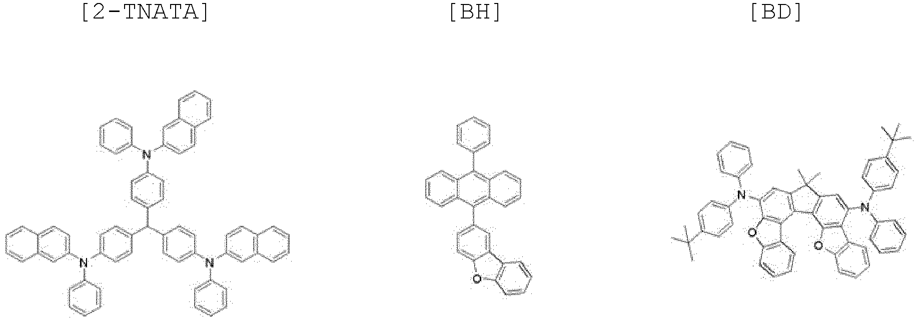

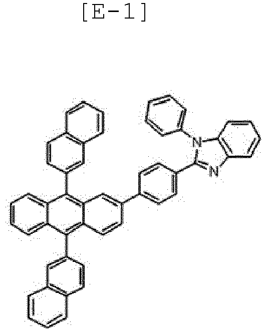

### Comparative Examples 1 to 3

Organic light-emitting devices were fabricated in the same manner as in Examples above, except that [Compound A] to [Compound C] were used instead of the compounds used in Examples 1 to 12. The luminous efficacy of the organic light-emitting device was measured at 10 mA/cm². The structures of [Compound A] to [Compound C] are as follows.

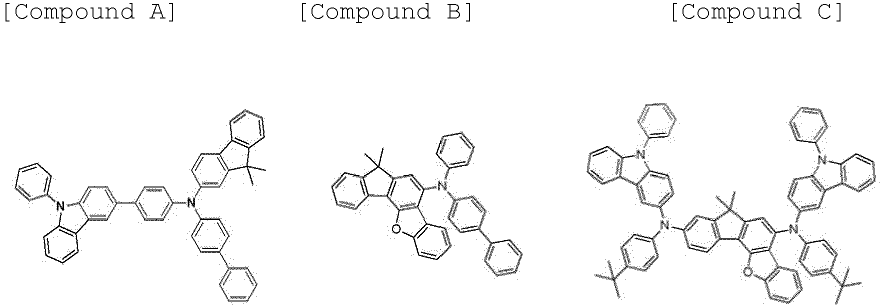

**[Table 1]**

| Item | Hole transport compound | Dopant | CIEx | CIEy | Cd/A |
|---|---|---|---|---|---|
| Example 1 | Formula 5 | BD | 0.136 | 0.112 | 10.73 |
| Example 2 | Formula 8 | BD | 0.136 | 0.112 | 10.59 |
| Example 3 | Formula 47 | BD | 0.137 | 0.113 | 10.78 |
| Example 4 | Formula 54 | BD | 0.137 | 0.112 | 11.96 |
| Example 5 | Formula 52 | BD | 0.136 | 0.112 | 11.90 |
| Example 6 | Formula 41 | BD | 0.137 | 0.111 | 10.04 |
| Example 7 | Formula 88 | BD | 0.136 | 0.112 | 10.20 |
| Example 8 | Formula 101 | BD | 0.136 | 0.112 | 11.04 |
| Example 9 | Formula 102 | BD | 0.137 | 0.113 | 11.00 |
| Example 10 | Formula 103 | BD | 0.136 | 0.113 | 10.68 |
| Example 11 | Formula 104 | BD | 0.136 | 0.112 | 10.76 |
| Example 12 | Formula 91 | BD | 0.137 | 0.112 | 10.52 |
| Comparative Example 1 | Formula A | BD | 0.136 | 0.112 | 8.01 |
| Comparative Example 2 | Formula B | BD | 0.136 | 0.112 | 9.06 |
| Comparative Example 3 | Formula C | BD | 0.136 | 0.112 | 8.64 |

As can be seen from [Table 1] above, the organic light-emitting device containing each of the compounds according to the present invention exhibits remarkably improved luminous efficacy, compared to an organic light-emitting device containing each of the compounds according to Comparative Examples 1 to 3.

### Examples 13 to 15: Fabrication of organic light-emitting devices

ITO glass was patterned such that a light-emitting area of the ITO glass was adjusted to 2 mm × 2 mm and was then washed. The ITO glass was mounted in a vacuum chamber, a base pressure was set to 1 × 10⁻⁷ torr, DNTPD (700 Å) and the compound A (250 Å) were sequentially deposited on ITO, and an amine compound according to the present invention shown in [Table 1] was deposited thereon to a thickness of 50Å to form an electron blocking layer (EBL), and a mixture of [BH] as a host and [BD] as a dopant (3 wt%) was deposited to a thickness of 250Å to form a light-emitting layer. Then, [Formula E-1] was deposited thereon to a thickness of 300Å to form an electron transport layer, Liq was deposited thereon to a thickness of 10Å to form an electron injection layer, and Al was deposited thereon to a thickness of 1,000Å to form a cathode. As a result, an organic light-emitting device was fabricated. The properties of the organic light-emitting device were measured at 10 mA/cm².

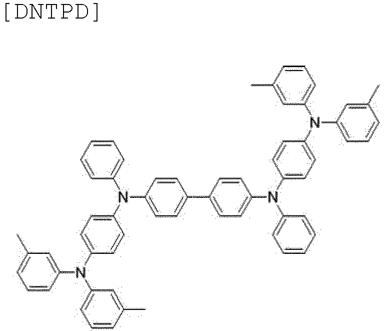

### Comparative Example 4

An organic light-emitting diode was fabricated in the same manner as in Examples, except that [EBL] was used as the electron blocking layer used in each of Examples 13 to 15, and the structure of [EBL] is as follows.

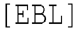

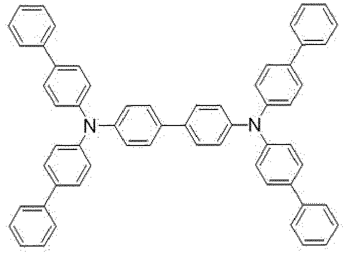

**[Table 2]**

| Item | Electron blocking layer | V | Cd/A | CIEx | CIEy |
|---|---|---|---|---|---|
| Example 13 | Formula 5 | 3.5 | 9.07 | 0.136 | 0.111 |
| Example 14 | Formula 52 | 3.55 | 9.4 | 0.135 | 0.112 |
| Example 15 | Formula 102 | 3.48 | 8.96 | 0.137 | 0.112 |
| Comparative Example 4 | EBL | 3.71 | 6.46 | 0.136 | 0.112 |

As can be seen from [Table 2] above, the organic light-emitting device using the compound according to the present invention for the electron blocking layer can be operated at a lower voltage and exhibit remarkably improved luminous efficacy, compared to an organic light-emitting device using the conventional compound according to Comparative Example 4 for the electron blocking layer.

### [Industrial Applicability]

The amine compound having a fused ring according to the present invention can be used as a material for an organic layer, particularly, as a material for a hole transport or electron blocking layer in the device to realize a highly efficient organic light-emitting device and thus is industrially applicable to flat panel displays, flexible displays, monochromatic or white flat panel lighting systems, monochromatic or white flexible lighting systems, vehicle displays, displays for virtual or augmented reality and the like.

## Claims

1. An organic light-emitting compound represented by the following [Formula A] or [Formula B]: 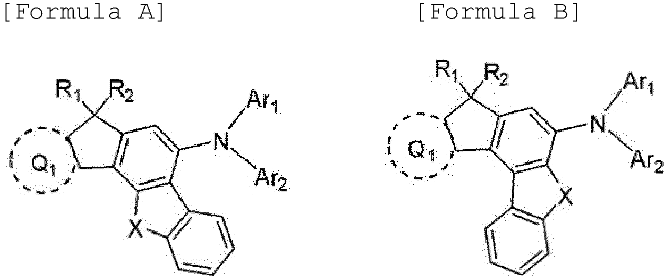
wherein Q₁ is selected from a substituted or unsubstituted C6-C30 aryl group, and a substituted or unsubstituted C2-C50 heteroaryl group;
X is an oxygen atom (O) or a sulfur atom (S);
R₁ and R₂ are identical to or different from each other, and are each independently selected from hydrogen, deuterium, a substituted or unsubstituted C1-C30 alkyl group, a substituted or unsubstituted C3-C30 cycloalkyl group, a substituted or unsubstituted C6-C50 aryl group, and a substituted or unsubstituted C2-C50 heteroaryl group, with the proviso that R₁ and R₂ are bonded to each other to form an alicyclic or aromatic monocyclic or polycyclic ring; and
Ar₁ and Ar₂ are identical to or different from each other, and are each independently a substituted or unsubstituted C6-C50 aryl group, and a substituted or unsubstituted C2-C50 heteroaryl group, with the proviso that at least one of Ar₁ and Ar₂ is represented by the following Structural Formula 1: 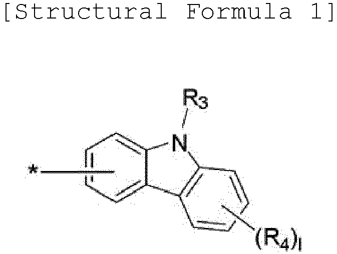
wherein R₃ is selected from hydrogen, deuterium, a substituted or unsubstituted C1-C30 alkyl group, a substituted or unsubstituted C3-C30 cycloalkyl group, a substituted or unsubstituted C6-C50 aryl group, and a substituted or unsubstituted C2-C50 heteroaryl group;
R₄ is selected from hydrogen, deuterium, a cyano group, a halogen group, a hydroxyl group, a nitro group, a C1-C24 alkyl group, a C1-C24 halogenated alkyl group, a C1-C24 cycloalkyl group, a C1-C24 alkenyl group, a C1-C24 alkynyl group, a C1-C24 heteroalkyl group, a C6-C30 aryl group, a C6-C30 arylalkyl group, a C2-C30 heteroaryl group, a C2-C30 heteroarylalkyl group, a C1-C24 alkoxy group, a C1-C24 alkylamino group, a C6-C30 arylamino group, a C2-C30 heteroarylamino group, a C1-C24 alkylsilyl group, a C6-C30 arylsilyl group, and a C6-C30 aryloxy group;
1 is an integer from 0 to 4, provided that when 1 is 2 or more, R₄' s are identical to or different from each other; and
"-*" means a site bonding to a nitrogen atom at the positions Ar₁ and Ar₂ in [Formula A] or [Formula B].

2. The organic light-emitting compound according to claim 1, wherein the "substituted" in the term "substituted or unsubstituted" indicates substitution of each of Q₁, R₁ to R₃ and Ar₁ to Ar₂ with one or more substituents selected from deuterium, a cyano group, a halogen group, a hydroxyl group, a nitro group, an alkyl group, a halogenated alkyl group, a cycloalkyl group, an alkenyl group, an alkynyl group, a heteroalkyl group, an aryl group, an arylalkyl group, an alkylaryl group, a heteroaryl group, a heteroarylalkyl group, an alkoxy group, an amine group, a silyl group, an aryloxy group and a mixed aliphatic-aromatic ring group, or substitution with a substituent including two or more of the substituents linked to each other.

3. The organic light-emitting compound according to claim 1, wherein [Formula A] or [Formula B] is selected from compounds represented by the following [Compound 1] to [Compound 150]:

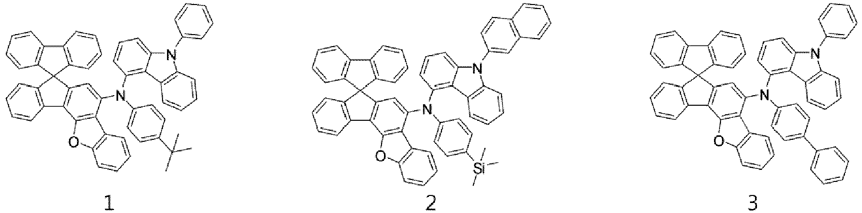

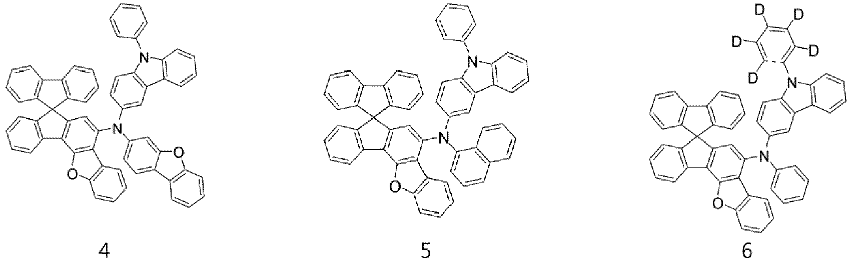

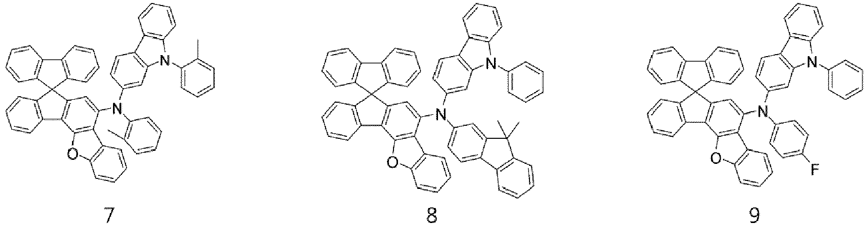

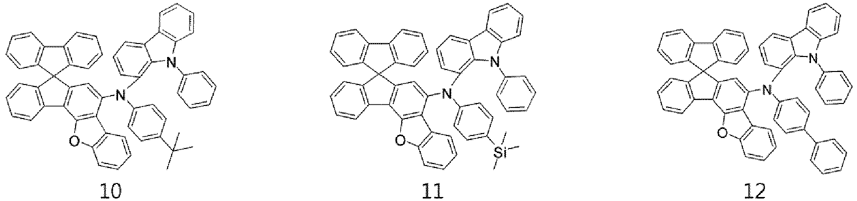

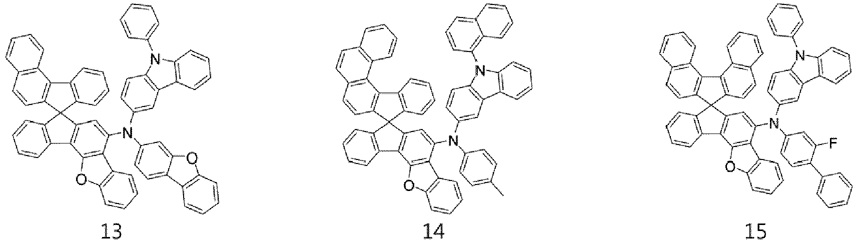

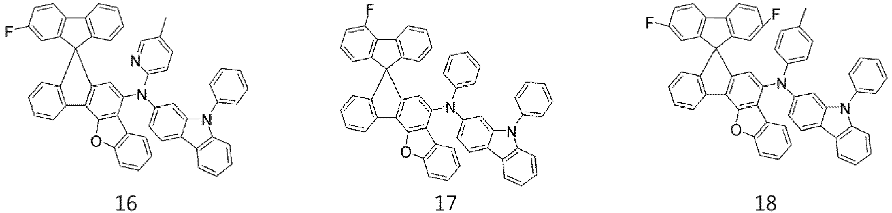

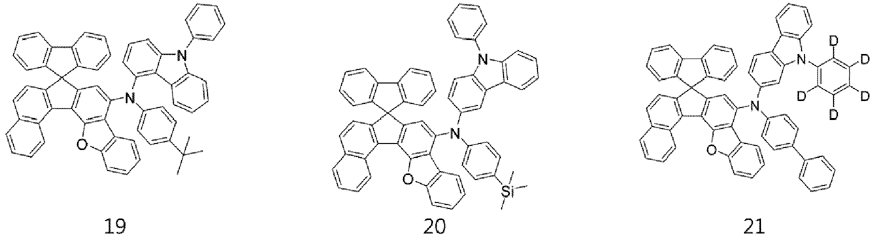

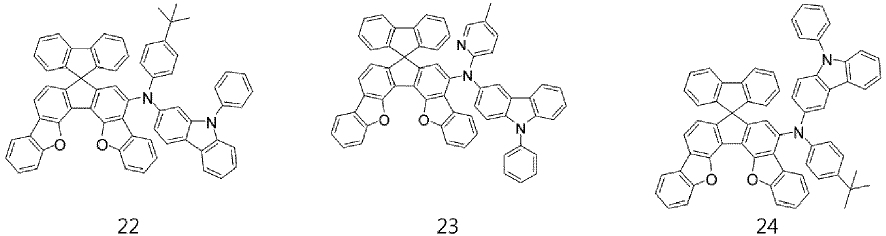

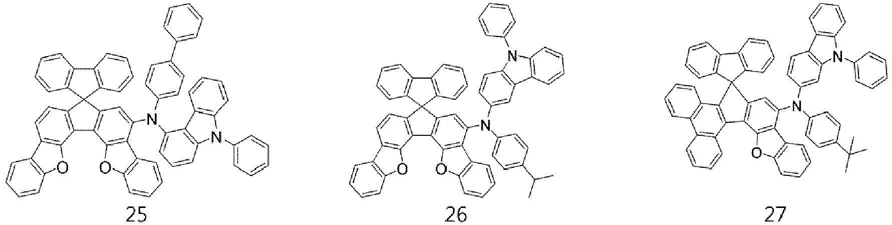

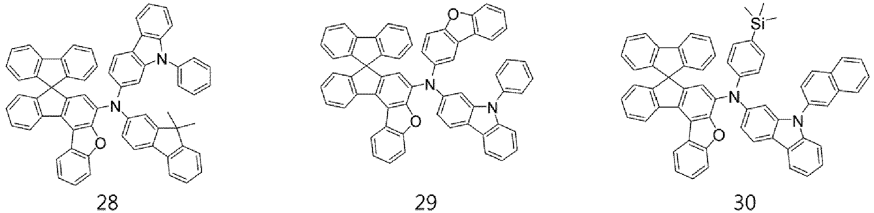

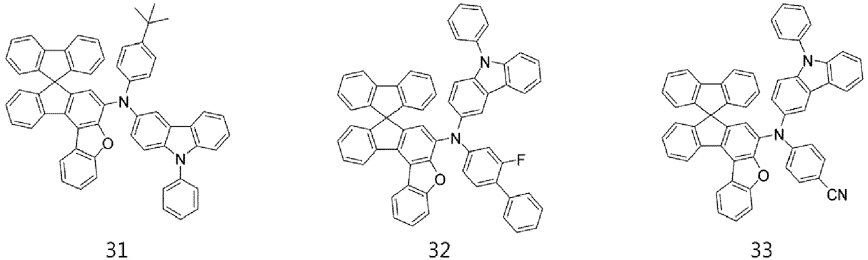

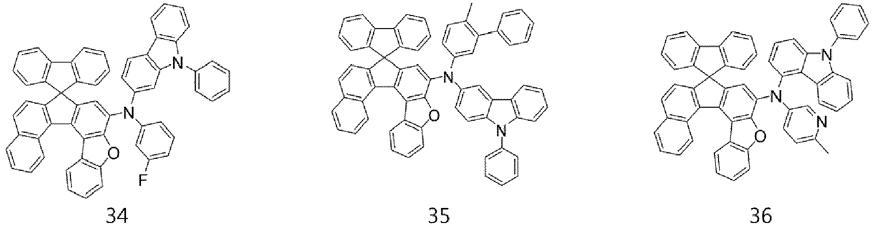

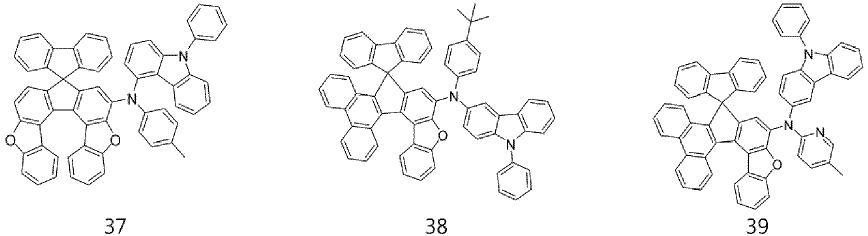

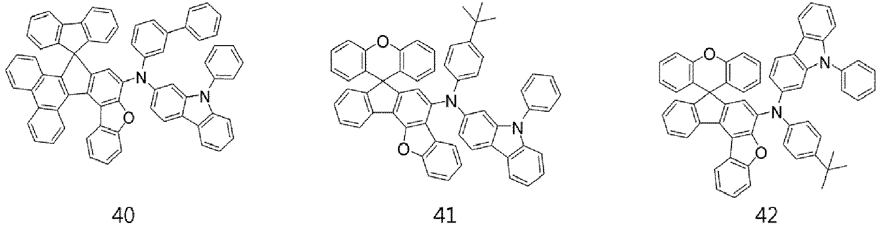

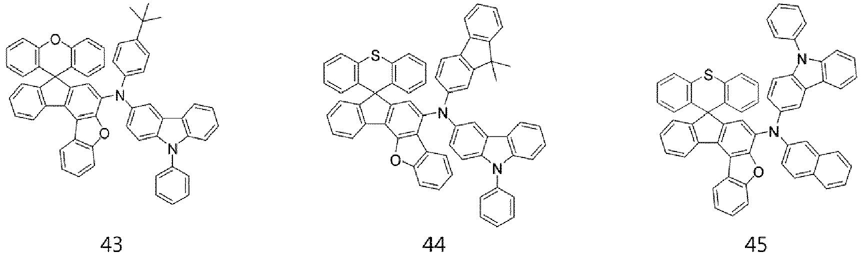

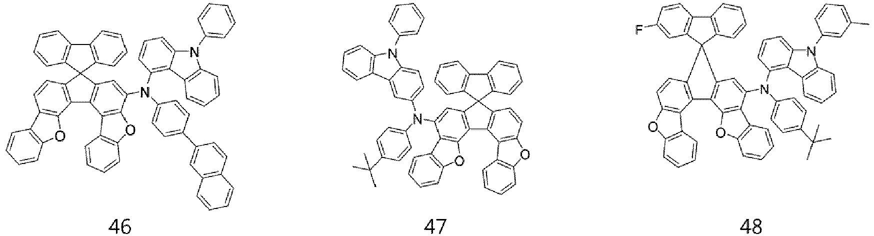

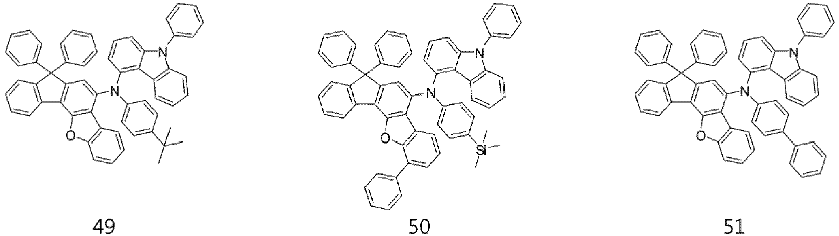

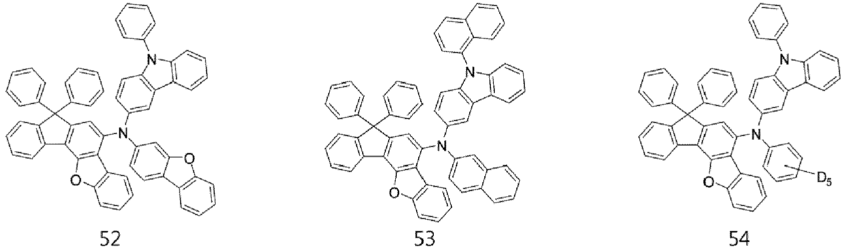

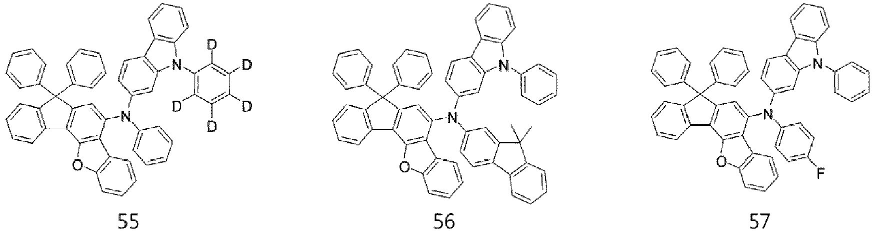

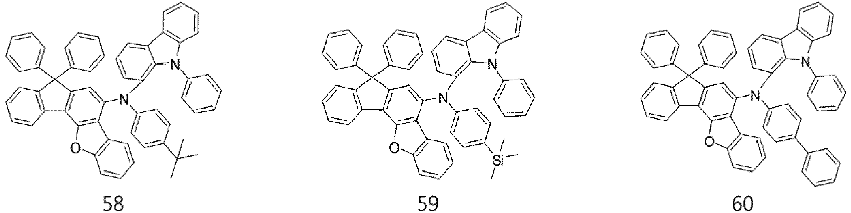

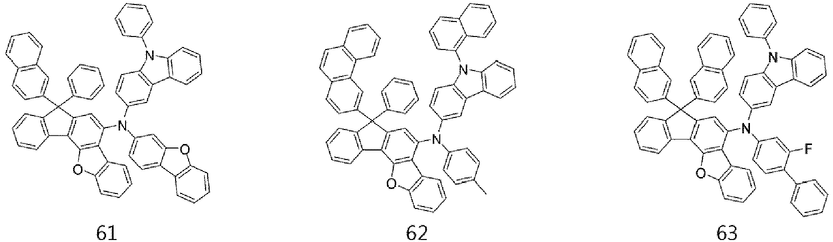

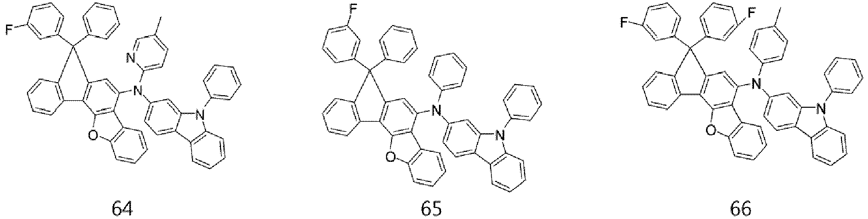

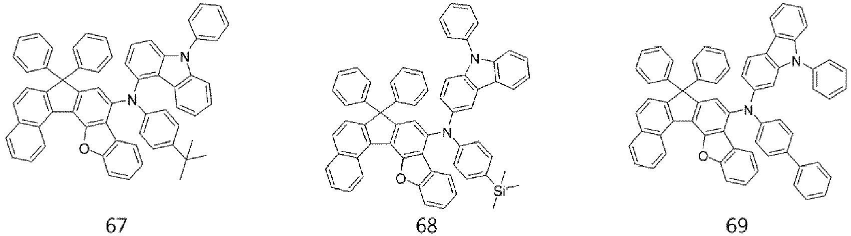

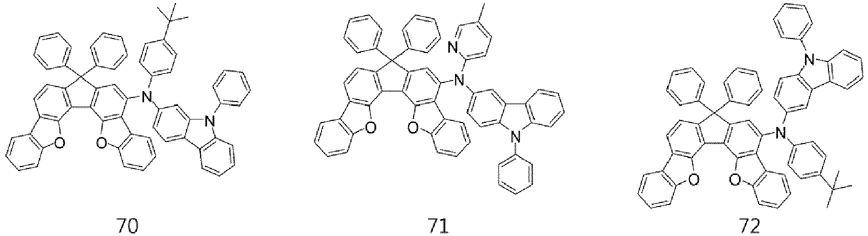

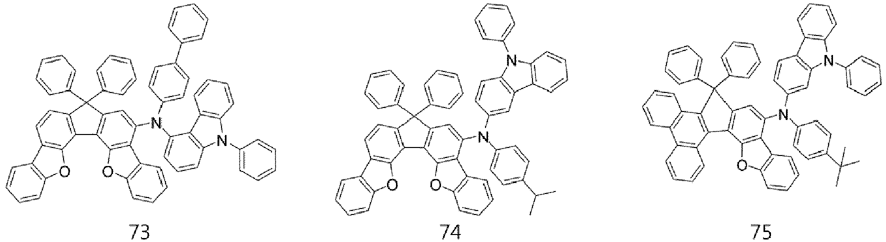

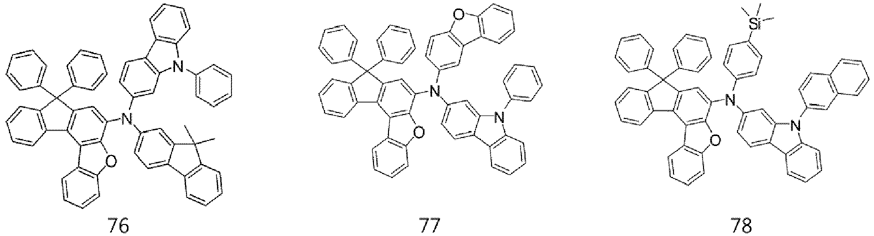

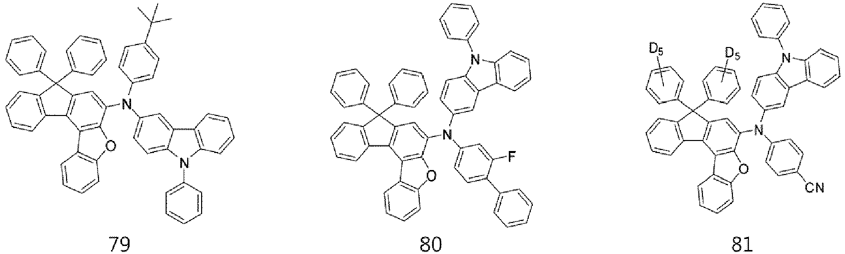

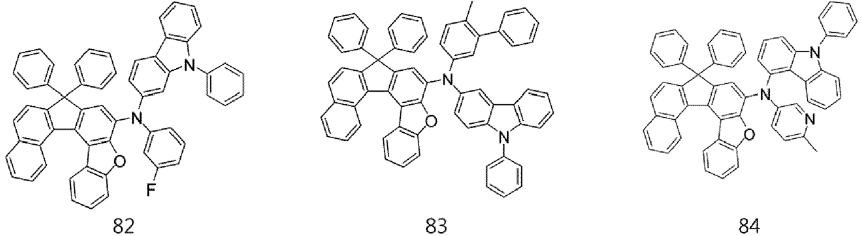

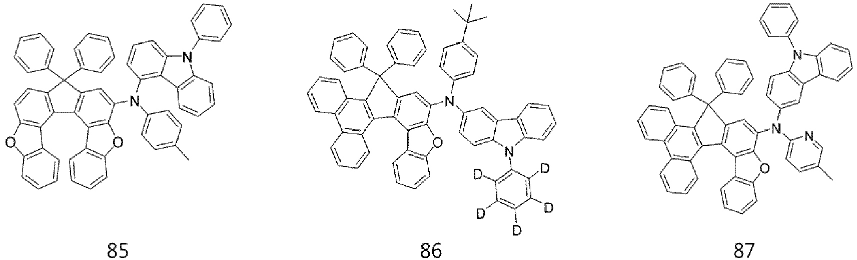

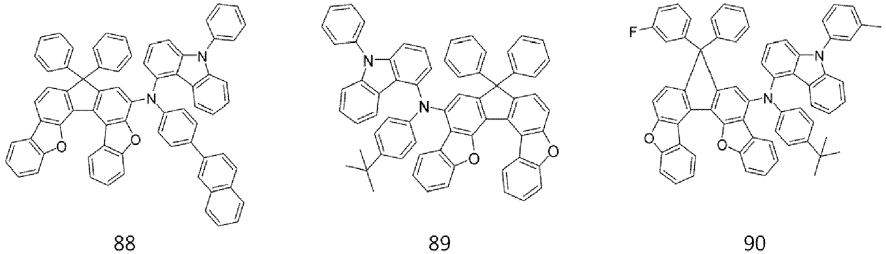

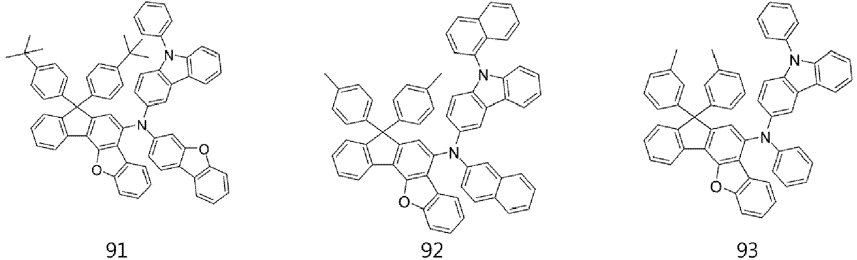

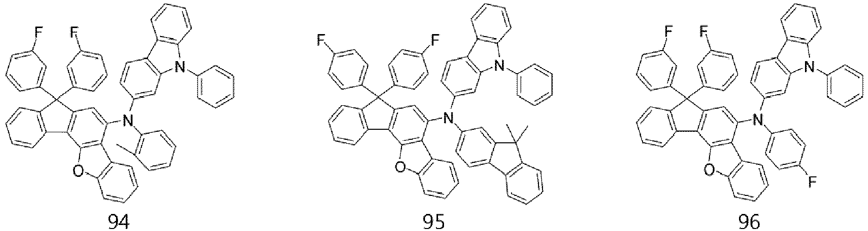

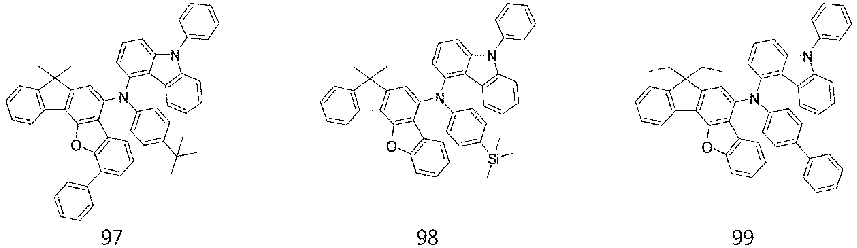

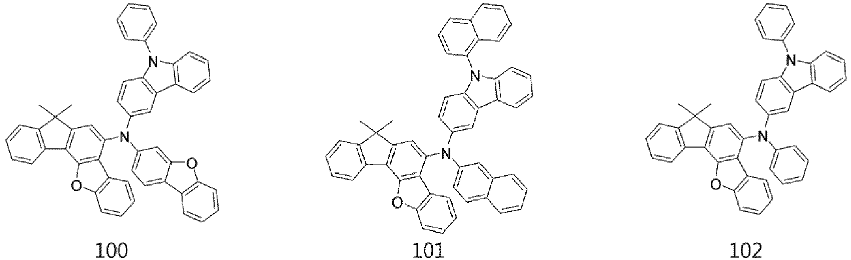

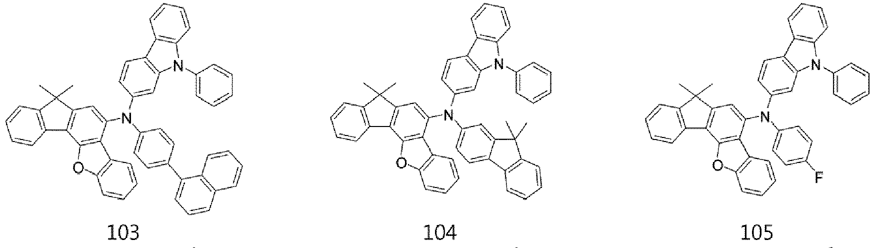

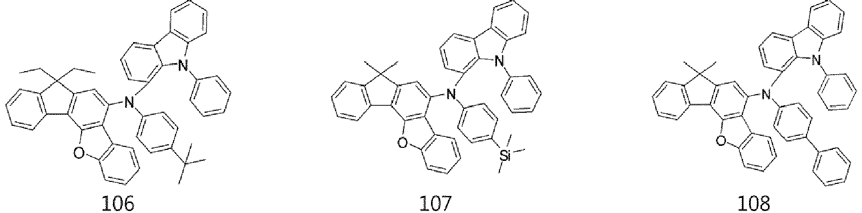

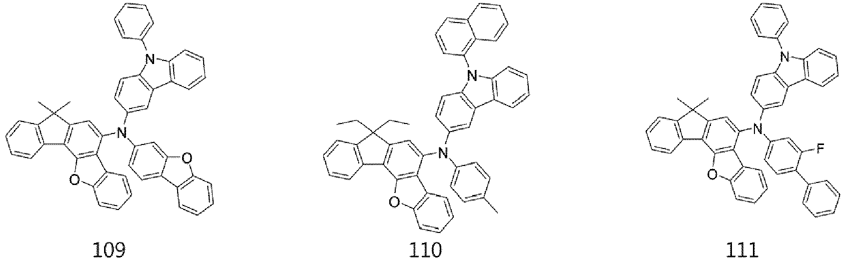

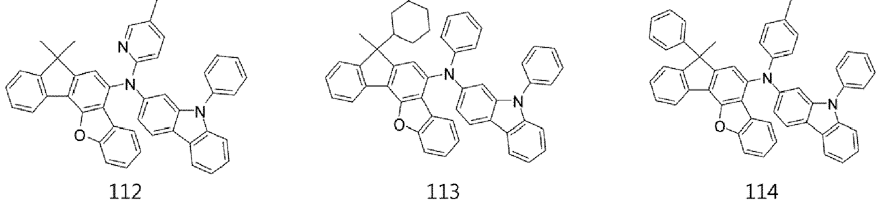

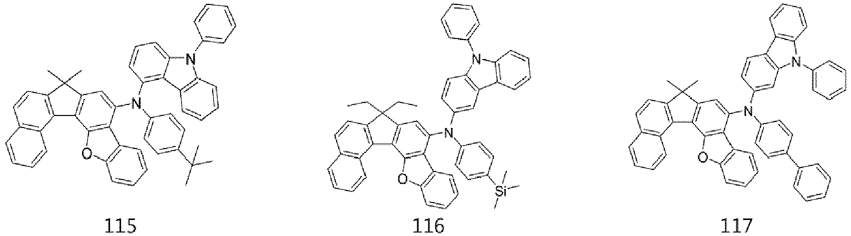

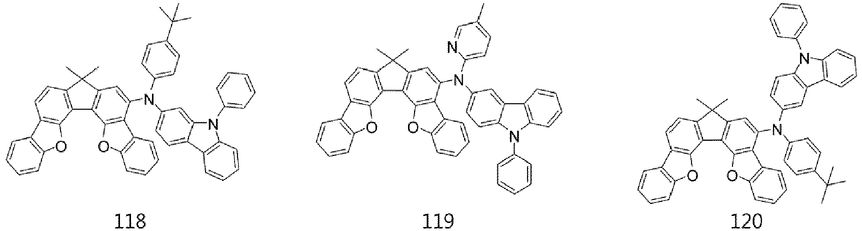

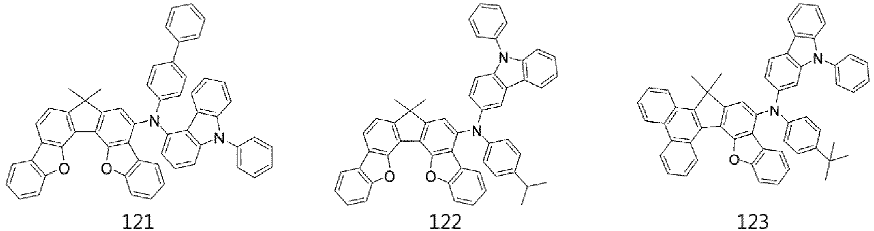

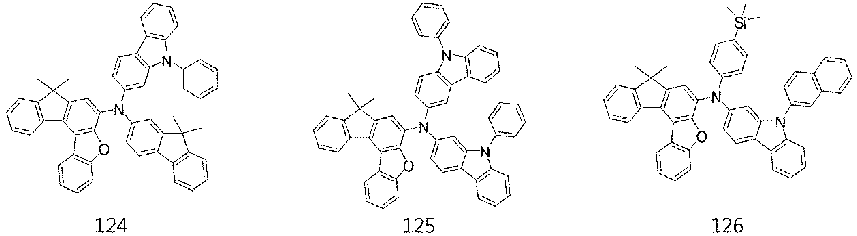

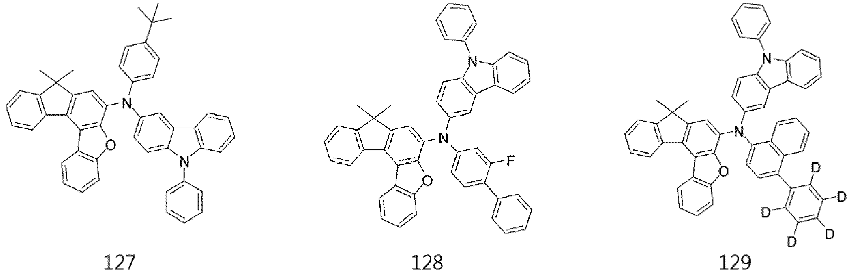

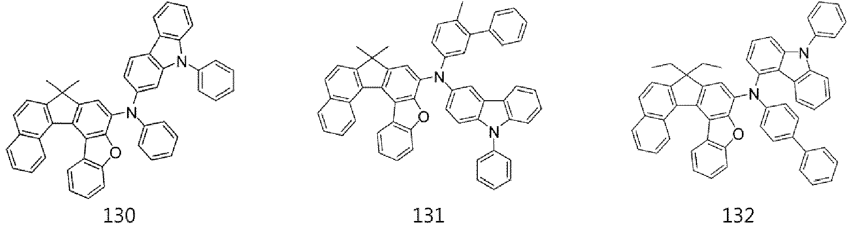

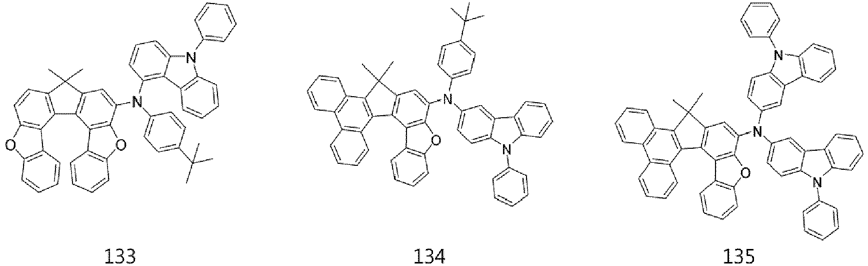

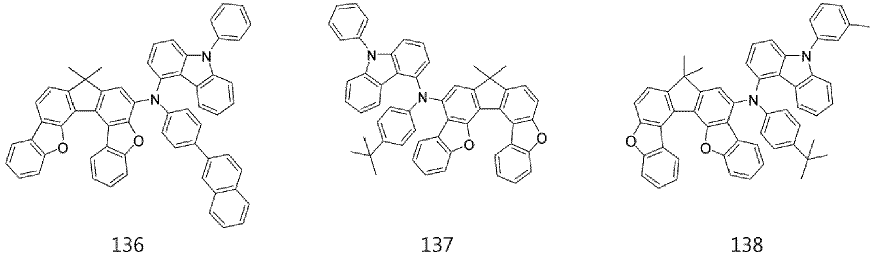

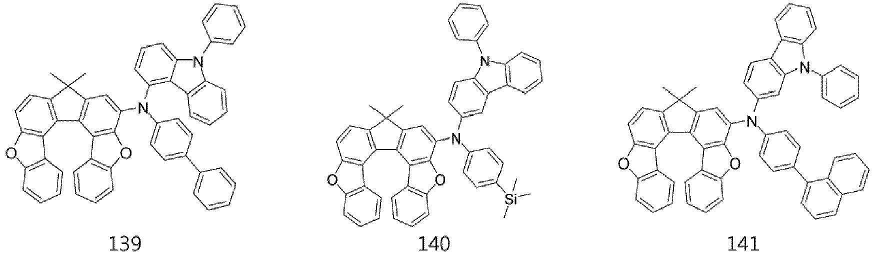

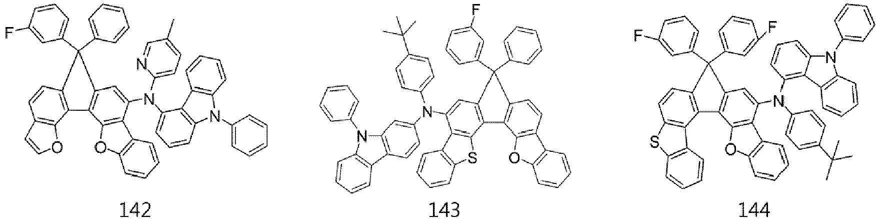

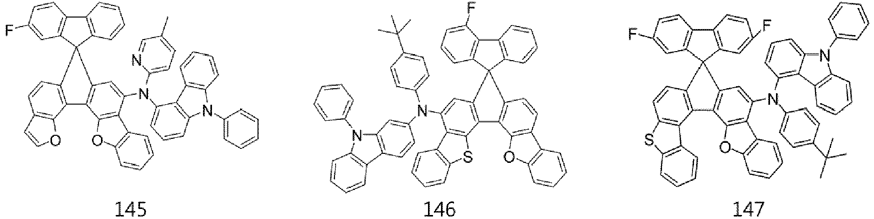

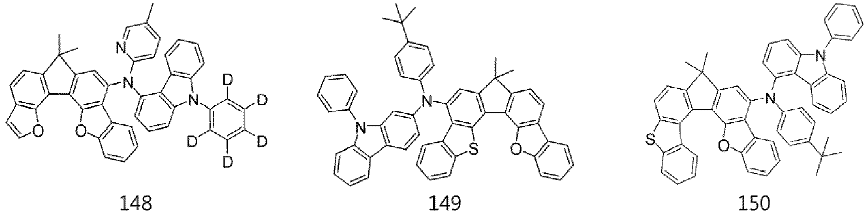

4. An organic light-emitting device comprising:
a first electrode;
a second electrode facing the first electrode; and
an organic layer interposed between the first electrode and the second electrode,
wherein the organic layer comprises at least one of the compound represented by [Formula A] or [Formula B] according to claim 1.

5. The organic light-emitting device according to claim 4, wherein the organic layer comprises at least one of an electron injection layer, an electron transport layer, a hole injection layer, a hole transport layer, an electron blocking layer, a hole blocking layer, and a light-emitting layer,
wherein at least one of the layers comprises the organic light-emitting compound represented by [Formula A] or [Formula B].

6. The organic light-emitting device according to claim 5, wherein the hole transport layer comprises the organic light-emitting compound represented by [Formula A] or [Formula B].

7. The organic light-emitting device according to claim 5, wherein the electron blocking layer comprises the organic light-emitting compound represented by [Formula A] or [Formula B].

8. The organic light-emitting device according to claim 5, wherein at least one layer selected from the layers is formed by a deposition process or a solution process.

9. The organic light-emitting device according to claim 4, wherein the organic light-emitting device is used for a display or lighting system selected from flat panel displays, flexible displays, monochromatic or white flat panel lighting systems, monochromatic or white flexible lighting systems, vehicle displays, and displays for virtual or augmented reality.
